# EUROPÄISCHE PATENTSCHRIFT

(11) **EP 0 906 293 B1**
(45) Veröffentlichungstag und Bekanntmachung des Hinweises auf die Patenterteilung: **17.09.2003**
(21) Anmeldenummer: 97923920.9
(22) Anmeldetag: 16.05.1997
(51) Int. Cl.: C07D 249/12, C07D 271/06, A01N 47/38, A01N 43/836, C07C 323/67, C07C 317/14, C07C 331/32

(54) **SUBSTITUIERTE SULFONYLAMINO(THIO)CARBONYLVERBINDUNGEN UND IHRE VERWENDUNG ALS HERBIZIDE**
SUBSTITUTED SULPHONYL AMINO(THIO)CARBONYL COMPOUNDS AND THEIR USE AS HERBICIDES
COMPOSES DE SULFONYLAMINO(THIO)CARBONYLE SUBSTITUES ET LEUR UTILISATION COMME HERBICIDES

(30) Priorität: 30.05.1996 DE 19621685
(43) Veröffentlichungstag der Anmeldung: 07.04.1999
(73) Patentinhaber: Bayer CropScience AG, 40789 Monheim (DE)
(72) Erfinder: SCHALLNER, Otto, D-40789 Monheim (DE); DREWES, Mark-Wilhelm, D-40764 Langenfeld (DE); FINDEISEN, Kurt, D-51375 Leverkusen (DE); GESING, Ernst-Rudolf, F., D-40699 Erkrath (DE); JANSEN, Johannes-Rudolf, D-40789 Monheim (DE); KIRSTEN, Rolf, D-40789 Monheim (DE); KLUTH, Joachim, D-40764 Langenfeld (DE); MÜLLER, Klaus-Helmut, D-40593 Düsseldorf (DE); KÖNIG, Klaus, D-51519 Odenthal (DE); PHILIPP, Ulrich, D-50674 Köln (DE); RIEBEL, Hans-Jochem, D-42113 Wuppertal (DE); ANDRES, Peter, D-40764 Langenfeld (DE); DOLLINGER, Markus, D-51381 Leverkusen (DE)
(86) Internationale Anmeldenummer: EP9702520
(87) Internationale Veröffentlichungsnummer: WO97046540

(56) Entgegenhaltungen:
- EP-A- 0 023 141
- EP-A- 0 023 422
- EP-A- 0 341 489
- EP-A- 0 422 469
- EP-A- 0 425 948
- EP-A- 0 431 291
- EP-A- 0 459 244
- EP-A- 0 534 266
- EP-A- 0 569 810
- EP-A- 0 570 171
- WO-A-94/08979
- WO-A-95/27703
- WO-A-96/11188
- DE-A- 3 624 103
- US-A- 4 645 527
- DATABASE CROSSFIRE Beilstein Informationssysteme GmbH, Frankfurt DE XP002037542 & GAZZ. CHIM. ITAL., Bd. 90, 1960, Seiten 1277-89,
- DATABASE CROSSFIRE Beilstein Informationssysteme GmbH, Frankfurt DE XP002037543 & J. CHEM. SOC., Bd. 73, 1898, Seite 753
- COLLECTION OF CZECHOSLOVAK CHEMICAL COMMUNICATIONS, Bd. 47, Nr. 1, 1982, PRAGUE CS, Seiten 72-87, XP002037538 K. SINDELAR ET AL.: "Tricyclic psychotropic agents containing two chalcogen atoms in the central ring: Synthesis of 11-(dimethylaminoalkyl) derivatives of 11H-dibenzo[b,e]-1,4-dioxepin and 11H-dibenzo[b,e]-1,4-dithiepin"
- CHEMICAL ABSTRACTS, vol. 112, no. 21, 21.Mai 1990 Columbus, Ohio, US; abstract no. 198428t, Seite 722; XP002037539 & CHEMICAL ABSTRACTS, 12TH COLLECTIVE INDEX, FORMULA, Seite 4158F & PL 134 567 A (AKADEMIA MEDYCZNA GDANSK)
- CHEMICAL ABSTRACTS, vol. 96, no. 13, 29.März 1982 Columbus, Ohio, US; abstract no. 100769h, S.G. SHEIKO ET AL.: "Bactericide activity of some benzene derivatives activated by nitro- and trifluoromethylsulfonyl groups" Seite 401; XP002037540 & FIZIOL. AKT. VESHCHESTVA, Bd. 13, 1981, Seiten 24-6,
- CHEMICAL ABSTRACTS, vol. 85, no. 19, 8.November 1976 Columbus, Ohio, US; abstract no. 142812v, Seite 499; XP002037541 & JP 76 070 740 A (MITSUI TOATSU CHEMICALS, INC.)

## Beschreibung

Die Erfindung betrifft neue substituierte Sulfonylamino(thio)carbonylverbindungen, mehrere Verfahren und neue Zwischenprodukte zu ihrer Herstellung sowie ihre Verwendung als Herbizide.

Es ist bereits bekannt, dass bestimmte Sulfonylaminocarbonylverbindungen herbizide Eigenschaften aufweisen (vgl. EP 341489, EP 422469, EP 425948, EP 431291, EP 507171, EP 534266, DE 4029753, WO 95/27703, EP 569 810A). Die Wirkung dieser Verbindungen ist jedoch nicht in allen Belangen zufriedenstellend.

Es wurden nun die neuen Sulfonylamino(thio)carbonylverbindungen der allgemeinen Formel (I), 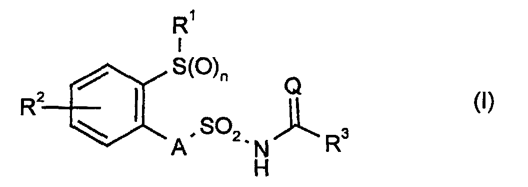 in welcher
- n: für die Zahlen 0, 1 oder 2 steht,
- A: für eine Einfachbindung steht,
- Q: für Sauerstoff oder Schwefel steht,
- R¹: für Wasserstoff, Formyl, für jeweils gegebenenfalls durch Cyano, Fluor, Chlor, Brom, Phenyl oder C₁-C₄-Alkoxy substituiertes Alkyl, Alkoxy, Alkylamino, Alkoxyamino, Dialkylamino, N-Alkoxy-N-alkyl-amino, Alkylcarbonyl, Alkoxycarbonyl, Alkylsulfonyl, Alkenyl oder Alkinyl mit jeweils bis zu 6 Kohlenstoffatomen, oder für jeweils gegebenenfalls durch Cyano, Fluor, Chlor, Brom oder C₁-C₄-Alkyl substituiertes C₃-C₆-Cycloalkyl, C₃-C₆-Cycloalkyl-carbonyl oder C₃-C₆-Cycloalkyl-sulfonyl steht,
- R²: für Cyano, Fluor, Chlor, Brom oder für jeweils gegebenenfalls durch Cyano, Fluor, Chlor, Brom oder C₁-C₄-Alkoxy substituiertes Alkyl, Alkoxy, Alkylthio, Alkylsulfinyl, Alkylsulfonyl, Dialkylaminosulfonyl, Alkenyl, Alkinyl, Alkenyloxy oder Alkinyloxy mit jeweils bis zu 6 Kohlenstoffatomen steht und
- R³: für gegebenenfalls substituiertes Heterocyclyl der nachstehenden Formel steht, 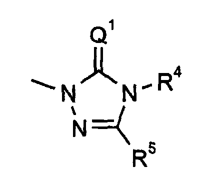
worin
- Q¹: jeweils für Sauerstoff oder Schwefel steht sowie
- R⁴: für Wasserstoff, Hydroxy, Amino, Cyano, für C₂-C₁₀-Alkylidenamino, für gegebenenfalls durch Fluor, Chlor, Brom, Cyano, C₁-C₄-Alkoxy, C₁-C₄-Alkyl-carbonyl oder C₁-C₄-Alkoxy-carbonyl substituiertes C₁-C₆-Alkyl, für jeweils gegebenenfalls durch Fluor, Chlor und/oder Brom substituiertes C₂-C₆-Alkenyl oder C₂-C₆-Alkinyl, für jeweils gegebenenfalls durch Fluor, Chlor, Brom, Cyano, C₁-C₄-Alkoxy oder C₁-C₄-Alkoxy-carbonyl substituiertes C₁-C₆-Alkoxy, C₁-C₆-Alkylamino oder C₁-C₆-Alkyl-carbonylamino, für C₃-C₆-Alkenyloxy, für Di-(C₁-C₄-alkyl)-amino, für jeweils gegebenenfalls durch Fluor, Chlor, Brom, Cyano und/oder C₁-C₄-Alkyl substituiertes C₃-C₆-Cycloalkyl, C₃-C₆-Cycloalkylamino oder C₃-C₆-Cycloalkyl-C₁-C₄-alkyl, oder für jeweils gegebenenfalls durch Fluor, Chlor, Brom, Cyano, Nitro, C₁-C₄-Alkyl, Trifluormethyl und/oder C₁-C₄-Alkoxy substituiertes Phenyl oder Phenyl-C₁-C₄-alkyl steht,
- R⁵: für Wasserstoff, Hydroxy, Mercapto, Amino, Cyano, Fluor, Chlor, Brom, Iod, für gegebenenfalls durch Fluor, Chlor, Brom, Cyano, C₁-C₄-Alkoxy, C₁-C₄-Alkyl-carbonyl oder C₁-C₄-Alkoxy-carbonyl substituiertes C₁-C₆-Alkyl, für jeweils gegebenenfalls durch Fluor, Chlor und/oder Brom substituiertes C₂-C₆-Alkenyl oder C₂-C₆-Alkinyl, für jeweils gegebenenfalls durch Fluor, Chlor, Cyano, C₁-C₄-Alkoxy oder C₁-C₄-Alkoxy-carbonyl substituiertes C₁-C₆-Alkoxy, C₁-C₆-Alkylthio, C₁-C₆-Alkylamino oder C₁-C₆-Alkyl-carbonylamino, für C₃-C₆-Alkenyloxy, C₃-C₆-Alkinyloxy, C₃-C₆-Alkenylthio, C₃-C₆-Alkinylthio, C₃-C₆-Alkenylamino oder C₃-C₆-Alkinylamino, für Di-(C₁-C₄-alkyl)-amino, für jeweils gegebenenfalls durch Methyl und/oder Ethyl substituiertes Aziridino, Pyrrolidino, Piperidino oder Morpholino, für jeweils gegebenenfalls durch Fluor, Chlor, Brom, Cyano und/oder C₁-C₄-Alkyl substituiertes C₃-C₆-Cycloalkyl, C₅-C₆-Cycloalkenyl, C₃-C₆-Cycloalkyloxy, C₃-C₆-Cycloalkylthio, C₃-C₆-Cycloalkylamino, C₃-C₆-Cycloalkyl-C₁-C₄-alkyl, C₃-C₆-Cycloalkyl-C₁-C₄-alkoxy, C₃-C₆-Cycloalkyl-C₁-C₄-alkylthio oder C₃-C₆-Cycloalkyl-C₁-C₄-alkylamino, oder für jeweils gegebenenfalls durch Fluor, Chlor, Brom, Cyano, Nitro, C₁-C₄-Alkyl, Trifluormethyl, C₁-C₄-Alkoxy und/oder C₁-C₄-Alkoxy-carbonyl substituiertes Phenyl, Phenyl-C₁-C₄-alkyl, Phenoxy, Phenyl-C₁-C₄-alkoxy, Phenylthio, Phenyl-C₁-C₄-alkylthio, Phenylamino oder Phenyl-C₁-C₄-alkylamino steht, oder
- R⁴ und R⁵: zusammen für gegebenenfalls verzweigtes Alkandiyl mit 3 bis 11 Kohlenstoffatomen stehen,
sowie die Salze von Verbindungen der Formel (I).
Gegenstand der Erfindung sind weiter vorzugsweise Natrium-, Kalium-, Magnesium-, Calcium-, Ammonium-, C₁-C₄-Alkyl-ammonium-, Di-(C₁-C₄-alkyl)-ammonium-, Tri-(C₁-C₄-alkyl)-ammonium-, Tetra-(C₁-C₄-alkyl)-ammonium, Tri-(C₁-C₄-alkyl)-sulfonium-, C₅- oder C₆-Cycloalkyl-ammonium- und Di-(C₁-C₂-alkyl)-benzyl-ammonium-Salze von Verbindungen der Formel (I), in welcher n, A, Q , R¹, R² und R³ die vorzugsweise angegebenen Bedeutungen haben.

Man erhält die neuen substituierten Sulfonylamino(thio)carbonylverbindungen der allgemeinen Formel (I), wenn man
(a) Aminosulfonylverbindungen der allgemeinen Formel (II) 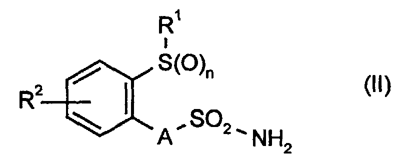 in welcher
   - n, A, R¹ und R²: die oben angegebene Bedeutung haben,
   mit (Thio)Carbonsäurederivaten der allgemeinen Formel (III) 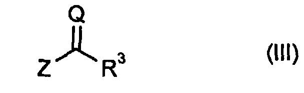 in welcher
   - Q und R³: die oben angegebene Bedeutung haben und
   - Z: für Halogen, Alkoxy, Aryloxy oder Arylalkoxy steht,
   gegebenenfalls in Gegenwart eines Säureakzeptors und gegebenenfalls in Gegenwart eines Verdünnungsmittels umsetzt,
   oder wenn man
(b) Sulfonyliso(thio)cyanate der allgemeinen Formel (IV) 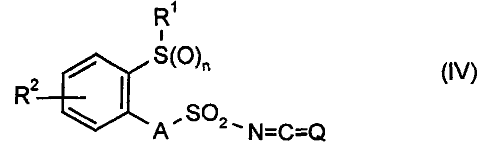 in welcher
   - n, A, Q, R¹ und R²: die oben angegebene Bedeutung haben,
   mit Heterocyclen der allgemeinen Formel (V)

   H-R³ (V)

   in welcher
   - R³: die oben angegebene Bedeutung hat,
   gegebenenfalls in Gegenwart eines Reaktionshilfsmittels und gegebenenfalls in Gegenwart eines Verdünnungsmittels umsetzt,
   oder wenn man
(c) Chlorsulfonylverbindungen der allgemeinen Formel (VI) 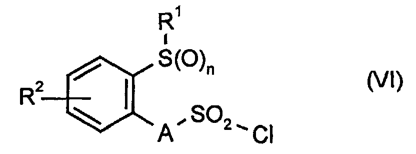 in welcher
   - n, A, R¹ und R²: die oben angegebene Bedeutung haben,
   mit Heterocyclen der allgemeinen Formel (V)

   H-R³ (V)

   in welcher
   - R³: die oben angegebene Bedeutung hat,
   und Metall(thio)cyanaten der allgemeinen Formel (VII)

   MQCN (VII)

   in welcher
   - Q: die oben angegebene Bedeutung hat,
   gegebenenfalls in Gegenwart eines Reaktionshilfsmittels und gegebenenfalls in Gegenwart eines Verdünnungsmittels umsetzt,
   oder wenn man
(d) Chlorsulfonylverbindungen der allgemeinen Formel (VI) 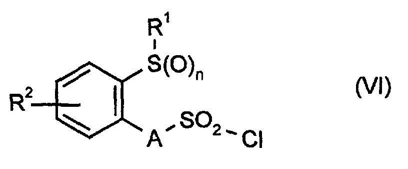 in welcher
   - n, A, R¹ und R²: die oben angegebene Bedeutung haben,
   mit (Thio)Carbonsäureamiden der allgemeinen Formel (VIII) 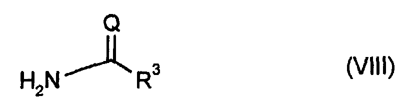 in welcher
   - Q und R³: die oben angegebene Bedeutung haben,
   gegebenenfalls in Gegenwart eines Säureakzeptors und gegebenenfalls in Gegenwart eines Verdünnungsmittels umsetzt,
   oder wenn man
(e) Sulfonylamino(thio)carbonylverbindungen der allgemeinen Formel (IX) 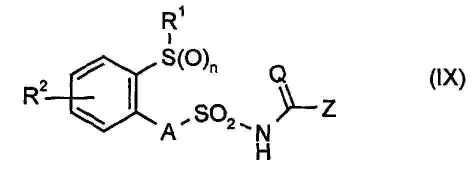 in welcher
   - n, A, Q, R¹ und R²: die oben angegebene Bedeutung haben und
   - Z: für Halogen, Alkoxy, Aryloxy oder Arylalkoxy steht,
   mit Heterocyclen der allgemeinen Formel (V)

   H-R³ (V)

   in welcher
   - R³: die oben angegebene Bedeutung hat,
   gegebenenfalls in Gegenwart eines Säureakzeptors und gegebenenfalls in Gegenwart eines Verdünnungsmittels umsetzt,
   oder wenn man
(f) Heterocyclen der allgemeinen Formel (V)

   H-R³ (V)

   in welcher
   - R³: die oben angegebene Bedeutung hat,
   mit Chlorsulfonyliso(thio)cyanat gegebenenfalls in Gegenwart eines Verdünnungsmittels umsetzt und die hierbei gebildeten Addukte in situ mit Benzolderivaten der allgemeinen Formel (X) 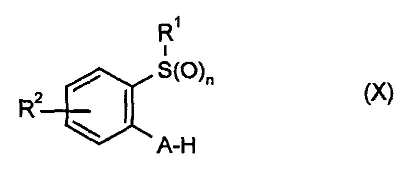
in welcher
- n, A, R¹ und R²: die oben angegebene Bedeutung haben,
gegebenenfalls in Gegenwart eines Säureakzeptors und gegebenenfalls in Gegenwart eines Verdünnungsmittels umsetzt,
und gegebenenfalls die nach den Verfahren (a), (b), (c), (d), (e) oder (f) erhaltenen Verbindungen der Formel (I) nach üblichen Methoden in Salze überführt.

Die neuen substituierten Sulfonylamino(thio)carbonylverbindungen der allgemeinen Formel (I) zeichnen sich durch starke herbizide Wirksamkeit aus.

Gegenstand der Erfindung sind insbesondere Verbindungen der Formel (I), in welcher
- n: für die Zahlen 0, 1 oder 2 steht,
- A: für eine Einfachbindung steht,
- Q: für Sauerstoff oder Schwefel steht,
- R¹: für Wasserstoff, Formyl, für jeweils gegebenenfalls durch Fluor, Chlor, Brom, Methoxy oder Ethoxy substituiertes Methyl, Ethyl, n- oder i-Propyl, n-, i- oder s-Butyl, Methoxy, Ethoxy, n- oder i-Propoxy, n-, i-, s- oder t-Butoxy, Methylamino, Ethylamino, n- oder i-Propylamino, n-, i-, s- oder t-Butylamino, Methoxyamino, Ethoxyamino, n- oder i-Propoxyamino, n-, i-, s- oder t-Butoxyamino, Dimethylamino, Diethylamino, N-Methoxy-N-methyl-amino, Acetyl, Propionyl, Butyroyl, Methoxycarbonyl, Ethoxycarbonyl, n- oder i-Propoxy-carbonyl, Methylsulfonyl, Ethylsulfonyl, n- oder i-Propylsulfonyl, n-, i-, s- oder t-Butylsulfonyl, Propenyl, Butenyl, Propinyl oder Butinyl, oder für jeweils gegebenenfalls durch Fluor, Chlor oder Methyl substituiertes Cyclopropyl, Cyclopropylcarbonyl oder Cyclopropylsulfonyl steht,
- R²: für Cyano, Fluor, Chlor, Brom, oder für jeweils gegebenenfalls durch Fluor, Chlor, Methoxy oder Ethoxy substituiertes Methyl, Ethyl, n- oder i-Propyl, n-, i-oder s-Butyl, Methoxy, Ethoxy, n- oder i-Propoxy, n-, i- oder s-Butoxy, Methylthio, Ethylthio, n- oder i-Propylthio, n-, i-, s- oder t-Butylthio, Methylsulfinyl, Ethylsulfinyl, Methylsulfonyl, Ethylsulfonyl, Dimethylaminosulfonyl oder Diethylaminosulfonyl, für Propenyl, Butenyl, Propinyl, Butinyl, Propenyloxy, Butenyloxy, Propinyloxy oder Butinyloxy steht und
- R³: für gegebenenfalls substituiertes Heterocyclyl der nachstehenden Formel steht 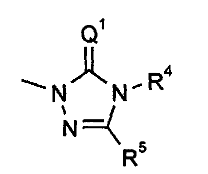
worin
- Q¹: für Sauerstoff oder Schwefel steht sowie
- R⁴: für Wasserstoff, Hydroxy, Amino, für C₃-C₈-Alkylidenamino, für jeweils gegebenenfalls durch Fluor, Chlor, Cyano, Methoxy oder Ethoxy substituiertes Methyl, Ethyl, n- oder i-Propyl, n-, i-, s- oder t-Butyl, für jeweils gegebenenfalls durch Fluor, Chlor oder Brom substituiertes Propenyl, Butenyl, Propinyl oder Butinyl, für jeweils gegebenenfalls durch Fluor, Chlor, Cyano, Methoxy oder Ethoxy substituiertes Methoxy, Ethoxy, n- oder i-Propoxy, n-, i-, s- oder t-Butoxy, Methylamino, Ethylamino, n- oder i-Propylamino, n-, i-, s- oder t-Butylamino, für Propenyloxy oder Butenyloxy, für Dimethylamino oder Diethylamino, für jeweils gegebenenfalls durch Fluor, Chlor, Methyl und/oder Ethyl substituiertes Cyclopropyl, Cyclobutyl, Cyclopentyl, Cyclohexyl, Cyclopropylamino, Cyclobutylamino, Cyclopentylamino, Cyclohexylamino, Cyclopropylmethyl, Cyclobutylmethyl, Cyclopentylmethyl oder Cyclohexylmethyl, oder für jeweils gegebenenfalls durch Fluor, Chlor, Methyl, Trifluormethyl und/oder Methoxy substituiertes Phenyl oder Benzyl steht,
- R⁵: für Wasserstoff, Hydroxy, Mercapto, Amino, Fluor, Chlor, Brom, für jeweils gegebenenfalls durch Fluor, Chlor, Cyano, Methoxy oder Ethoxy substituiertes Methyl, Ethyl, n- oder i-Propyl, n-, i-, s- oder t-Butyl, für jeweils gegebenenfalls durch Fluor, Chlor oder Brom substituiertes Ethenyl, Propenyl, Butenyl, Propinyl oder Butinyl, für jeweils gegebenenfalls durch Fluor, Chlor, Cyano, Methoxy oder Ethoxy substituiertes Methoxy, Ethoxy, n- oder i-Propoxy, n-, i-, s- oder t-Butoxy, Methylthio, Ethylthio, n- oder i-Propylthio, n-, i-, s- oder t-Butylthio, Methylamino, Ethylamino, n- oder i-Propylamino, n-, i-, s- oder t-Butylamino, für Propenyloxy, Butenyloxy, Propinyloxy, Butinyloxy, Propenyl-thio, Propadienylthio Butenylthio, Propinylthio, Butinylthio, Propenylamino, Butenylamino, Propinylamino oder Butinylamino, für Dimethylamino, Diethylamino oder Dipropylamino, für jeweils gegebenenfalls durch Fluor, Chlor, Methyl und/oder Ethyl substituiertes Cyclopropyl, Cyclobutyl, Cyclopentyl, Cyclohexyl, Cyclopentenyl, Cyclohexenyl, Cyclopropyloxy, Cyclobutyloxy, Cyclopentyloxy, Cyclohexyloxy, Cyclopropylthio, Cyclobutylthio, Cyclopentylthio, Cyclohexylthio, Cyclopropylamino, Cyclobutylamino, Cyclopentylamino, Cyclohexylamino, Cyclopropylmethyl, Cyclobutylmethyl, Cyclopentylmethyl, Cyclohexylmethyl, Cyclopropylmethoxy, Cyclobutylmethoxy, Cyclopentylmethoxy, Cyclohexylmethoxy, Cyclopropylmethylthio, Cyclobutylmethylthio, Cyclopentylmethylthio, Cyclohexylmethylthio, Cyclopropylmethylamino, Cyclobutylmethylamino, Cyclopentylmethylamino oder Cyclohexylmethylamino, oder für jeweils gegebenenfalls durch Fluor, Chlor, Methyl, Trifluormethyl, Methoxy und/oder Methoxycarbonyl substituiertes Phenyl, Benzyl, Phenoxy, Benzyloxy, Phenylthio, Benzylthio, Phenylamino oder Benzylamino steht, oder
- R⁴ und R⁵: zusammen für gegebenenfalls verzweigtes Alkandiyl mit 3 bis 11 Kohlenstoffatomen stehen.

Eine ganz besonders bevorzugte Gruppe erfindungsgemäßer Verbindungen sind die Verbindungen der Formel (I), in welcher
- n: für die Zahlen 0, 1 oder 2 steht,
- A: für eine Einfachbindung steht,
- Q: für Sauerstoff oder Schwefel steht,
- R¹: für jeweils gegebenenfalls durch Fluor und/oder Chlor substituiertes Methyl, Ethyl, n- oder i-Propyl, n-, i-, s- oder t-Butyl steht,
- R²: für Fluor, Chlor, Brom, oder für jeweils gegebenenfalls durch Fluor und/oder Chlor substituiertes Methyl, Ethyl, Methoxy, Ethoxy, Methylthio oder Ethylthio - jeweils in 6-Position - steht und
- R³: für gegebenenfalls substituiertes Triazolinyl der nachstehenden Formel steht 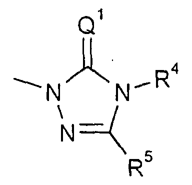
worin
- Q¹: für Sauerstoff oder Schwefel steht sowie
- R⁴: für jeweils gegebenenfalls durch Fluor, Chlor, Cyano, Methoxy oder Ethoxy substituiertes Methyl, Ethyl, n- oder i-Propyl, für Propenyl oder Propinyl, für Methoxy, Ethoxy, n- oder i-Propoxy, oder für Cyclopropyl steht,
- R⁵: für Wasserstoff, Chlor, Brom, für jeweils gegebenenfalls durch Fluor, Chlor, Cyano, Methoxy oder Ethoxy substituiertes Methyl, Ethyl, n- oder i-Propyl, für jeweils gegebenenfalls durch Fluor und/oder Chlor substituiertes Propenyl oder Propinyl, für jeweils gegebenenfalls durch Fluor, Chlor, Cyano, Methoxy oder Ethoxy substituiertes Methoxy, Ethoxy, n- oder i-Propoxy, Methylthio, Ethylthio, n- oder i-Propylthio, für Propenyloxy oder Cyclopropyl, steht.

Die oben aufgeführten allgemeinen oder in Vorzugsbereichen aufgeführten Restedefinitionen gelten sowohl für die Endprodukte der Formel (I) als auch entsprechend für die jeweils zur Herstellung benötigten Ausgangsstoffe bzw. Zwischenprodukte. Diese Restedefinitionen können untereinander, also auch zwischen den angegebenen bevorzugten Bereichen beliebig kombiniert werden.

Verwendet man beispielsweise 2-Fluor-6-methylthio-benzolsulfonamid und 5-Ethoxy-4-methyl-2-phenoxycarbonyl-2,4-dihydro-3H-1,2,4-triazol-3-thion als Ausgangsstoffe, so kann der Reaktionsablauf beim erfindungsgemäßen Verfahren (a) durch das folgende Formelschema skizziert werden:

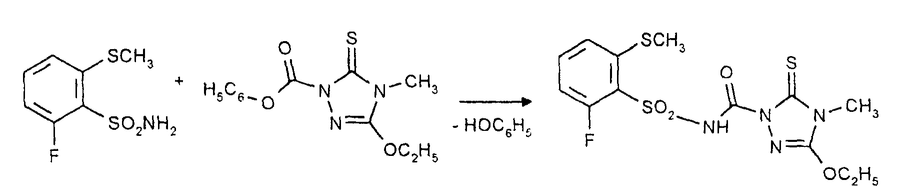

Verwendet man beispielsweise 2-Ethylthio-6-methyl-phenylsulfonyl-isothiocyanat und 5-Ethyl-4-methoxy-2,4-dihydra-3H-1,2,4-triazol-3-on als Ausgangsstoffe, so kann der Reaktionsablauf beim erfindungsgemäßen Verfahren (b) durch das folgende Formelschema skizziert werden:

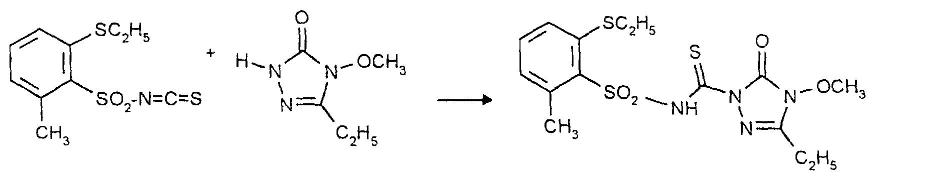

Verwendet man beispielsweise 2-Methylthio-3-methyl-benzolsulfochlorid, 5-Ethylthio-4-methoxy-2,4-dihydro-3H-1,2,4-triazol-3-on und Kaliumcyanat als Ausgangsstoffe, so kann der Reaktionsablauf beim erfindungsgemäßen Verfahren (c) durch das folgende Formelschema skizziert werden:

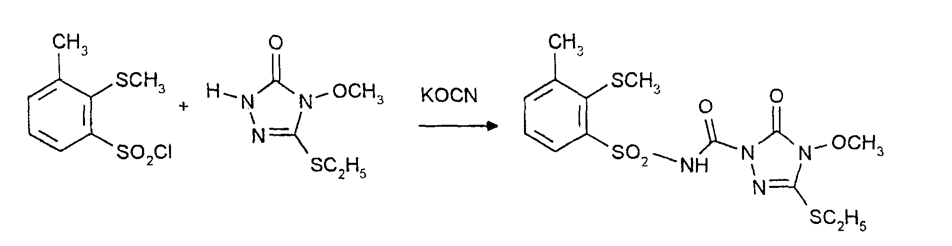

Verwendet man beispielsweise 2-Ethylthio-4-fluor-benzolsulfochlorid und 5-Methyl-1,2,4-oxadiazol-3-carboxamid als Ausgangsstoffe, so kann der Reaktionsablauf beim erfindungsgemäßen Verfahren (d) durch das folgende Formelschema skizziert werden:

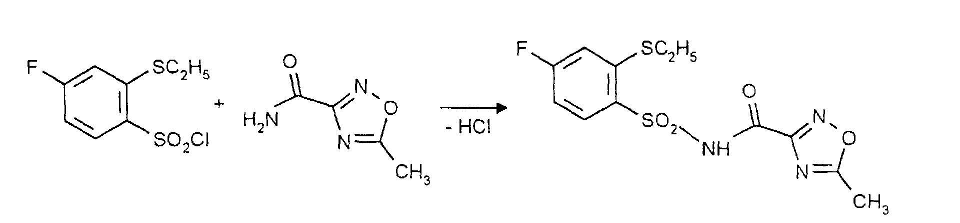

Verwendet man beispielsweise N-(2-Chlor-6-prapylthio-phenylsulfonyl)-O-methylurethan und 4-Methyl-5-methylthio-2,4-dihydro-3H-1,2,4-triazol-3-on als Ausgangsstoffe, so kann der Reaktionsablauf beim erfindungsgemäßen Verfahren (e) durch das folgende Formelschema skizziert werden:

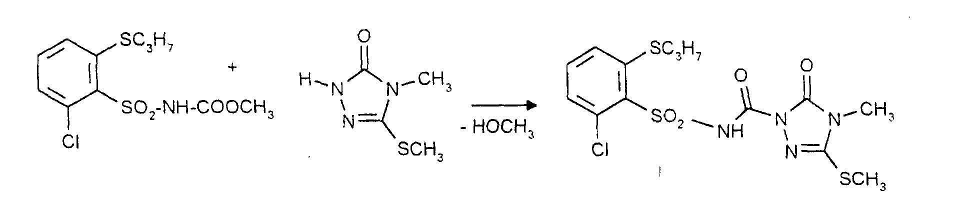

Verwendet man beispielsweise 5-Chlor-4-ethyl-2,4-dihydro-3H-1,2,4-triazol-3-on und Chlorsulfonylisocyanat sowie anschließend 2-Ethylthio-6-methyl-anilin als Ausgangsstoffe, so kann der Reaktionsablauf beim erfindungsgemäßen Verfahren (f) durch das folgende Formelschema skizziert werden:

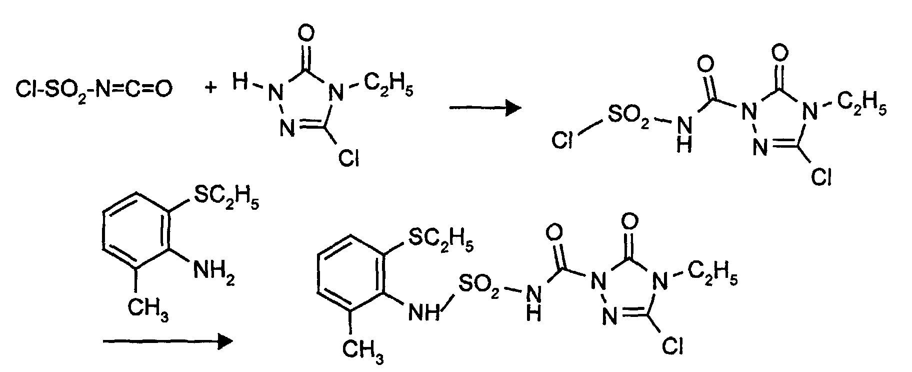

Die beim erfindungsgemäßen Verfahren (a) zur Herstellung der Verbindungen der Formel (I) als Ausgangsstoffe zu verwendenden Aminosulfonylverbindungen sind durch die Formel (II) allgemein definiert. In der Formel (II) haben n, A, R¹ und R² vorzugsweise bzw. insbesondere diejenige Bedeutung, die bereits oben bei der Beschreibung der erfindungsgemäßen Verbindungen der Formel (I) vorzugsweise bzw. als insbesondere bevorzugt für n, A, R¹ und R² angegeben wurde.

Die Ausgangsstoffe der Formel (II) sind teilweise aus der Literatur (vgl. EP 135332, US 4604131, EP 23 141 A, EP 23 422 A, US 4 645 527, Verbindungen mit Beilstein Registry No. 2 647 409 und 2 695 486, CAS Vol. 112, No. 21, 198428t) bekannt; sie sind teilweise als neue Stoffe auch Gegenstand der vorliegenden Anmeldung.

Man erhält die neuen Aminosulfonylverbindungen der Formel (II), in welcher n für Null steht, A für eine Einfachbindung steht und R¹ für jeweils gegebenenfalls substituiertes Alkyl, Alkenyl, Alkinyl oder Cycloalkyl steht, wenn man t-Butylaminosulfonylverbindungen der allgemeinen Formel (XI) 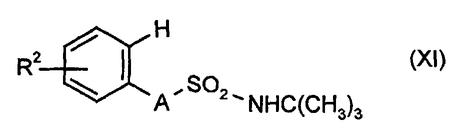 in welcher
- A und R²: die oben angegebene Bedeutung haben,
mit einer metallorganischen Verbindung, wie z.B. Butyllithium in Hexan, gegebenenfalls in Gegenwart eines (weiteren) inerten Verdünnungsmittels, wie z.B.

Tetrahydrofuran, und unter Inertgasatmosphäre, wie z.B. unter Argon, bei Temperaturen zwischen -50°C und +20°C metalliert - d.h. das in der Formel (XI) eingezeichnete Wasserstoffatom durch ein Metallatom ersetzt - dann im gleichen Reaktionsmedium mit Schwefel bei Temperaturen zwischen -30°C und +30°C umsetzt - d.h. das Metallatom durch Schwefel ersetzt - dann im gleichen Reaktionsmedium mit einem Alkylierungsmittel der allgemeinen Formel (XII)

X¹-R¹ (XII)

in welcher
- R¹: für jeweils gegebenenfalls substituiertes Alkyl, Alkenyl, Alkinyl oder Cycloalkyl steht und
- X¹: für Halogen, vorzugsweise Chlor, Brom oder Iod steht,
bei Temperaturen zwischen 0°C und 100°C umsetzt und dann - vorzugsweise nach Zwischenisolierung - die so erhaltenen t-Butylaminosulfonylverbindungen der allgemeinen Formel (XIII) 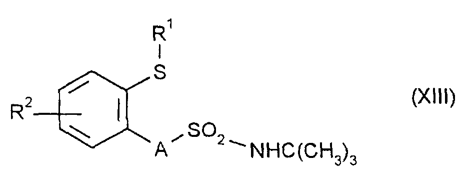 in welcher
- A, R¹ und R²: die oben angegebene Bedeutung haben,
mit einer starken Säure, wie z.B. Trifluoressigsäure, gegebenenfalls in Gegenwart eines Verdünnungsmittels, wie z.B. Methylenchlorid, bei Temperaturen zwischen 0°C und 50°C umsetzt (vgl. die Herstellungsbeispiele).

Man erhält die neuen Verbindungen der Formel (II), in welcher n für Null steht, A für eine Einfachbindung steht und R¹ für H steht, wenn man die t-Butylaminosulfonylverbindungen der allgemeinen Formel (XI) wie oben beschrieben nach Metallierung mit Schwefel umsetzt, dann - gegebenenfalls nach Zwischenisolierung - das hierbei gebildete Produkt der allgemeinen Formel (XIV) 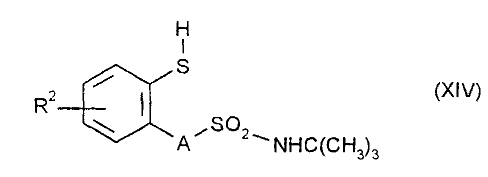 in welcher
- A und R²: die oben angegebene Bedeutung haben,
mit einer starken Säure, wie z.B. Trifluoressigsäure, gegebenenfalls in Gegenwart eines Verdünnungsmittels, wie z.B. Methylenchlorid, bei Temperaturen zwischen 0°C und 50°C umsetzt und das so erhaltene Isomerisierungsprodukt der allgemeinen Formel (XV) 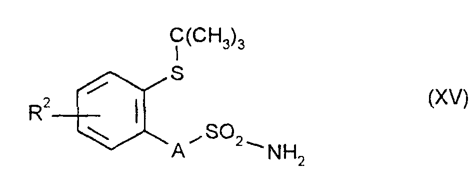 in welcher
- A und R²: die oben angegebene Bedeutung haben,
- gegebenenfalls nach Zwischenisolierung - mit einer Lewis-Säure, wie z.B. Bor(III)-bromid, in Gegenwart eines Verdünnungsmittels, wie z.B. Methylenchlorid, bei Temperaturen zwischen 0°C und 50°C umsetzt (vgl. die Herstellungsbeispiele).

Man erhält die neuen Verbindungen der Formel (II), in welcher n für Null steht, A für eine Einfachbindung steht und R¹ für Wasserstoff oder für jeweils gegebenenfalls substituiertes Alkyl, Alkenyl, Alkinyl oder Cycloalkyl steht, wenn man die t-Butylaminosulfonylverbindungen der allgemeinen Formel (XI) wie oben beschrieben nach Metallierung mit Schwefel umsetzt, dann - gegebenenfalls nach Zwischenisolierung - die hierbei gebildeten Produkte der allgemeinen Formel (XIV) - oben - mit einem geeigneten Oxidationsmittel, wie z.B. Dimethylsulfoxid, bei Temperaturen zwischen 20°C und 120°C umsetzt, dann - gegebenenfalls nach Zwischenisolierung - die so gebildeten Disulfide der allgemeinen Formel (XVI) 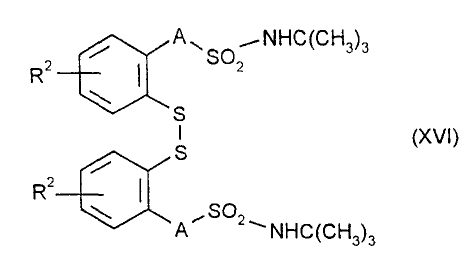 in welcher
- A und R²: die oben angegebene Bedeutung haben,
mit einer starken Säure, wie z.B. Trifluoressigsäure, gegebenenfalls in Gegenwart eines Verdünnungsmittels, wie z.B. Methylenchlorid, bei Temperaturen zwischen 0°C und 50°C umsetzt, dann - gegebenenfalls nach Zwischenisolierung - die hierbei gebildeten Disulfide der allgemeinen Formel (XVII) 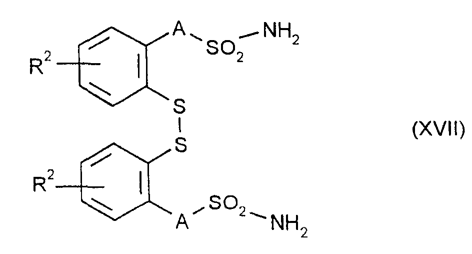 in welcher
- A und R²: die oben angegebene Bedeutung haben,
mit einem Reduktionsmittel, wie z.B. Natriumtetrahydridoborat (Natriumboranat), gegebenenfalls in Gegenwart eines Verdünnungsmittels, wie z.B. Methanol, bei Temperaturen zwischen 0°C und 50°C umsetzt und gegebenenfalls dann die so erhaltenen Verbindungen der Formel (II), in welcher R¹ für Wasserstoff steht, mit einem Alkylierungsmittel der allgemeinen Formel (XII)

X¹-R¹ (XII)

in welcher
- R¹: für jeweils gegebenenfalls substituiertes Alkyl, Alkenyl, Alkinyl oder Cycloalkyl steht und
- X¹: für Halogen, vorzugsweise Chlor, Brom oder Iod steht,
bei Temperaturen zwischen 0°C und 100°C umsetzt (vgl. die Herstellungsbeispiele).

Man erhält die neuen Aminosulfonylverbindungen der Formel (II), in welcher A für eine Einfachbindung steht, R¹ für jeweils gegebenenfalls substituiertes Alkyl, Alkenyl, Alkinyl oder Cycloalkyl steht und R² für jeweils gegebenenfalls substituiertes Alkyl, Alkenyl oder Alkinyl - in 6-Position - steht, wenn man t-Butylaminosulfonylverbindungen der allgemeinen Formel (XVIII) 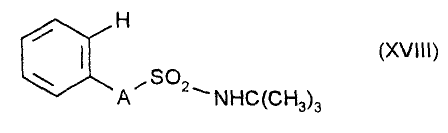 in welcher
- A: die oben angegebene Bedeutung hat,
mit einer metallorganischen Verbindung, wie z.B. Butyllithium in Hexan, gegebenenfalls in Gegenwart eines (weiteren) inerten Verdünnungsmittels, wie z.B. Tetrahydrofuran, und unter Inertgasatmosphäre, wie z.B. unter Argon, bei Temperaturen zwischen -50°C und +20°C metalliert - d.h. das in der Formel (XVIII) eingezeichnete Wasserstoffatom durch ein Metallatom ersetzt - dann im gleichen Reaktionsmedium mit Schwefel bei Temperaturen zwischen -30°C und +30°C umsetzt - d.h. das Metallatom durch Schwefel ersetzt - dann im gleichen Reaktionsmedium mit einem Alkylierungsmittel der allgemeinen Formel (XII)

X¹-R¹ (XII)

in welcher
- R¹: für jeweils gegebenenfalls substituiertes Alkyl, Alkenyl, Alkinyl oder Cycloalkyl steht und
- X¹: für Halogen, vorzugsweise Chlor, Brom oder Iod steht,
bei Temperaturen zwischen 0°C und 100°C umsetzt und dann - vorzugsweise nach Zwischenisolierung - die so erhaltenen t-Butylaminosulfonylverbindungen der allgemeinen Formel (XIX) 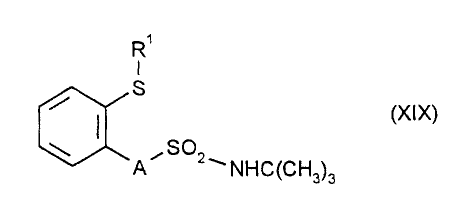 in welcher
- A und R¹: die oben angegebene Bedeutung haben,
mit einer metallorganischen Verbindung, wie z.B. Butyllithium in Hexan, gegebenenfalls in Gegenwart eines (weiteren) inerten Verdünnungsmittels, wie z.B. Tetrahydrofuran, und unter Inertgasatmosphäre, wie z.B. unter Argon, bei Temperaturen zwischen -50°C und +20°C metalliert und dann im gleichen Reaktionsmedium mit einem Alkylierungsmittel der allgemeinen Formel (XX)

X²-R² (XX)

in welcher
- R²: für jeweils gegebenenfalls substituiertes Alkyl, Alkenyl oder Alkinyl steht und
- X²: für Halogen, vorzugsweise Chlor, Brom oder Iod steht,
bei Temperaturen zwischen 0°C und 100°C umsetzt (vgl. die Herstellungsbeispiele).

Die Verbindungen der Formel (II), in welcher n für Null steht, können durch Umsetzung mit geeigneten Oxidationsmitteln, wie z.B. 3-Chlor-perbenzoesäure, auf übliche Weise in entsprechende Verbindungen der Formel (II), in welcher n für 1 oder 2 steht, umgewandelt werden (vgl. die Herstellungsbeispiele).

Einige der als Vorprodukte benötigten n-Butylaminosulfonylverbindungen der Formel (XIII) - oben - können auch durch Umsetzung von geeigneten Disulfiden der Formel (XVI) - oben - mit geeigneten Alkylierungsmitteln der Formel (XII) - oben - in Gegenwart von Natrium-Hydroxymethansulfinat-Dihydrat und in Gegenwart von Dinatriumhydrogenphosphat sowie in Gegenwart eines Verdünnungsmittels, wie z.B. N,N-Dimethyl-formamid, erhalten werden (vgl. die Herstellungsbeispiele).

Die beim erfindungsgemäßen Verfahren (a) zur Herstellung der Verbindungen der Formel (I) weiter als Ausgangsstoffe zu verwendenden (Thio)Carbonsäurederivate sind durch die Formel (III) allgemein definiert. In der Formel (III) haben Q und R³ vorzugsweise bzw. insbesondere diejenige Bedeutung, die bereits oben bei der Beschreibung der erfindungsgemäßen Verbindungen der Formel (I) vorzugsweise bzw. als insbesondere bevorzugt für Q und R³ angegeben wurde; Z steht vorzugsweise für Fluor, Chlor, Brom, C₁-C₄-Alkoxy, Phenoxy oder Benzyloxy, insbesondere für Chlor, Methoxy, Ethoxy oder Phenoxy.

Die Ausgangsstoffe der Formel (III) sind bekannt und/oder können nach an sich bekannten Verfahren hergestellt werden (vgl. EP 459244, EP 341489, EP 422469, EP 425948, EP 431291, EP 507171, EP 534266).

Die beim erfindungsgemäßen Verfahren (b) zur Herstellung der Verbindungen der Formel (I) als Ausgangsstoffe zu verwendenden Sulfonyliso(thio)cyanate sind durch die Formel (IV) allgemein definiert. In der Formel (IV) haben n, A, Q, R¹ und R² vorzugsweise bzw. insbesondere diejenige Bedeutung, die bereits oben bei der Beschreibung der erfindungsgemäßen Verbindungen der Formel (I) vorzugsweise bzw. als insbesondere bevorzugt für n, A, Q, R¹ und R² angegeben wurde.

Die Ausgangsstoffe der Formel (IV) sind teilweise (vgl. EP 135332, EP 23 141, EP 23 442 A aus der Literatur bekannt.

Man erhält die neuen Sulfonyliso(thio)cyanate der Formel (IV), wenn man Aminosulfonylverbindungen der allgemeinen Formel (II) - oben - mit Phosgen bzw. Thiophosgen, gegebenenfalls in Gegenwart eines Alkylisocyanats, wie z.B. Butylisocyanat, gegebenenfalls in Gegenwart eines Reaktionshilfsmittels, wie z.B.

Diazabicyclo[2.2.2]octan, und in Gegenwart eines Verdünnungsmittels, wie z.B. Toluol, Xylol oder Chlorbenzol, bei Temperaturen zwischen 80°C und 150°C umsetzt und nach Ende der Umsetzung die flüchtigen Komponenten unter vermindertem Druck abdestilliert.

Die bei den erfindungsgemäßen Verfahren (b), (c), (e) und (f) zur Herstellung der Verbindungen der Formel (I) weiter als Ausgangsstoffe zu verwendenden Heterocyclen sind durch die Formel (V) allgemein definiert. In der Formel (V) hat R³ vorzugsweise bzw. insbesondere diejenige Bedeutung, die bereits oben bei der Beschreibung der erfindungsgemäßen Verbindungen der Formel (I) vorzugsweise bzw. als insbesondere bevorzugt für R³ angegeben wurde.

Die Ausgangsstoffe der Formel (V) sind bekannt und/oder können nach an sich bekannten Verfahren hergestellt werden (vgl. EP 341489, EP 422469, EP 425948, EP 431291, EP 507171, EP 534266).

Die bei den erfindungsgemäßen Verfahren (c) und (d) zur Herstellung der Verbindungen der Formel (I) als Ausgangsstoffe zu verwendenden Chlorsulfonylverbindungen sind durch die Formel (VI) allgemein definiert. In der Formel (VI) haben n, A, R¹ und R² vorzugsweise bzw. insbesondere diejenige Bedeutung, die bereits oben bei der Beschreibung der erfindungsgemäßen Verbindungen der Formel (I) vorzugsweise bzw. als insbesondere bevorzugt für n, A, R¹ und R² angegeben wurde.

Die Ausgangsstoffe der Formel (VI) sind teilweise aus der Literatur bekannt (EP 23 141 A; EP 23 422 A; US 4 645 527; DE 3 6234 103 A; Verbindungen mit Beilstein Registry No. 2 648 548 und 3 277 821; Coll. Czech. Chem. Com., 47, 1, 1982, 72-87; CAS, Vol. 96. No. 13, 100769 h; CAS, Vol. 85, No. 19, 142812 v).

Man erhält die neuen Chlorsulfonylverbindungen der Formel (VI), wenn man entsprechende Aminoverbindungen der allgemeinen Formel (XXI) 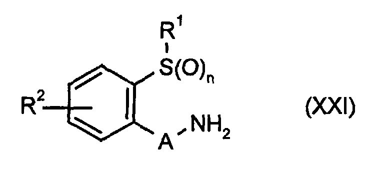 in welcher
- n, A, R¹ und R²: die oben angegebene Bedeutung haben,
mit einem Alkalimetallnitrit, wie z.B. Natriumnitrit, in Gegenwart von Salzsäure bei Temperaturen zwischen -10°C und +10°C umsetzt und die so erhaltene Diazoniumsalzlösung mit Schwefeldioxid in Gegenwart eines Verdünnungsmittels, wie z.B. Dichlormethan, 1,2-Dichlor-ethan oder Essigsäure, und in Gegenwart eines Katalysators, wie z.B. Kupfer-(I)-chlorid und/oder Kupfer(II)-chlorid, bei Temperaturen zwischen -10°C und +50°C umsetzt.

Die beim erfindungsgemäßen Verfahren (d) zur Herstellung der Verbindungen der Formel (I) als Ausgangsstoffe zu verwendenden (Thio)Carbonsäureamide sind durch die Formel (VIII) allgemein definiert. In der Formel (VIII) haben Q und R³ vorzugsweise bzw. insbesondere diejenige Bedeutung, die bereits oben bei der Beschreibung der erfindungsgemäßen Verbindungen der Formel (I) vorzugsweise bzw. als insbesondere bevorzugt für Q und R³ angegeben wurde.

Die Ausgangsstoffe der Formel (VIII) sind bekannt und/oder können nach an sich bekannten Verfahren hergestellt werden (vgl. EP 459244).

Die beim erfindungsgemäßen Verfahren (e) zur Herstellung der Verbindungen der Formel (I) als Ausgangsstoffe zu verwendenden Sulfonylamino(thio)carbonylverbindungen sind durch die Formel (IX) allgemein definiert. In der Formel (IX) haben n, A, Q, R¹ und R² vorzugsweise bzw. insbesondere diejenige Bedeutung, die bereits oben bei der Beschreibung der erfindungsgemäßen Verbindungen der Formel (I) vorzugsweise bzw. als insbesondere bevorzugt für n, A, Q, R¹ und R² angegeben wurde; Z steht vorzugsweise für Fluor, Chlor, Brom, C₁ -C₄-Alkoxy, Phenoxy oder Benzyloxy, insbesondere für Chlor, Methoxy, Ethoxy oder Phenoxy.

Die Ausgangsstoffe der Formel (IX) sind bekannt und/oder können nach an sich bekannten Verfahren hergestellt werden.

Die beim erfindungsgemäßen Verfahren (f) zur Herstellung der Verbindungen der Formel (I) als Ausgangsstoffe zu verwendenden Benzolderivate sind durch die Formel (X) allgemein definiert. In der Formel (X) haben n, A, R¹ und R² vorzugsweise bzw. insbesondere diejenige Bedeutung, die bereits oben bei der Beschreibung der erfindungsgemäßen Verbindungen der Formel (I) vorzugsweise bzw. als insbesondere bevorzugt für n, A, R¹ und R² angegeben wurde.

Die Ausgangsstoffe der Formel (X) sind bekannt und/oder können nach an sich bekannten Verfahren hergestellt werden.

Die erfindungsgemäßen Verfahren (a), (b), (c), (d), (e) und (f) zur Herstellung der neuen Verbindungen der Formel (I) werden vorzugsweise unter Verwendung von Verdünnungsmitteln durchgeführt. Als Verdünnungsmittel kommen dabei praktisch alle inerten organischen Lösungsmittel infrage. Hierzu gehören vorzugsweise aliphatische und aromatische, gegebenenfalls halogenierte Kohlenwasserstoffe wie Pentan, Hexan, Heptan, Cyclohexan, Petrolether, Benzin, Ligroin, Benzol, Toluol, Xylol, Methylenchlorid, Ethylenchlorid, Chloroform, Tetrachlormethan, Chlorbenzol und o-Dichlorbenzol; Ether wie Diethyl- und Dibutylether, Glykoldimethylether und Diglykoldimethylether, Tetrahydrofuran und Dioxan; Ketone wie Aceton, Methyl-ethyl-, Methyl-isopropyl- und Methyl-isobutyl-keton; Ester wie Essigssäuremethylester und -ethylester; Nitrile wie z.B. Acetonitril und Propionitril; Amide wie z.B. Dimethylformamid, Dimethylacetamid und N-Methylpyrrolidon sowie Dimethylsulfoxid, Tetramethylensulfon und Hexamethylphosphorsäuretriamid.

Als Reaktionshilfsmittel bzw. als Säureakzeptoren können bei den erfindungsgemäßen Verfahren (a), (b), (c), (d), (e) und (f) alle üblicherweise für derartige Umsetzungen verwendbaren Säurebindemittel eingesetzt werden. Vorzugsweise in Frage kommen Alkalimetallhydroxide wie z.B. Natrium- und Kaliumhydroxid, Erdalkalihydroxide wie z.B. Calciumhydroxid, Alkalicarbonate und -alkoholate wie Natrium- und Kaliumcarbonat, Natrium- und Kalium-tert-butylat, ferner basische Stickstoffverbindungen, wie Trimethylamin, Triethylamin, Tripropylamin, Tributylamin, Diisobutylamin, Dicyclohexylamin, Ethyldiisopropylamin, Ethyldicyclohexylamin, N,N-Dimethylbenzylamin, N,N-Dimethyl-anilin, Pyridin, 2-Methyl-, 3-Methyl-, 4-Methyl-, 2,4-Dimethyl-, 2,6-Dimethyl-, 2-Ethyl-, 4-Ethyl- und 5-Ethyl-2-methyl-pyridin, 1,5-Diazabicyclo[4,3,0]-non-5-en (DBN), 1,8-Diazabicyclo-[5,4,0]-undec-7-en (DBU) und 1,4-Diazabicyclo-[2,2,2]-octan (DABCO).

Die Reaktionstemperaturen können bei den erfindungsgemäßen Verfahren (a), (b), (c), (d), (e) und (f) in einem größeren Bereich variiert werden. Im allgemeinen arbeitet man bei Temperaturen zwischen -20°C und +150°C, vorzugsweise bei Temperaturen zwischen 0°C und +100°C.

Die erfindungsgemäßen Verfahren (a), (b), (c), (d), (e) und (f) werden im allgemeinen unter Normaldruck durchgeführt. Es ist jedoch auch möglich, unter erhöhtem oder vermindertem Druck zu arbeiten.

Zur Durchführung der erfindungsgemäßen Verfahren (a), (b), (c), (d), (e) und (f) werden die jeweils benötigten Ausgangsstoffe im allgemeinen in angenähert äquimolaren Mengen eingesetzt. Es ist jedoch auch möglich, eine der jeweils eingesetzten Komponenten in einem größeren Überschuß zu verwenden. Die Reaktionen werden im allgemeinen in einem geeigneten Verdünnungsmittel in Gegenwart eines Säureakzeptors durchgeführt, und das Reaktionsgemisch wird mehrere Stunden bei der jeweils erforderlichen Temperatur gerührt. Die Aufarbeitung erfolgt bei den erfindungsgemäßen Verfahren (a), (b), (c), (d), (e) und (f) jeweils nach üblichen Methoden (vgl. die Herstellungsbeispiele).

Aus den erfindungsgemäßen Verbindungen der allgemeinen Formel (I) können gegebenenfalls Salze hergestellt werden. Man erhält solche Salze in einfacher Weise nach üblichen Salzbildungsmethoden, beispielsweise durch Lösen oder Dispergieren einer Verbindung der Formel (I) in einem geeigneten Lösungsmittel, wie z.B. Methylenchlorid, Aceton, tert-Butyl-methylether oder Toluol, und Zugabe einer geeigneten Base. Die Salze können dann - gegebenenfalls nach längerem Rühren - durch Einengen oder Absaugen isoliert werden.

Die erfindungsgemäßen Wirkstoffe können als Defoliants, Desiccants, Krautabtötungsmittel und insbesondere als Unkrautvernichtungsmittel verwendet werden. Unter Unkraut im weitesten Sinne sind alle Pflanzen zu verstehen, die an Orten aufwachsen, wo sie unerwünscht sind. Ob die erfindungsgemäßen Stoffe als totale oder selektive Herbizide wirken, hängt im wesentlichen von der angewendeten Menge ab.

Die erfindungsgemäßen Wirkstoffe können z.B. bei den folgenden Pflanzen verwendet werden:
Dikotyle Unkräuter der Gattungen: Sinapis, Lepidium, Galium, Stellaria, Matricaria, Anthemis, Galinsoga, Chenopodium, Urtica, Senecio, Amaranthus, Portulaca, Xanthium, Convolvulus, Ipomoea, Polygonum, Sesbania, Ambrosia, Cirsium, Carduus, Sonchus, Solanum, Rorippa, Rotala, Lindernia, Lamium, Veronica, Abutilon, Emex, Datura, Viola, Galeopsis, Papaver, Centaurea, Trifolium, Ranunculus, Taraxacum.
Dikotyle Kulturen der Gattungen: Gossypium, Glycine, Beta, Daucus, Phaseolus, Pisum, Solanum, Linum, Ipomoea, Vicia, Nicotiana, Lycopersicon, Arachis, Brassica, Lactuca, Cucumis, Cucurbita.
Monokotyle Unkräuter der Gattungen: Echinochloa, Setaria, Panicum, Digitaria, Phleum, Poa, Festuca, Eleusine, Brachiaria, Lolium, Bromus, Avena, Cyperus, Sorghum, Agropyron, Cynodon, Monochoria, Fimbristylis, Sagittaria, Eleocharis, Scirpus, Paspalum, Ischaemum, Sphenoclea, Dactyloctenium, Agrostis, Alopecurus, Apera.
Monokotyle Kulturen der Gattungen: Oryza, Zea, Triticum, Hordeum, Avena, Secale, Sorghum, Panicum, Saccharum, Ananas, Asparagus, Allium.

Die Verwendung der erfindungsgemäßen Wirkstoffe ist jedoch keineswegs auf diese Gattungen beschränkt, sondern erstreckt sich in gleicher Weise auch auf andere Pflanzen.

Die Verbindungen eignen sich in Abhängigkeit von der Konzentration zur Totalunkrautbekämpfung z.B. auf Industrie- und Gleisanlagen und auf Wegen und Plätzen mit und ohne Baumbewuchs. Ebenso können die Verbindungen zur Unkrautbekämpfung in Dauerkulturen, z.B. Forst, Ziergehölz-, Obst-, Wein-, Citrus-, Nuß-, Bananen-, Kaffee-, Tee-, Gummi-, Ölpalm-, Kakao-, Beerenfruchtund Hopfenanlagen, auf Zier- und Sportrasen und Weideflächen und zur selektiven Unkrautbekämpfung in einjährigen Kulturen eingesetzt werden.

Die erfindungsgemäßen Verbindungen der Formel (I) eignen sich zur Bekämpfung von monokotylen und dikotylen Unkräutern sowohl im Vorauflauf- als auch im Nachauflauf-Verfahren. Sie zeigen starke herbizide Aktivität und ein breites Wirkungsspektrum bei Anwendung auf den Boden und auf oberirdische Pflanzenteile.

Die Wirkstoffe können in die üblichen Formulierungen übergeführt werden, wie Lösungen, Emulsionen, Spritzpulver, Suspensionen, Pulver, Stäubemittel, Pasten, lösliche Pulver, Granulate, Suspensions-Emulsions-Konzentrate, Wirkstoff-imprägnierte Natur- und synthetische Stoffe sowie Feinstverkapselungen in polymeren Stoffen.

Diese Formulierungen werden in bekannter Weise hergestellt, z. B. durch Vermischen der Wirkstoffe mit Streckmitteln, also flüssigen Lösungsmitteln und/oder festen Trägerstoffen, gegebenenfalls unter Verwendung von oberflächenaktiven Mitteln, also Emulgiermitteln und/oder Dispergiermitteln und/oder schaumerzeugenden Mitteln.

Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten und chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, mineralische und pflanzliche Öle, Alkohole, wie Butanol oder Glykol sowie deren Ether und Ester, Ketone wie Aceton, Methylethylketon, Methylisobutylketon oder Cyclohexanon, stark polare Lösungsmittel, wie Dimethylformamid und Dimethylsulfoxid, sowie Wasser.

Als feste Trägerstoffe kommen in Frage:

z.B. Ammoniumsalze und natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate, als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengeln; als Emulgier- und/oder schaumerzeugende Mittel kommen in Frage: z.B. nichtionogene und anionische Emulgatoren, wie Polyoxyethylen-Fettsäure-Ester, Polyoxyethylen-Fettalkohol-Ether, z.B. Alkylarylpolyglykolether, Alkylsulfonate, Alkylsulfate, Arylsulfonate sowie Eiweißhydrolysate; als Dispergiermittel kommen in Frage: z.B. Lignin-Sulfitablaugen und Methylcellulose.

Es können in den Formulierungen Haftmittel wie Carboxymethylcellulose, natürliche und synthetische pulvrige, körnige oder latexförmige Polymere verwendet werden, wie Gummiarabicum, Polyvinylalkohol, Polyvinylacetat, sowie natürliche Phospholipide, wie Kephaline und Lecithine und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Die erfindungsgemäßen Wirkstoffe können als solche oder in ihren Formulierungen auch in Mischung mit bekannten Herbiziden zur Unkrautbekämpfung Verwendung finden, wobei Fertigformulierungen oder Tankmischungen möglich sind.

Für die Mischungen kommen bekannte Herbizide infrage, beispielsweise Anilide, wie z.B. Diflufenican und Propanil; Arylcarbonsäuren, wie z.B. Dichlorpicolin-säure, Dicamba und Picloram; Aryloxyalkansäuren, wie z.B. 2,4-D, 2,4-DB, 2,4-DP, Fluroxypyr, MCPA, MCPP und Triclopyr; Aryloxy-phenoxyalkansäureester, wie z.B. Diclofop-methyl, Fenoxaprop-ethyl, Fluazifop-butyl, Haloxyfop-methyl und Quizalofop-ethyl; Azinone, wie z.B. Chloridazon und Norflurazon; Carbamate, wie z.B. Chlorpropham, Desmedipham, Phenmedipham und Propham; Chloracetanilide, wie z.B. Alachlor, Acetochlor, Butachlor, Metazachlor, Metolachlor, Pretilachlor und Propachlor; Dinitroaniline, wie z.B. Oryzalin, Pendimethalin und Trifluralin; Diphenylether, wie z.B. Acifluorfen, Bifenox, Fluoroglycofen, Fomesafen, Halosafen, Lactofen und Oxyfluorfen; Harnstoffe, wie z.B. Chlortoluron, Diuron, Fluometuron, Isoproturon, Linuron und Methabenzthiazuron; Hydroxylamine, wie z.B. Alloxydim, Clethodim, Cycloxydim, Sethoxydim und Tralkoxydim; Imidazolinone, wie z.B. Imazethapyr, Imazamethabenz, Imazapyr und Imazaquin; Nitrile, wie z.B. Bromoxynil, Dichlobenil und Ioxynil; Oxyacetamide, wie z.B. Mefenacet; Sulfonylharnstoffe, wie z.B. Amidosulfuron, Bensulfuron-methyl, Chlorimuron-ethyl, Chlorsulfuron, Cinosulfuron, Metsulfuron-methyl, Nicosulfuron, Primisulfuron, Pyrazosulfuronethyl, Thifensulfuron-methyl, Triasulfuron und Tribenuron-methyl; Thiolcarbamate, wie z.B. Butylate, Cycloate, Diallate, EPTC, Esprocarb, Molinate, Prosulfocarb,

Thiobencarb und Triallate; Triazine, wie z.B. Atrazin, Cyanazin, Simazin, Simetryne, Terbutryne und Terbutylazin; Triazinone, wie z.B. Hexazinon, Metamitron und Metribuzin; Sonstige, wie z.B. Aminotriazol, Benfuresate, Bentazone, Cinmethylin, Clomazone, Clopyralid, Difenzoquat, Dithiopyr, Ethofumesate, Fluorochloridone, Glufosinate, Glyphosate, Isoxaben, Pyridate, Quinchlorac, Quinmerac, Sulphosate und Tridiphane.

Auch eine Mischung mit anderen bekannten Wirkstoffen, wie Fungiziden, Insektiziden, Akariziden, Nematiziden, Schutzstoffen gegen Vogelfraß, Pflanzennährstoffen und Bodenstrukturverbesserungsmitteln ist möglich.

Die Wirkstoffe können als solche, in Form ihrer Formulierungen oder den daraus durch weiteres Verdünnen bereiteten Anwendungsformen, wie gebrauchsfertige Lösungen, Suspensionen, Emulsionen, Pulver, Pasten und Granulate angewandt werden. Die Anwendung geschieht in üblicher Weise, z.B. durch Gießen, Spritzen, Sprühen, Streuen.

Die erfindungsgemäßen Wirkstoffe können sowohl vor als auch nach dem Auflaufen der Pflanzen appliziert werden. Sie können auch vor der Saat in den Boden eingearbeitet werden.

Die angewandte Wirkstoffmenge kann in einem größeren Bereich schwanken. Sie hängt im wesentlichen von der Art des gewünschten Effektes ab. Im allgemeinen liegen die Aufwandmengen zwischen 1 g und 10 kg Wirkstoff pro Hektar Bodenfläche, vorzugsweise zwischen 5 g und 5 kg pro ha.

Die Herstellung und die Verwendung der erfindungsgemäßen Wirkstoffe geht aus den nachfolgenden Beispielen hervor.

### Herstellungsbeispiele:

### Beispiel 1

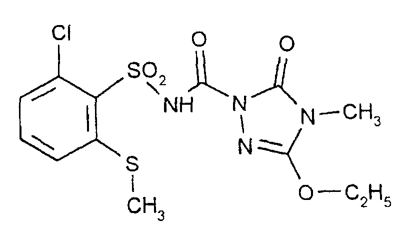

Eine Lösung von 3,3 g (14 mMol) 2-Chlor-6-methylthio-benzolsulfonamid, 3,7 g (14 mMol) 5-Ethoxy-4-methyl-2-phenoxycarbonyl-2,4-dihydro-3H-1,2,4-triazol-3-on und 2,3 g (15,4 mMol) Diazabicyclo[5.4.0]-undecen (DBU) in 30 ml Acetonitril wird sechs Stunden bei Raumtemperatur (ca. 20°C) gerührt. Danach wird das Lösungsmittel im Wasserstrahlvakuum abgezogen und der ölige Rückstand in 100 ml Methylenchlorid aufgenommen. Die Lösung wird nacheinander mit 1N-Salzsäure und gesättigter wässriger Kochsalzlösung gewaschen, über Natriumsulfat getrocknet und im Wasserstrahlvakuum vom Lösungsmittel befreit. Man erhält 6,6 g eines öligen Rückstands, der beim Verrühren mit 30 ml Ethanol kristallisiert. Nach Filtration und Trocknung im Vakuum bei 25°C erhält man 3,15 g (55,4 % der Theorie) 5-Ethoxy-4-methyl-2-(2-Chlor-6-methylthio-phenylsulfonyl-aminocarbonyl)-2,4-dihydro-3H-1,2,4-triazol-3-on vom Schmelzpunkt 144°C.

Analog Herstellungsbeispiel 1 sowie entsprechend der allgemeinen Beschreibung der erfindungsgemäßen Herstellungsverfahren können beispielsweise auch die in der nachstehenden Tabelle 1 aufgeführten Verbindungen der Formel (I) hergestellt werden.

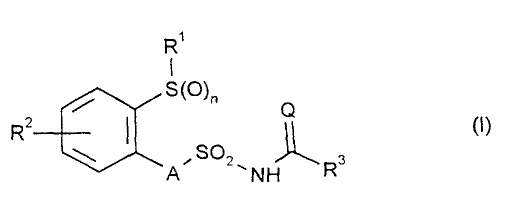

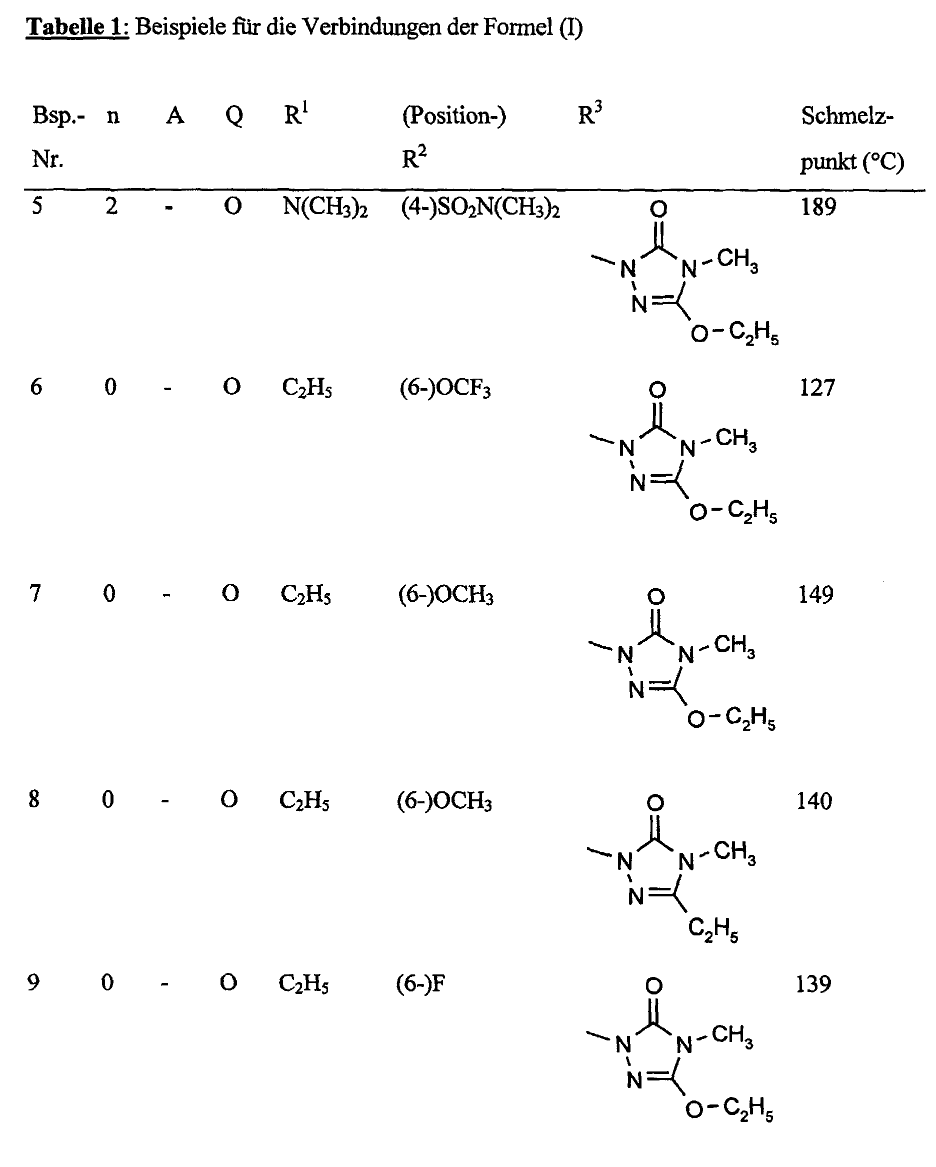

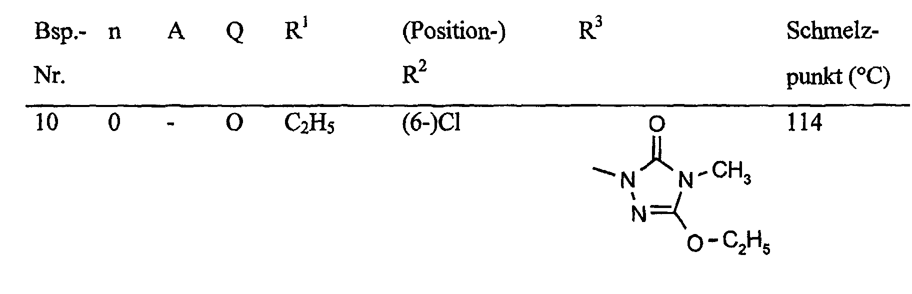

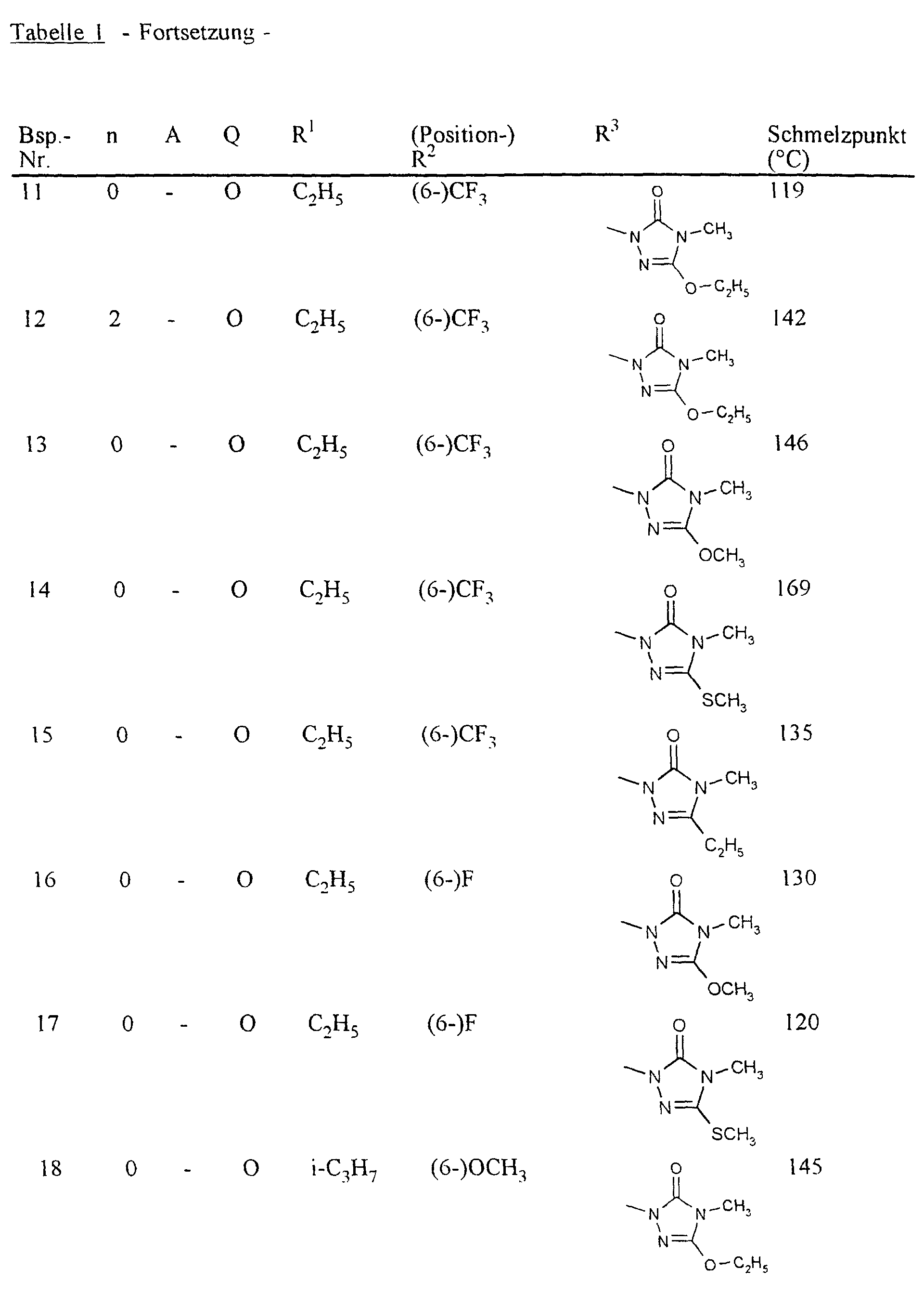

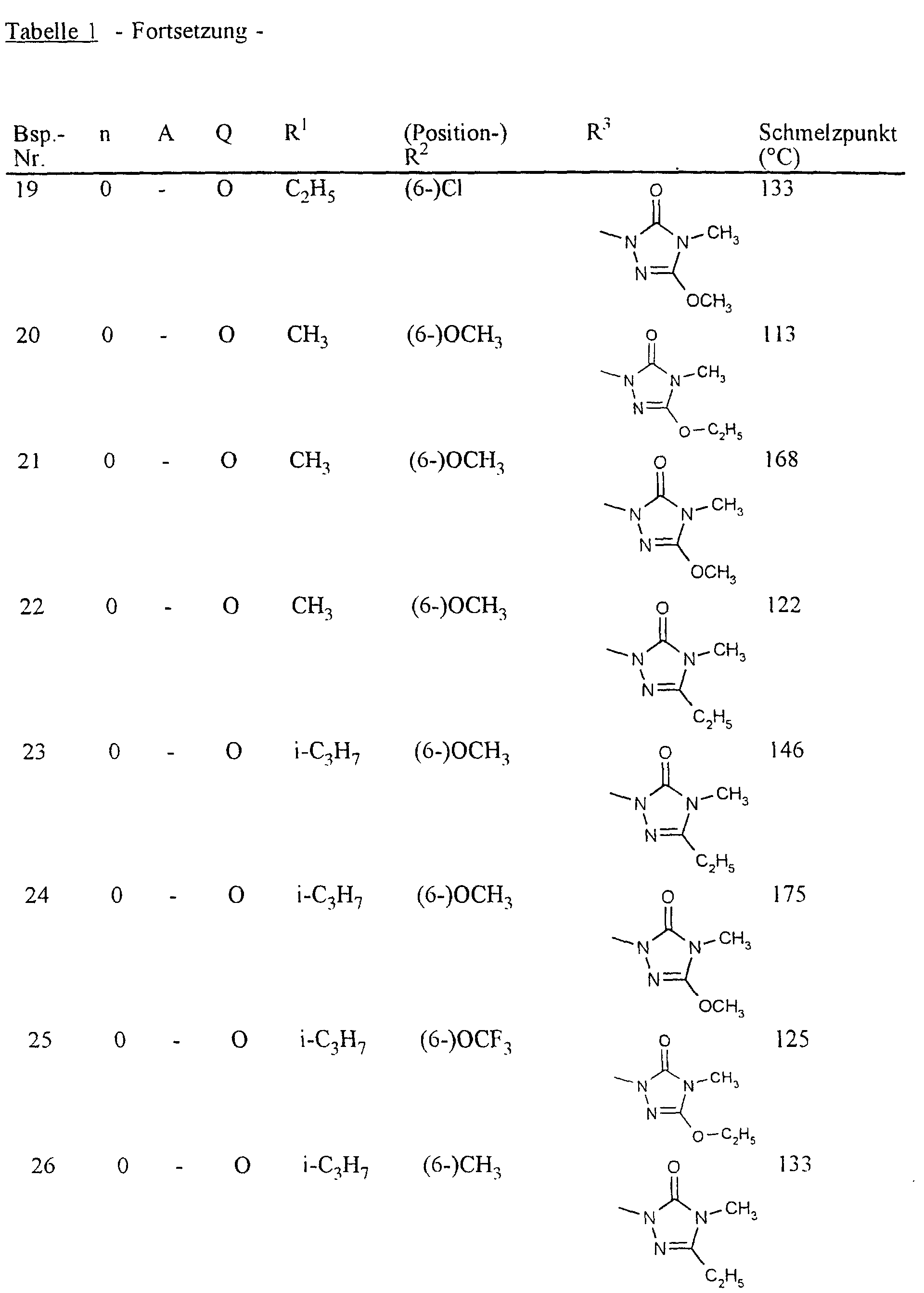

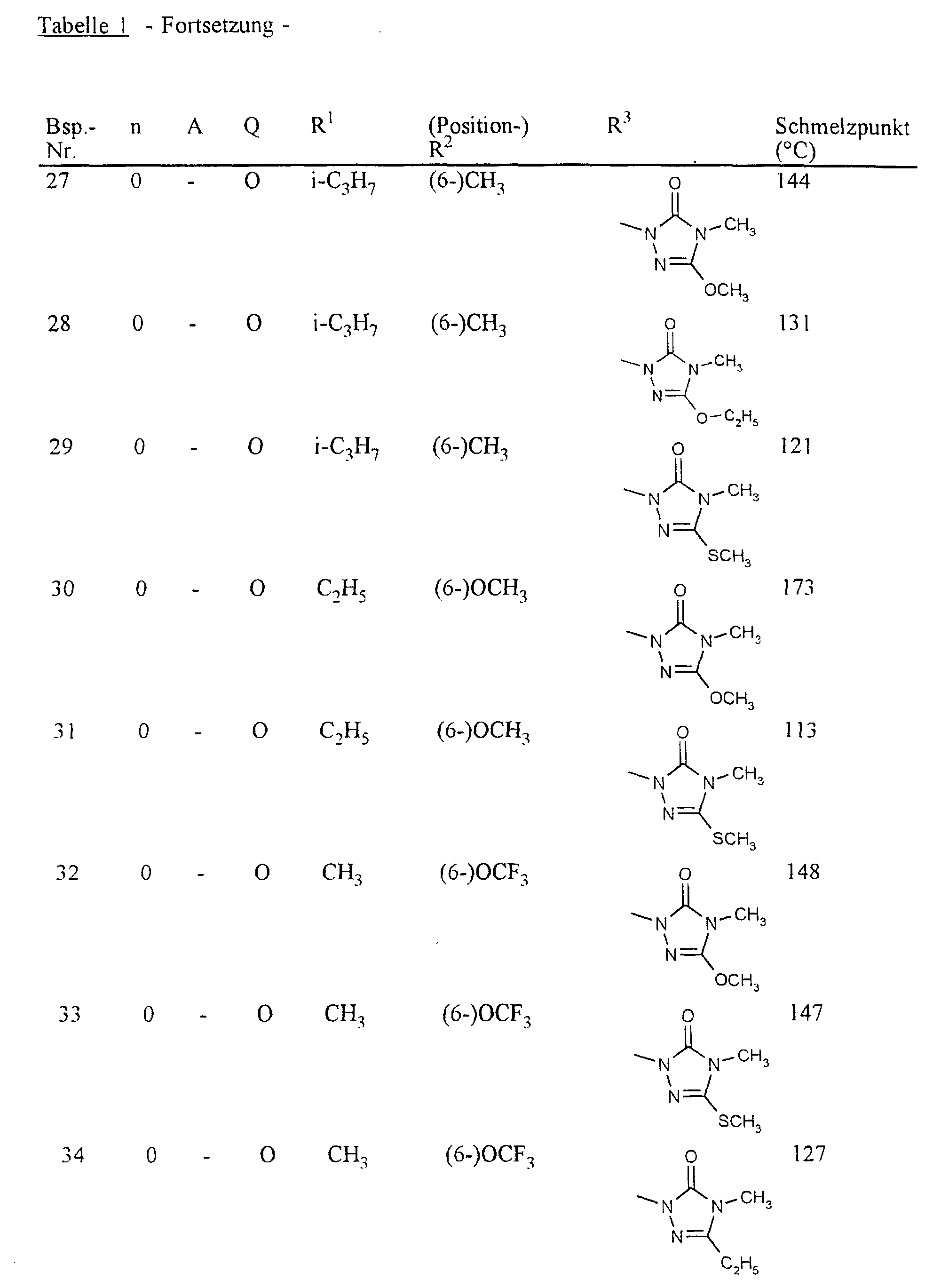

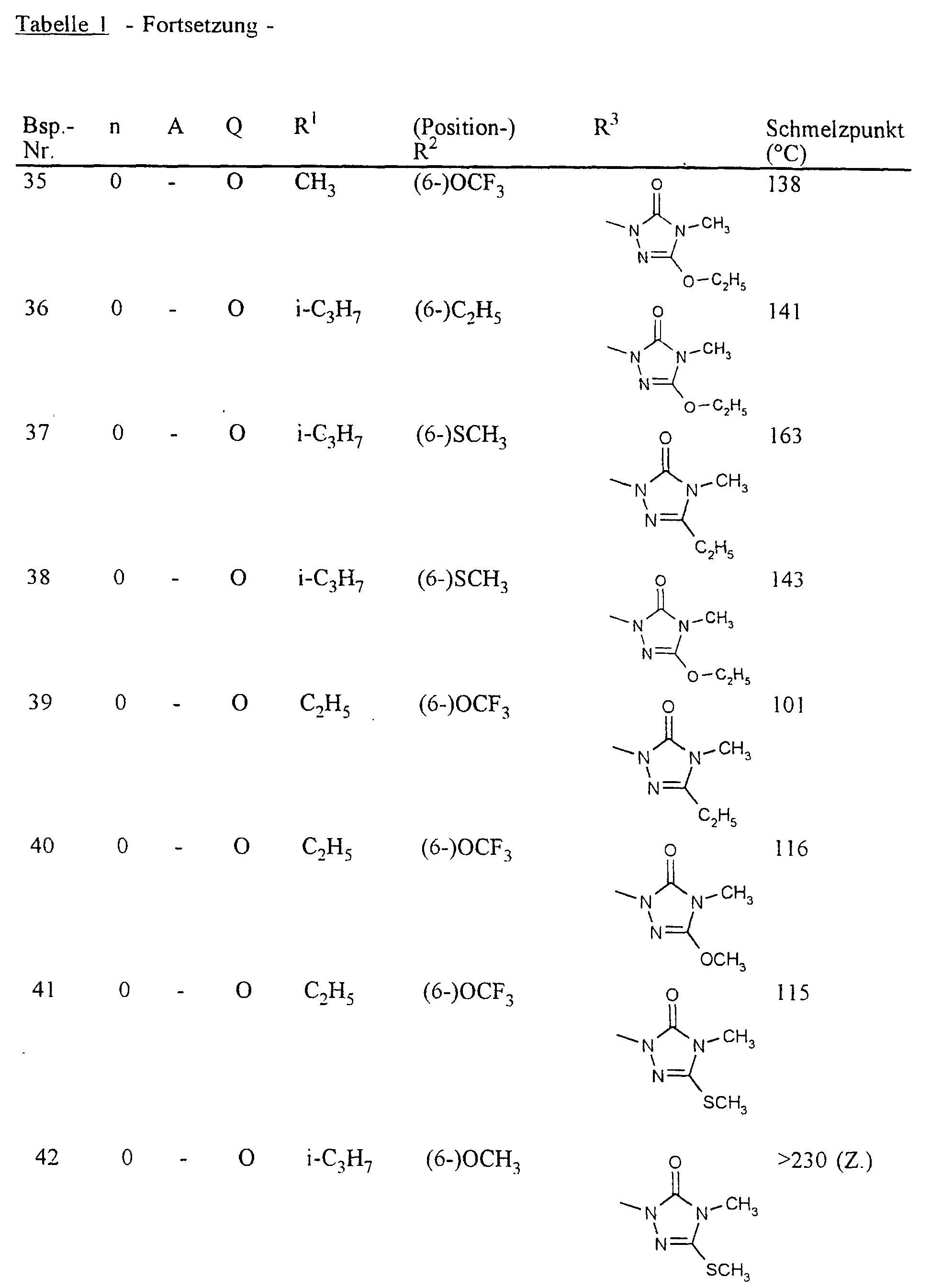

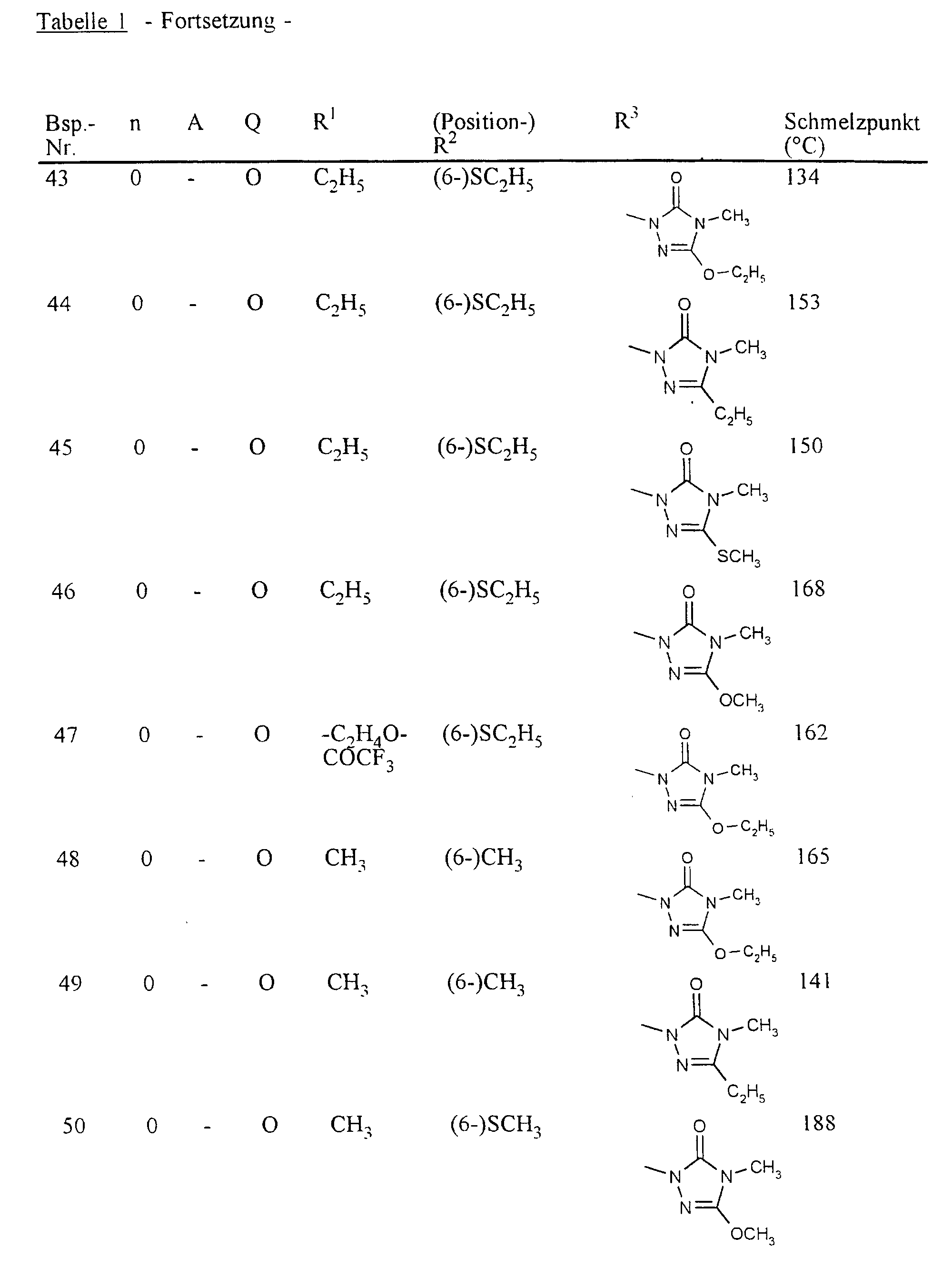

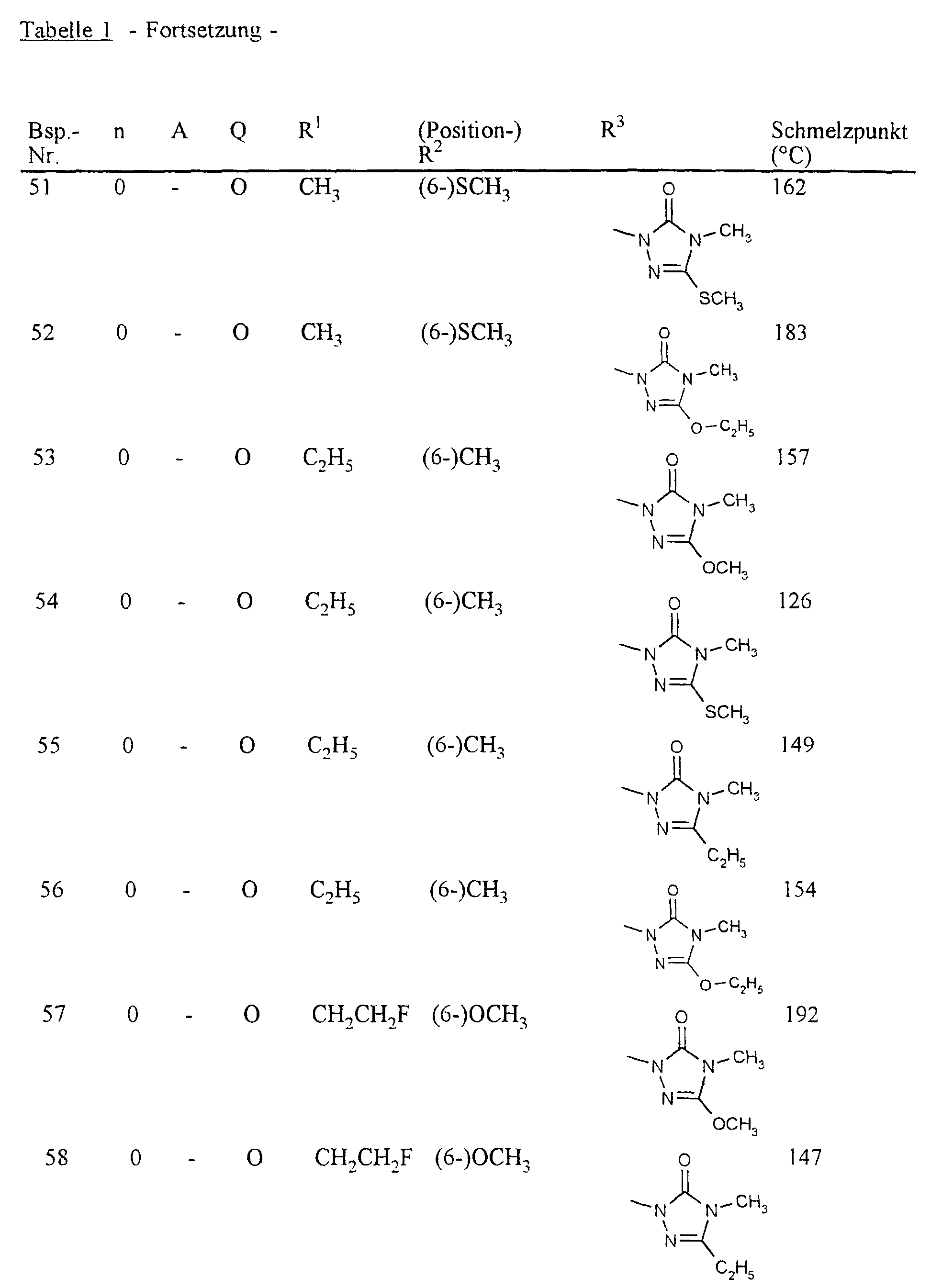

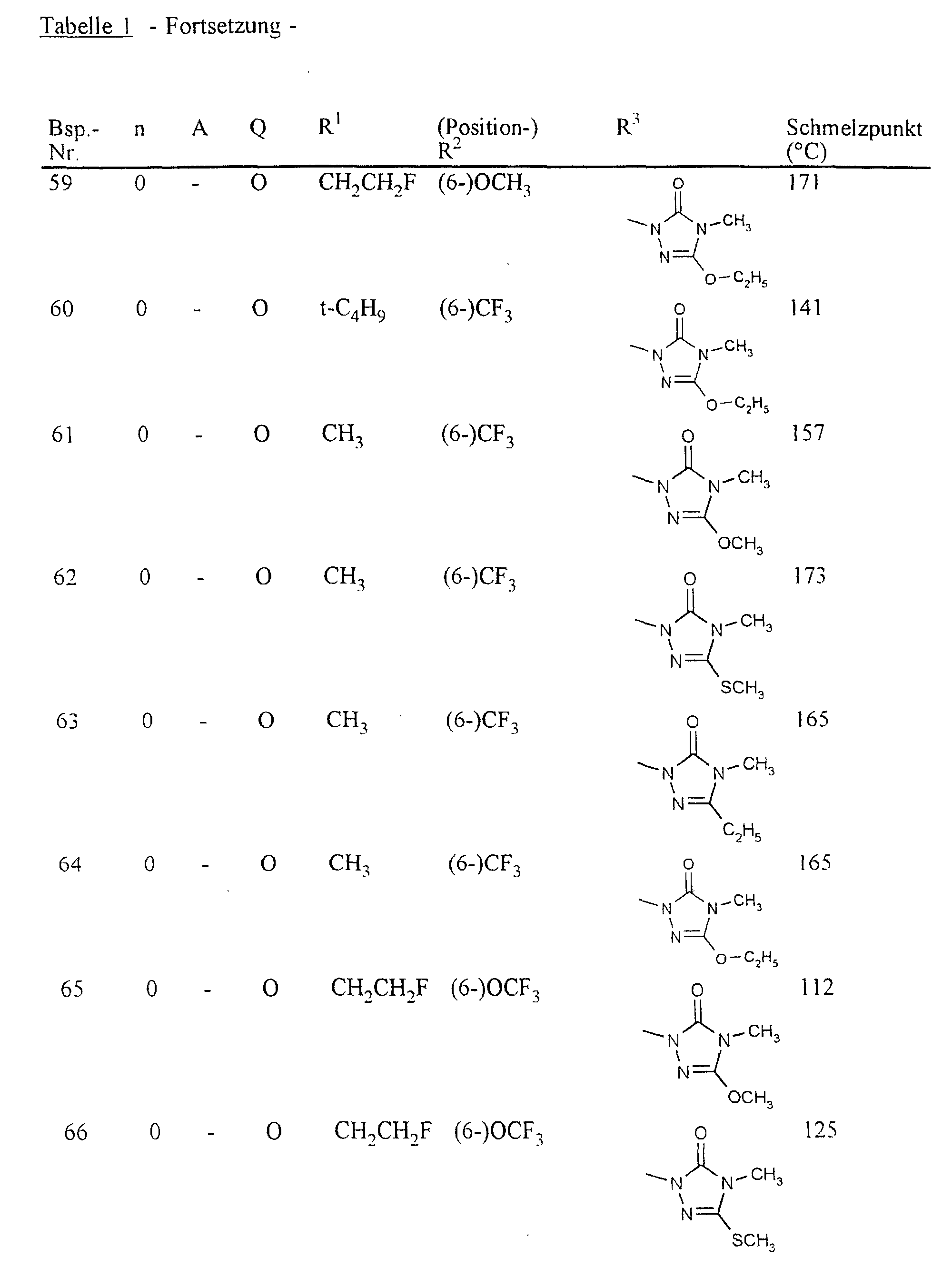

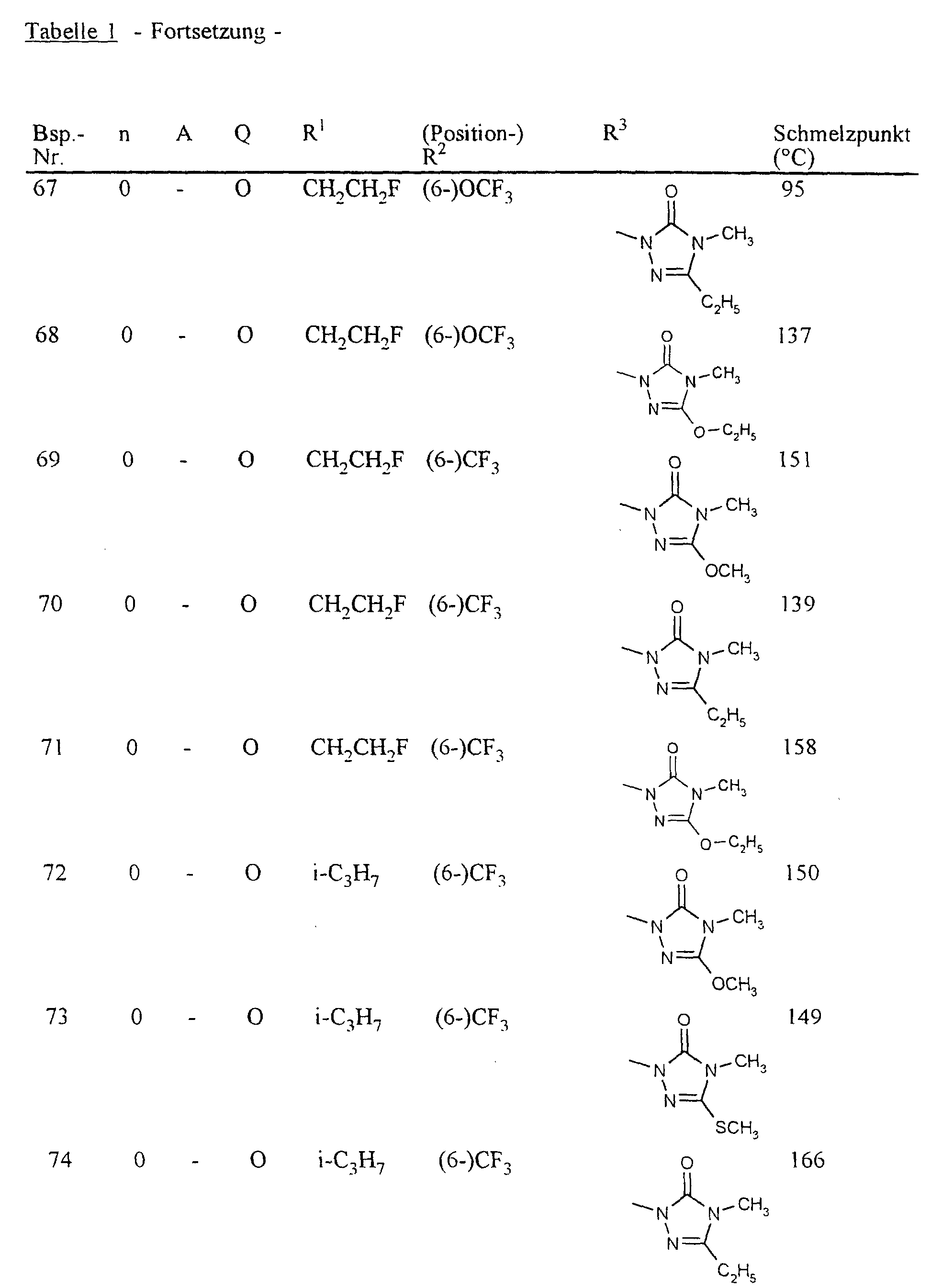

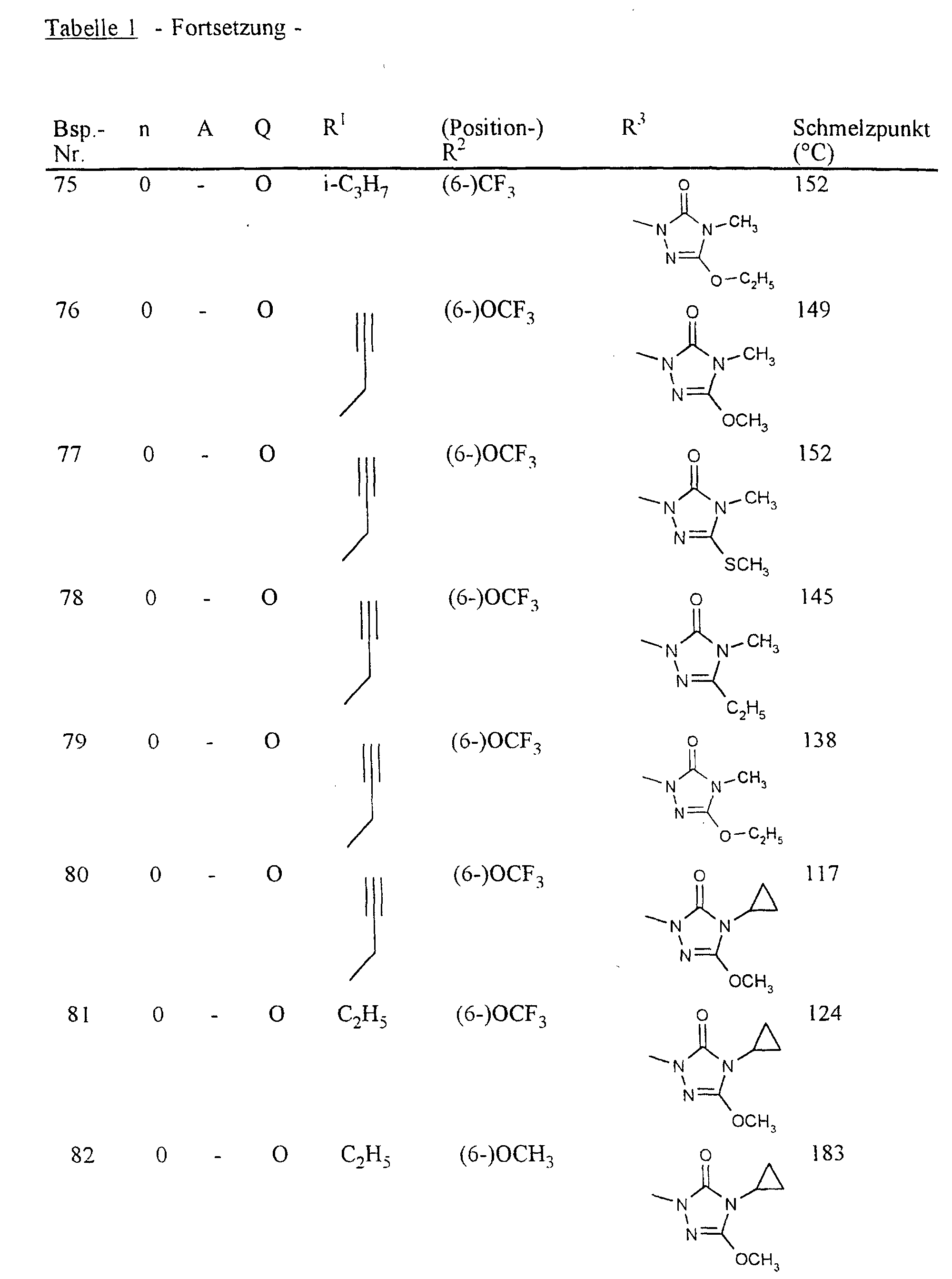

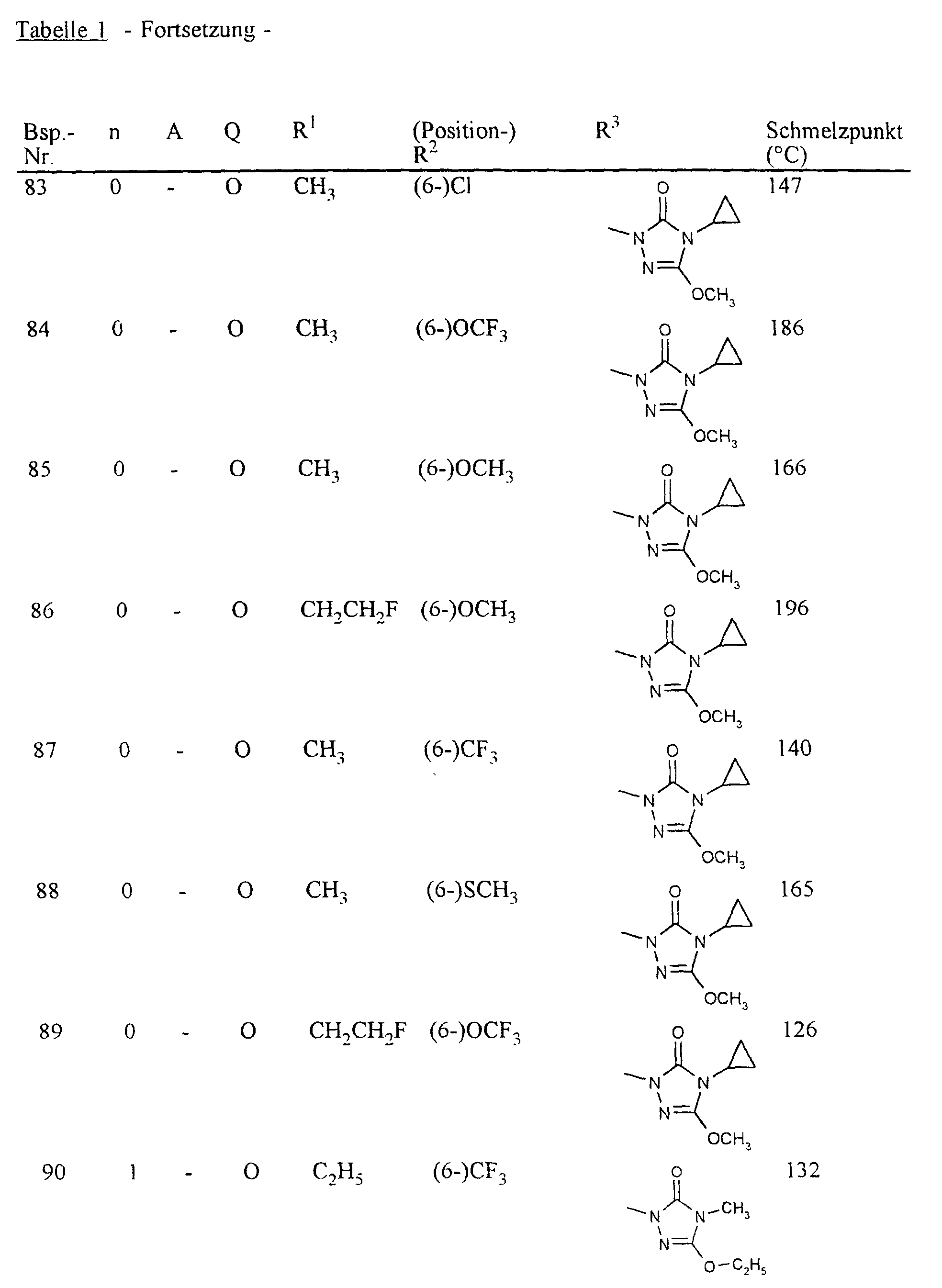

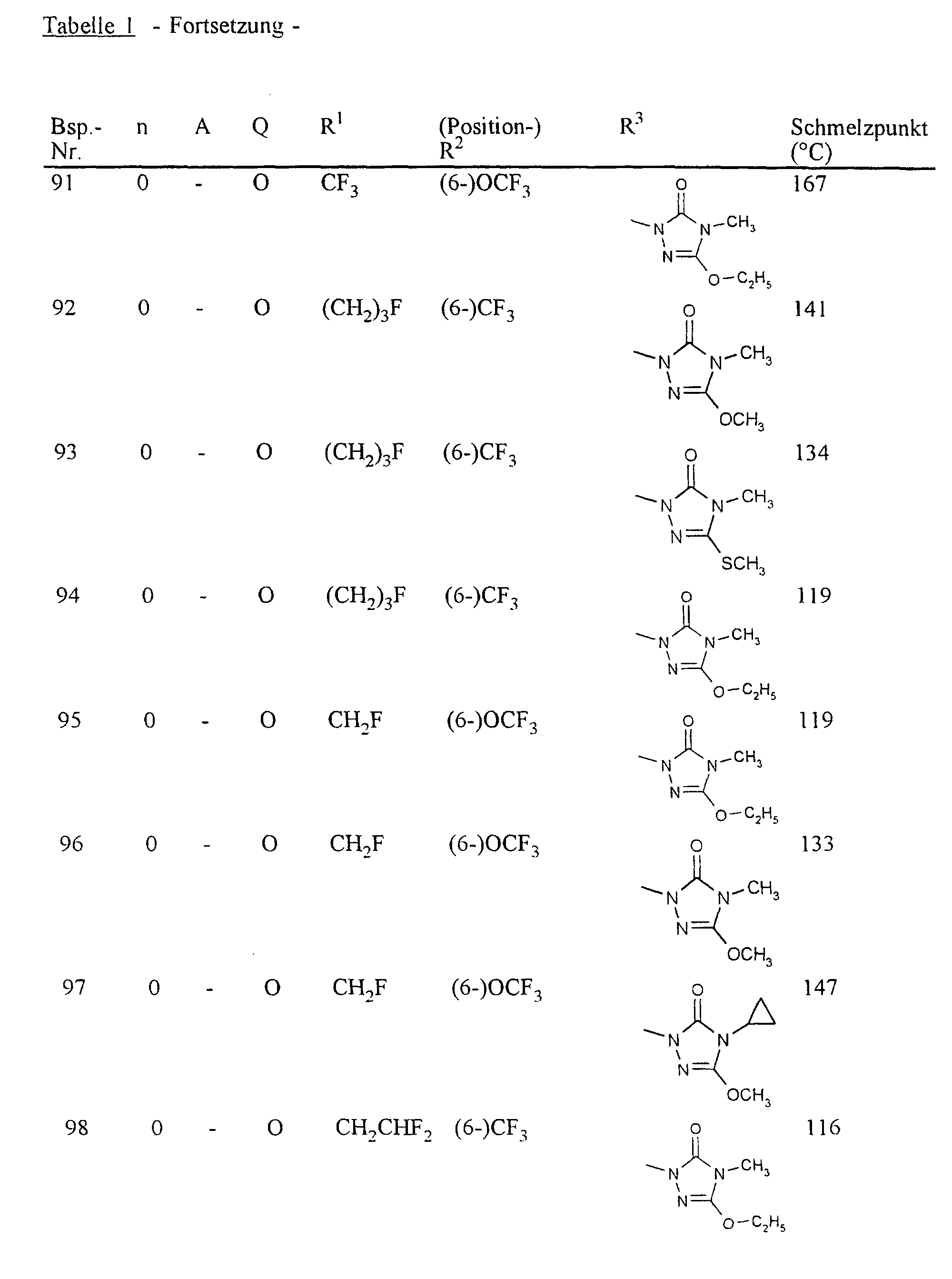

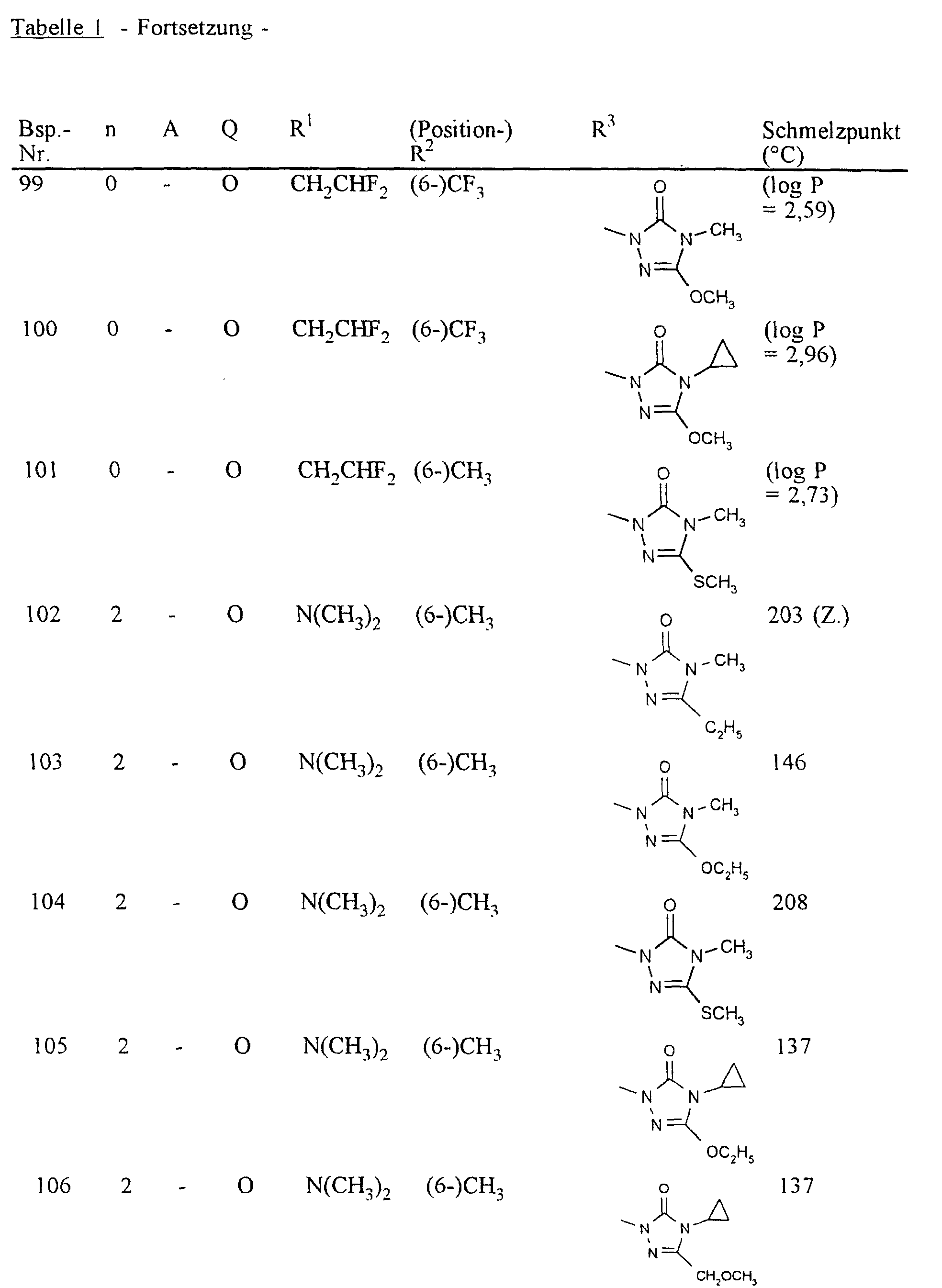

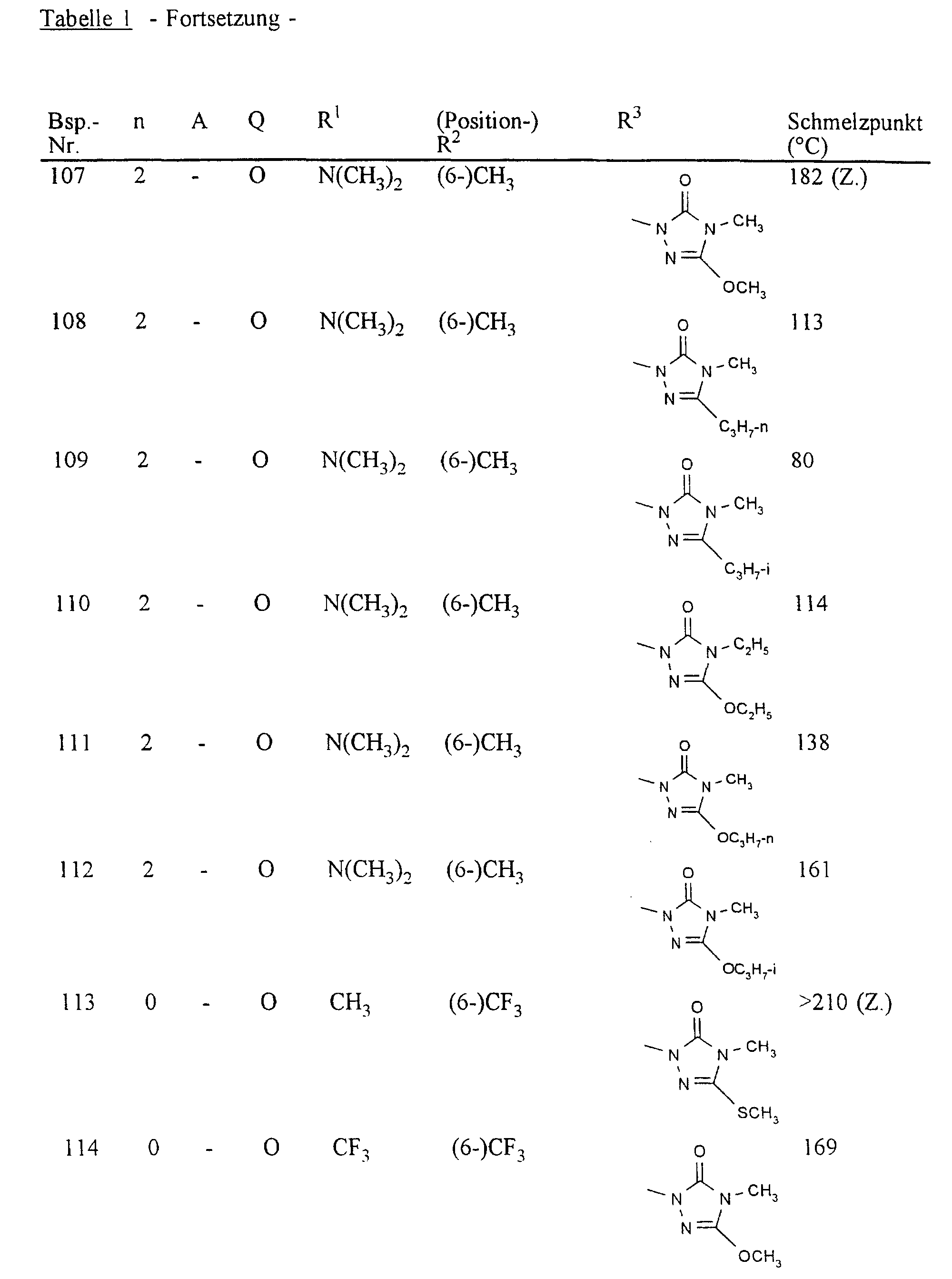

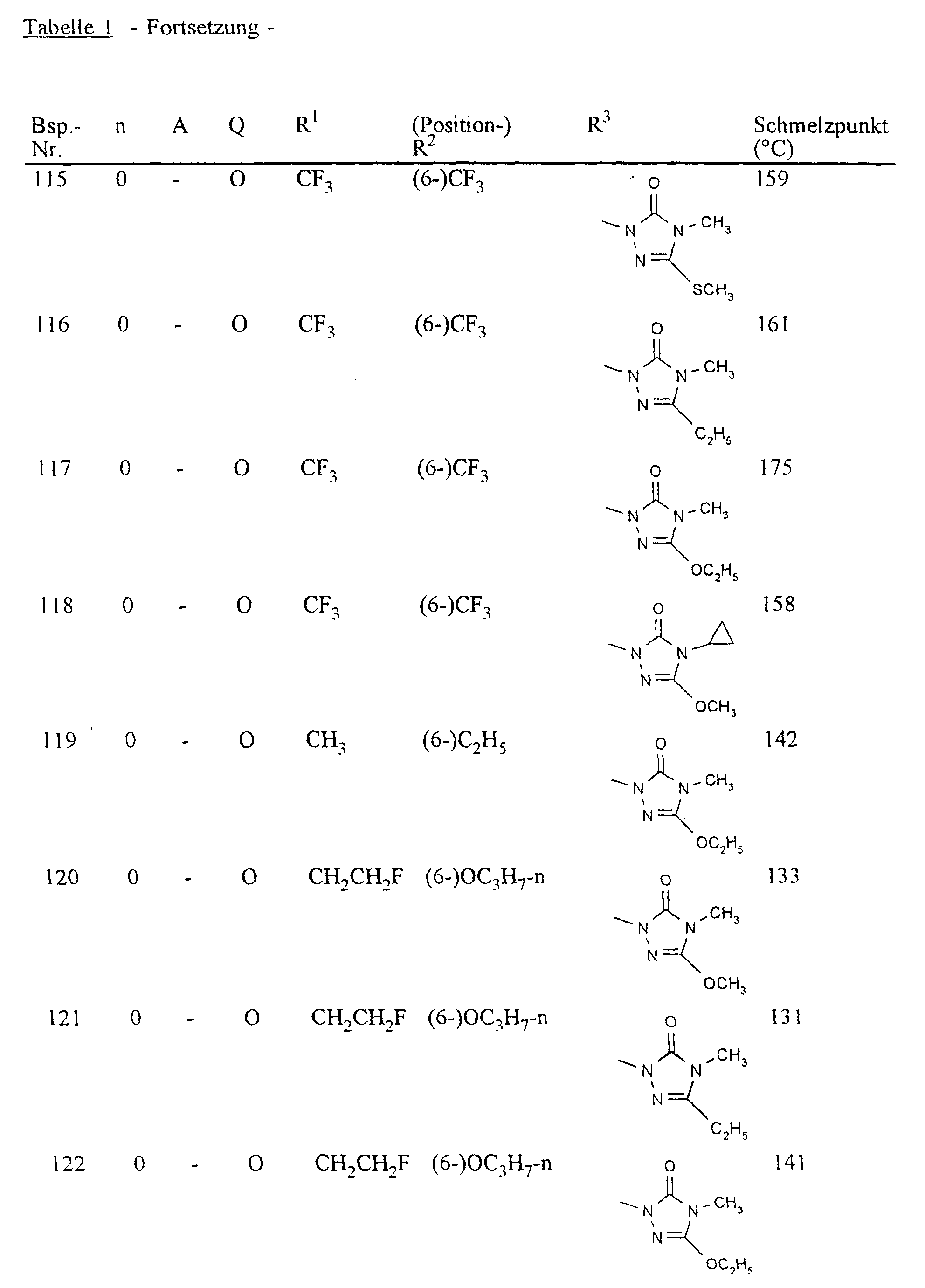

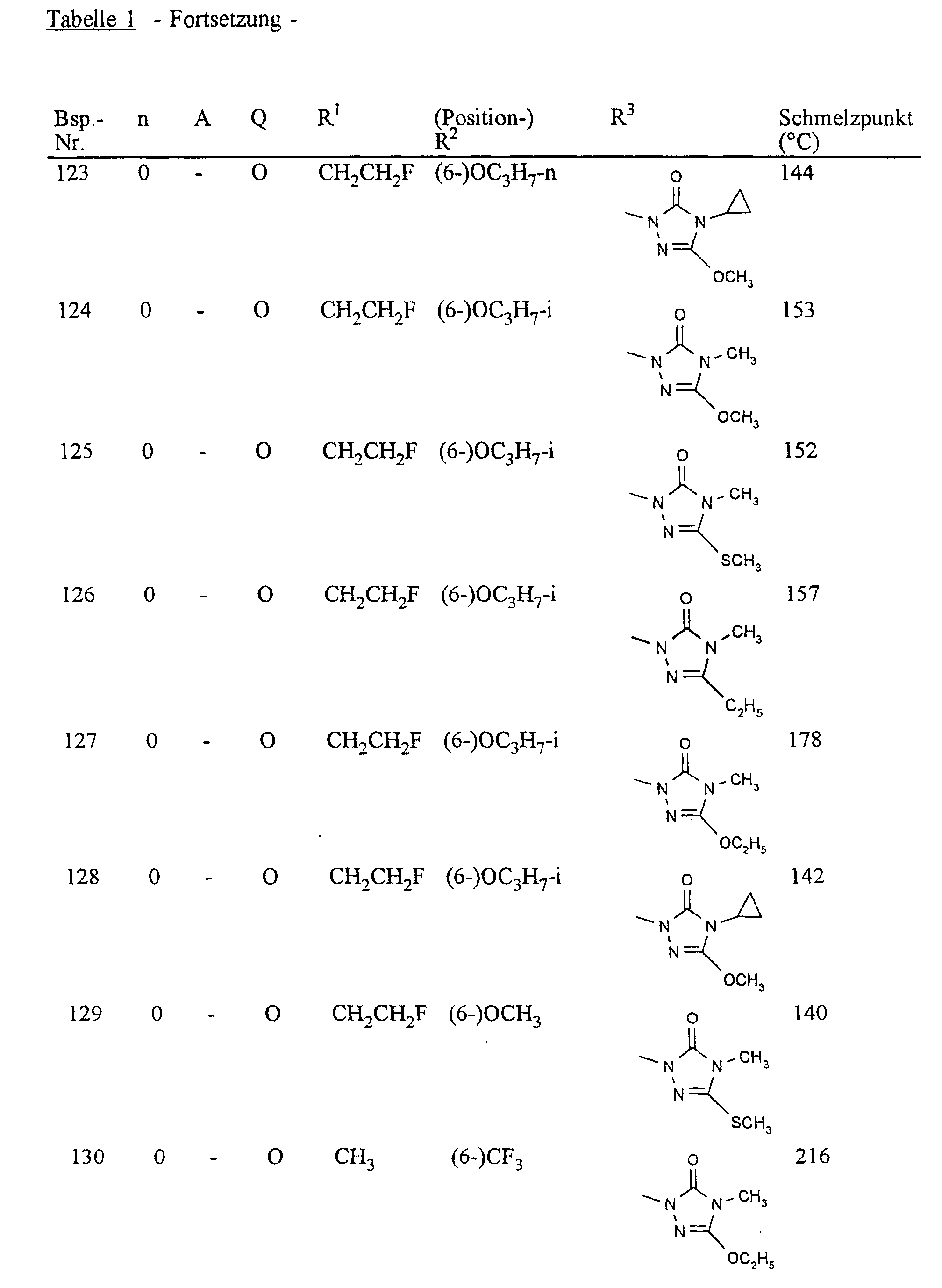

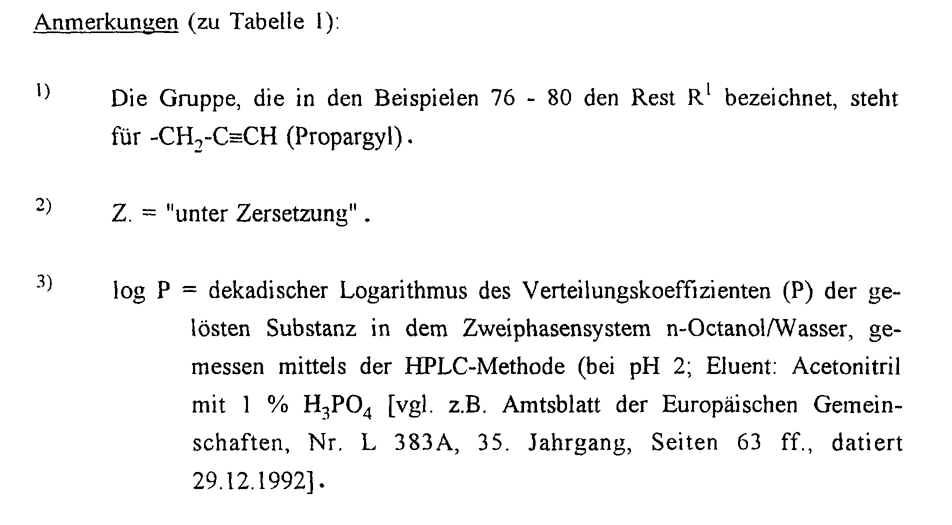

### Ausgangsstoffe der Formel (II):

### Beispiel (II-1)

### Stufe 1

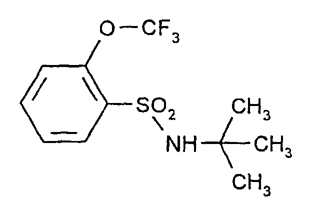

Zu einer Lösung von 99,0 g (0,384 Mol) 2-Trifluormethoxy-benzolsulfonsäurechlorid in 400 ml Acetonitril tropft man nacheinander bei 5°C 38,9 g (0,384 Mol) Triethylamin und 28,0 g (0,384 Mol) tert.-Butylamin. Die Reaktionsmischung wird 16 Stunden bei Raumtemperatur (ca. 20°C) gerührt und danach im Wasserstrahlvakuum eingeengt. Der ölige Rückstand wird in Dichlormethan gelöst, die Lösung mit 2N-Salzsäure gewaschen, über Magnesiumsulfat getrocknet und filtriert. Vom Filtrat wird das Lösungsmittel im Wasserstrahlvakuum sorgfältig abdestilliert.

Man erhält 107,6 g (95,3 % der Theorie) 2-Trifluormethoxy-benzolsulfonsäuretert.-butylamid als kristallinen Rückstand vom Schmelzpunkt 137°C.

### Stufe 2

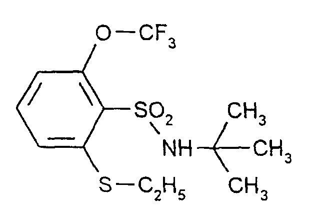

30.8 g (0,104 Mol) 2-Trifluormethoxy-benzolsulfonsäure-tert.-butylamid werden unter Argon in 280 ml (wasserfreiem) Tetrahydrofuran gelöst, auf -5°C abgekühlt und mit 156 ml (0,26 Mol) 15%iger n-Butyllithium-Lösung in Hexan versetzt. Nach dreistündigem Rühren bei 0°C bis -5°C werden 3,64 g (0,114Mol) Schwefel zugesetzt, und es wird weitere drei Stunden bei Raumtemperatur (ca. 20°C) gerührt. Die Reaktionsmischung wird danach mit 18,0 g (0,115 Mol) Iodethan versetzt, 16 Stunden bei Raumtemperatur gerührt und anschließend mit 560 ml Dichlormethan versetzt. Die Lösung wird mit 2N-Salzsäure gewaschen, über Magnesiumsulfat getrocknet und im Wasserstrahlvakuum eingeengt. Das als Rückstand erhaltene Rohprodukt wird mit Petrolether verrührt, abgesaugt und im Vakuum getrocknet.

Man erhält 31,4 g (84,6 % der Theorie) 2-Ethylthio-6-trifluormethoxy-benzolsulfonsäure-tert.-butylamid vom Schmelzpunkt 77°C.

### Stufe 3

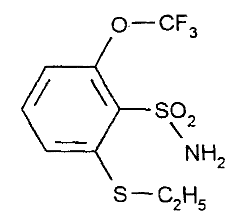

Zu einer Lösung von 30,4 g (0,085 Mol) 2-Ethylthio-6-trifluormethoxy-benzolsulfonsäure-tert-butylamid in 160 ml Dichlormethan tropft man bei Raumtemperatur (ca. 20°C) 163 ml (2,13 Mol) Trifluoressigsäure. Das Reaktionsgemisch wird ca. 24 Stunden bei Raumtemperatur gerührt, mit 300 ml Dichlormethan verdünnt, zweimal mit 300 ml Wasser gewaschen, über Magnesiumsulfat getrocknet und im Wasserstrahlvakuum eingeengt. Das als Rückstand erhaltene Rohprodukt wird mit Petrolether verrührt, abgesaugt und im Vakuum getrocknet.

Man erhält 21,7 g (84,7 % der Theorie) 2-Ethylthio-6-trifluormethoxy-benzolsulfonsäureamid vom Schmelzpunkt 146°C.

### Beispiel (II-2)

### Stufe 1

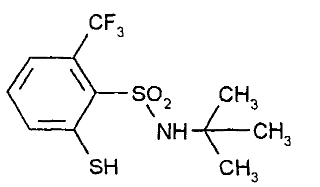

163,1 g (0,58 Mol) 2-Trifluormethyl-benzolsulfonsäure-tert.-butylamid werden unter Argon in 1 Liter (wasserfreiem) Tetrahydrofuran gelöst, auf -10°C abgekühlt und mit 884 ml (1,45 Mol) 15%iger n-Butyllithium-Lösung in Hexan versetzt. Nach dreistündigem Rühren bei 0°C bis -5°C werden 30,7 g (0,96 Mol) Schwefel zugesetzt, und es wird weitere 20 Stunden bei Raumtemperatur (ca. 20°C) gerührt. Die Reaktionsmischung wird danach unter Kühlung auf ca 20°C mit 100 ml 2N-Salzsäure, 1 Liter Wasser und 1 Liter Dichlormethan versetzt. Man stellt die wässrige Phase mit 2N-Salzsäure auf pH 1 und trennt die organische Phase ab. Sie wird mit Wasser gewaschen, über Magnesiumsulfat getrocknet und im Wasserstrahlvakuum eingeengt. Das als Rückstand erhaltene Rohprodukt wird mit Petrolether verrührt, abgesaugt und im Vakuum getrocknet.

Man erhält 160,4 g (84,6 % der Theorie) 2-Mercapto-6-trifluormethyl-benzolsulfonsäure-tert.-butylamid vom Schmelzpunkt 139°C.

### Stufe 2

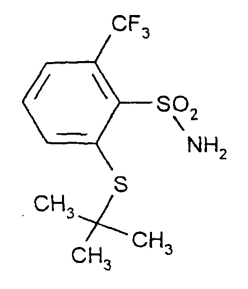

Zu einer Lösung von 17,0 g (0,054 Mol) 2-Mercapto-6-trifluormethyl-benzolsulfonsäure-tert-butylamid in 100 ml Dichlormethan tropft man bei Raumtemperatur (ca. 20°C) 104 ml (1,36 Mol) Trifluoressigsäure. Das Reaktionsgemisch wird ca. 24 Stunden bei Raumtemperatur gerührt, mit 300 ml Dichlormethan verdünnt, zweimal mit 200 ml Wasser gewaschen, über Magnesiumsulfat getrocknet und im Wasserstrahlvakuum eingeengt. Das als Rückstand erhaltene Rohprodukt wird mit Petrolether verrührt, abgesaugt und im Vakuum getrocknet.

Man erhält 13,8 g (81,2 % der Theorie) 2-tert.-Butylthio-6-trifluormethyl-benzolsulfonsäureamid vom Schmelzpunkt 91°C.

### Stufe 3

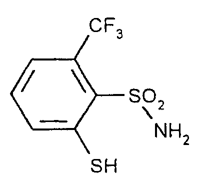

Zu einer Lösung von 7,3 g (0,023 Mol) 2-tert.-Butylthio-6-trifluormethyl-benzolsulfonsäure-tert-butylamid in 80 ml Dichlormethan tropft man bei Raumtemperatur (ca. 20°C) 23.3 ml (0,023 Mol) 1M-Bortribromid-Lösung in Dichlormethan. Das Reaktionsgemisch wird 4 Stunden bei Raumtemperatur gerührt, mit 100 ml Dichlormethan verdünnt, zweimal mit 100 ml Wasser gewaschen, über Magnesiumsulfat getrocknet und filtriert. Vom Filtrat wird das Lösungsmittel im Wasserstrahlvakuum sorgfältig abdestilliert.

Man erhält 5,3 g (88,7 % der Theorie) 2-Mercapto-6-trifluormethyl-benzolsulfonsäureamid vom Schmelzpunkt 155°C.

### Beispiel (II-3)

### Stufe 1

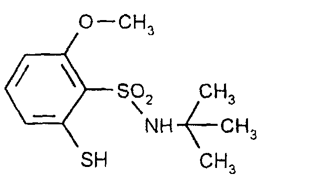

108 g (0,444 Mol) 2-Methoxy-benzolsulfonsäure-tert.-butylamid werden unter Argon in 759 ml (wasserfreiem) Tetrahydrofuran gelöst, auf -10°C abgekühlt und mit 678 ml (1,11 Mol) 15%iger n-Butyllithium-Lösung in Hexan versetzt. Nach dreistündigem Rühren bei 0°C bis -5°C werden 23,4 g (0,73 Mol) Schwefel zugesetzt, und die Mischung wird weitere 20 Stunden bei Raumtemperatur (ca. 20°C) gerührt. Die Reaktionsmischung wird danach unter Kühlung auf ca 20°C mit 2N-Salzsäure auf pH 1 gestellt. Der ausgefallene Feststoff wird durch Absaugen isoliert, mit Wasser gewaschen und im Wasserstrahlvakuum bei 50°C getrocknet.

Man erhält 72 g (59 % der Theorie) 2-Methoxy-6-mercapto-benzolsulfonsäuretert.-butylamid vom Schmelzpunkt 210°C.

Das Filtrat wird mit 1 Liter Wasser und 1,5 Liter Dichlormethan versetzt, die organische Phase abgetrennt, mit Wasser gewaschen, über Magnesiumsulfat getrocknet und im Wasserstrahlvakuum vom Lösungsmittel befreit. Man erhält weitere 35,5 g (29 % der Theorie) 2-Methoxy-6-mercapto-benzolsulfonsäure-tert.butylamid.

### Stufe 2

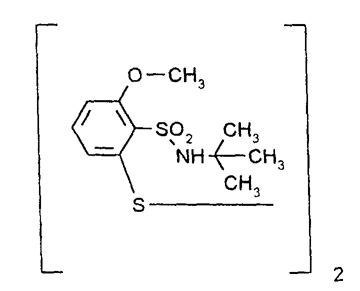

70 g (0,255 Mol) 2-Methoxy-6-mercapto-benzolsulfonsäure-tert.-butylamid werden in 180 ml Dimethylsulfoxid suspendiert und 22 Stunden auf 90°C erwärmt. Nach dem Abkühlen wird die Suspension auf ca. 1 Liter Wasser ausgetragen. Der ausgefallene Feststoff wird durch Absaugen isoliert, mit Wasser gewaschen und im Wasserstrahlvakuum bei 60°C getrocknet.

Man erhält 67,1 g (98 % der Theorie) Bis-(2-tert.-butylsulfamoyl-3-methoxyphenyl)-disulfid vom Schmelzpunkt 275°C.

### Stufe 3

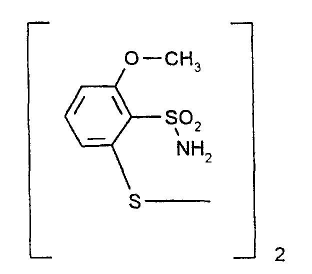

Zu einer Suspension von 20,4 g (0,037 Mol) Bis-(2-tert.-butylsulfamoyl-3-methoxy-phenyl)-disulfid in 70 ml Dichlormethan tropft man bei Raumtemperatur (ca. 20°C) 71 ml (0,93 Mol) Trifluoressigsäure. Das Reaktionsgemisch wird ca. 23 Stunden bei Raumtemperatur gerührt, abgesaugt,mit Dichlormethan gewaschen und im Vakuum bei 60°C getrocknet.

Man erhält 16,0 g (81 % der Theorie) Bis-(3-methoxy-2-sulfamoyl-phenyl)-disulfid vom Schmelzpunkt 263°C.

### Stufe 4

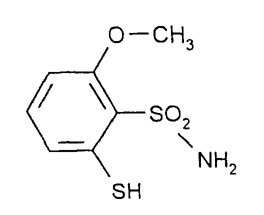

Zu einer Suspension von 19,2 g (0,044 Mol) Bis-(3-methoxy-2-sulfamoyl-phenyl)-disulfid in 180 ml Methanol gibt man portionsweise unter Stickstoff 12,7 g (0,334 Mol) festes Natriumborhydrid. Nach beendeter Zugabe rührt man 24 Stunden bei Raumtemperatur (ca. 20°C) und versetzt die Reaktionsmischung tropfenweise mit ca 100 ml 1N-Salzsäure. Der größte Teil des Methanols wird im Wasserstrahlvakuum abgezogen, der feste Rückstand mit 0,5N-Salzsäure verrührt, abgesaugt und im Vakuum bei 60°C getrocknet.
Man erhält 13,8 g (72 % der Theorie) 2-Methoxy-6-mercapto-benzolsulfonamid vom Schmelzpunkt 166°C.

### Stufe 5

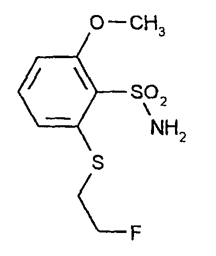

Eine Lösung von 7,5 g (34 mMol) 2-Methoxy-6-mercapto-benzolsulfonamid in 70 ml (wasserfreiem) Acetonitril wird mit 9,45 g (68,5 mMol) (wasserfreiem) Kaliumcarbonat versetzt und die Mischung wird 2 Stunden bei Raumtemperatur (ca. 20°C) gerührt. Danach gibt man tropfenweise 4,93 g (37,7 mMol) 1-Brom-2-fluor-ethan dazu und rührt weitere 24 Stunden bei Raumtemperatur. Das Reaktionsgemisch wird mit 150 ml Dichlormethan verdünnt, mit 1N-Salzsäure gewaschen, über Magnesiumsulfat getrocknet und filtriert. Vom Filtrat wird das Lösungsmittel im Wasserstrahlvakuum sorgfältig abdestilliert.

Man erhält 8,6 g (95 % der Theorie) 2-(2-Fluor-ethylthio)-6-methoxy-benzolsulfonamid vom Schmelzpunkt 127°C.

### Beispiel (II-4)

### Stufe 1

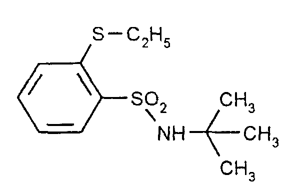

30 g (0,14 Mol) Benzolsulfonsäure-N-tert.-butylamid werden unter Argon in 400 ml (wasserfreiem) Tetrahydrofuran gelöst, auf -5°C abgekühlt und mit 210 ml (0,35 Mol) 15%iger n-Butyllithium-Lösung in Hexan versetzt. Nach dreistündigem Rühren bei 0°C bis -5°C werden 4,9 g (0,153Mol) Schwefel zugesetzt, und die Mischung wird weitere drei Stunden bei Raumtemperatur (ca. 20°C) gerührt. Die Reaktionsmischung wird danach mit 24,2 g (0,155 Mol) Iodethan versetzt, 24 Stunden bei Raumtemperatur gerührt und anschließend mit 800 ml Dichlormethan versetzt. Die Lösung wird mit 1N-Salzsäure gewaschen, über Magnesiumsulfat getrocknet und im Wasserstrahlvakuum eingeengt. Das als Rückstand erhaltene Rohprodukt wird mit Petrolether verrührt, abgesaugt und im Vakuum bei 40°C getrocknet.

Man erhält 34,1 g (89 % der Theorie) 2-Ethylthio-benzolsulfonsäure-N-tert.-butylamid vom Schmelzpunkt 88°C.

### Stufe 2

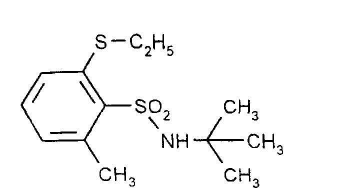

25 g (0,092 Mol) 2-Ethylthio-benzolsulfonsäure-tert.-butylamid werden unter Stickstoff in 200 ml (wasserfreiem) Tetrahydrofuran gelöst, auf -10°C abgekühlt und mit 140 ml (0,23 Mol) 15%iger n-Butyllithium-Lösung in Hexan versetzt. Nach dreistündigem Rühren bei -10°C bis -15°C wird die Reaktionsmischung mit 15,6 g (0,11 Mol) Iodmethan versetzt. Man rührt weitere zwei Stunden bei -15°C bis -20°C und läßt dann die Temperatur langsam auf Raumtemperatur (ca. 20°C) ansteigen. Nach 24 Stunden wird mit 800 ml Dichlormethan versetzt, die Lösung mit 2N-Salzsäure gewaschen, über Magnesiumsulfat getrocknet und im Wasserstrahlvakuum eingeengt.Das als Rückstand erhaltene Rohprodukt wird mit Petrolether verrührt, abgesaugt und im Vakuum bei 40°C getrocknet.

Man erhält 20,1 g (76,5 % der Theorie) 2-Ethylthio-6-methyl-benzolsulfonsäuretert.-butylamid vom Schmelzpunkt 94°C.

Analog zu den Beispielen (II-1) bis (II-4) können beispielsweise auch die in der nachstehenden Tabelle 2 aufgeführten Verbindungen der Formel (II) hergestellt werden.

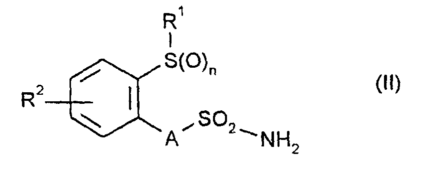

**Tabelle 2:**

| Beispiele für die Verbindungen der Formel (II) | | | | | |
|---|---|---|---|---|---|
| Bsp.-Nr. | n | A | R¹ | (Position-) R² | Schmelzpunkt (°C) |
| II-5 | 0 | - | CH₃ | (6-)C₂H₅ | |
| II-6 | 0 | - | C₂H₅ | (6-)OCH₃ | 154 |
| II-7 | 0 | - | C₂H₅ | (6-)F | 132 |
| II-8 | 0 | - | C₂H₅ | (6-)Cl | 124 |
| II-9 | 0 | - | C₂H₅ | (6-)CF₃ | 120 |
| II-10 | 1 | - | C₂H₅ | (6-)CF₃ | 112 |
| II-11 | 2 | - | C₂H₅ | (6-)CF₃ | 193 |
| II-12 | 0 | - | i-C₃H₇ | (6-)OCH₃ | 128 |
| II-13 | 0 | - | i-C₃H₇ | (6-)Cl | 83 |
| II-14 | 0 | - | CH₃ | (6-)Cl | 127 |
| II-15 | 0 | - | CH₃ | (6-)OCH₃ | 155 |
| II-16 | 0 | - | CH₃ | (6-)OCF₃ | 160 |
| II-17 | 0 | - | i-C₃H₇ | (6-)OCF₃ | 135 |
| II-18 | 0 | - | i-C₃H₇ | (6-)CH₃ | 87 |
| II-19 | 0 | - | i-C₃H₇ | (6-)C₂H₅ | 146 |
| II-20 | 0 | - | i-C₃H₇ | (6-)SCH₃ | 129 |
| II-21 | 0 | - | C₂H₅ | (6-)SC₂H₅ | 105 |
| II-22 | 0 | - | C₂H₄OCOCF₃ | (6-)OCH₃ | 135 |
| II-23 | 0 | - | CH₃ | (6-)SCH₃ | 120 |
| II-24 | 0 | - | C₂H₅ | (6-)CH₃ | 192 |
| II-25 | 0 | - | CH₃ | (6-)CH₃ | 164 |
| II-26 | 0 | - | CH₃ | (6-)CF₃ | 130 |
| II-27 | 0 | - | H | (6-)OCF₃ | 146 |
| II-28 | 0 | - | C₂H₄F | (6-)OCF₃ | |
| II-29 | 0 | - | C₂H₄F | (6-)CF₃ | 131 |
| II-30 | 0 | - | i-C₃H₇ | (6-)CF₃ | 138 |
| II-31 | 0 | - | CH₂C≡CH | (6-)OCF₃ | 105 |
| II-32 | 0 | - | CF₃ | (6-)OCF₃ | 85 |
| II-33 | 0 | - | C₂H₃F₂ | (6-)CF₃ | 110 |
| II-34 | 0 | - | C₃H₆F | (6-)CF₃ | 112 |
| II-35 | 0 | - | CH₂F | (6-)OCF₃ | 163 |
| II-36 | 0 | - | CF₃ | (6-)CF₃ | |
| II-37 | 0 | - | CF₃ | (6-)OCH₃ | |

### Anwendungsbeispiele:

### Beispiel A

### Pre-emergence-Test

| | |
|---|---|
| Lösungsmittel | 5 Gewichtsteile Aceton |
| Emulgator | 1 Gewichtsteil Alkylarylpolyglykolether |

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Samen der Testpflanzen werden in normalen Boden ausgesät. Nach ca. 24 Stunden wird der Boden mit der Wirkstoffzubereitung gespritzt, so daß die jeweils gewünschten Wirkstoffmengen pro Flächeneinheit ausgebracht werden. Die Konzentration der Spritzbrühe wird so gewählt, daß in 1 000 1 Wasser/ha die jeweils gewünschten Wirkstoffmengen ausgebracht werden.

Nach drei Wochen wird der Schädigungsgrad der Pflanzen bonitiert in % Schädigung im Vergleich zur Entwicklung der unbehandelten Kontrolle.

Es bedeuten:
- 0 % =: keine Wirkung (wie unbehandelte Kontrolle)
- 100 % =: totale Vernichtung

In diesem Test zeigen beispielsweise die Verbindungen gemäß Herstellungsbeispiel 1, 6, 7, 8, 11, 13, 14, 20, 21, 22, 24, 25, 30, 32, 33, 34, 35, 40, 41, 57, 59, 60, 61, 62 und 63 bei teilweise guter Verträglichkeit gegenüber Kulturpflanzen, wie z.B. Mais, Weizen, Baumwolle und Soja, sehr starke Wirkung gegen Unkräuter (vgl. Tabellen A-1 bis A-5).
"ai." = Wirkstoff ("active ingredient").

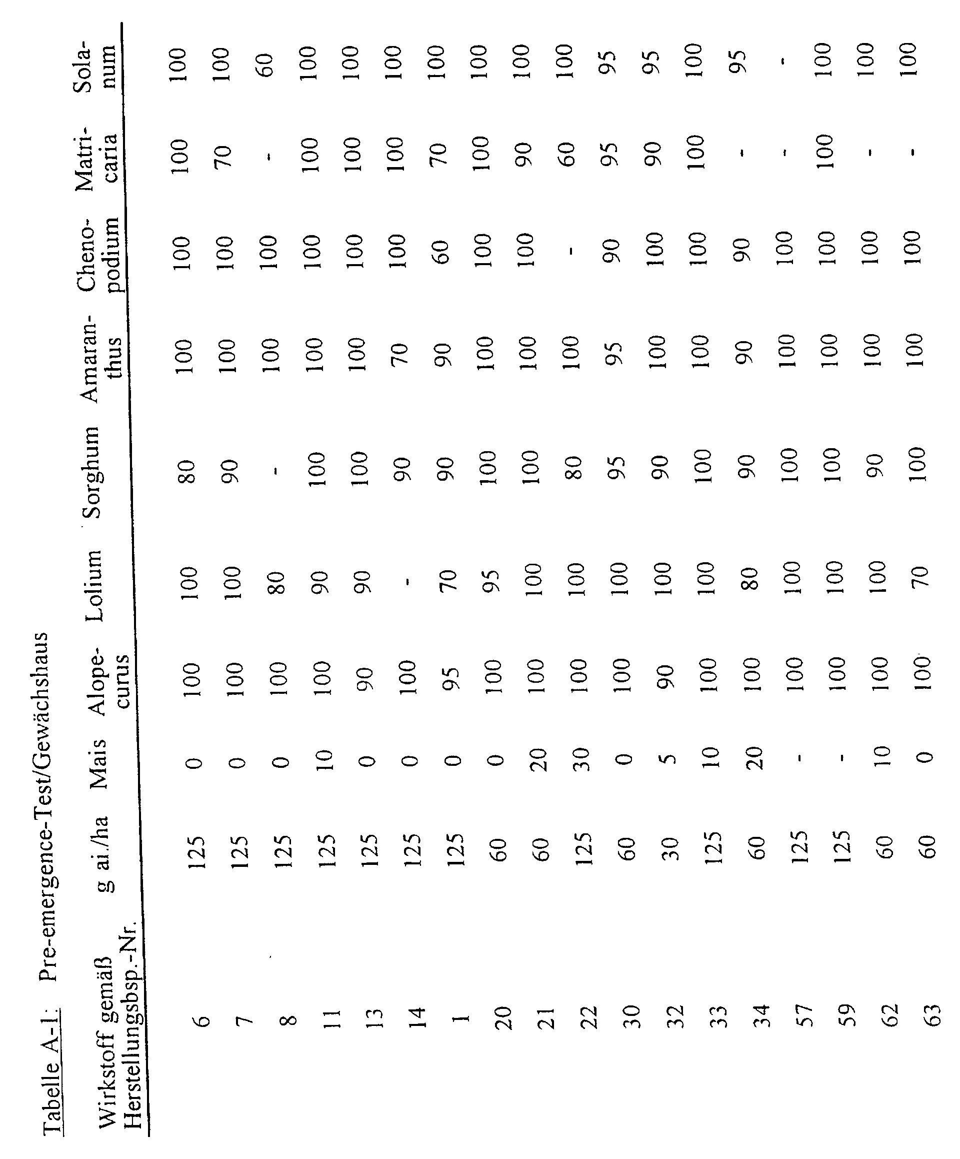

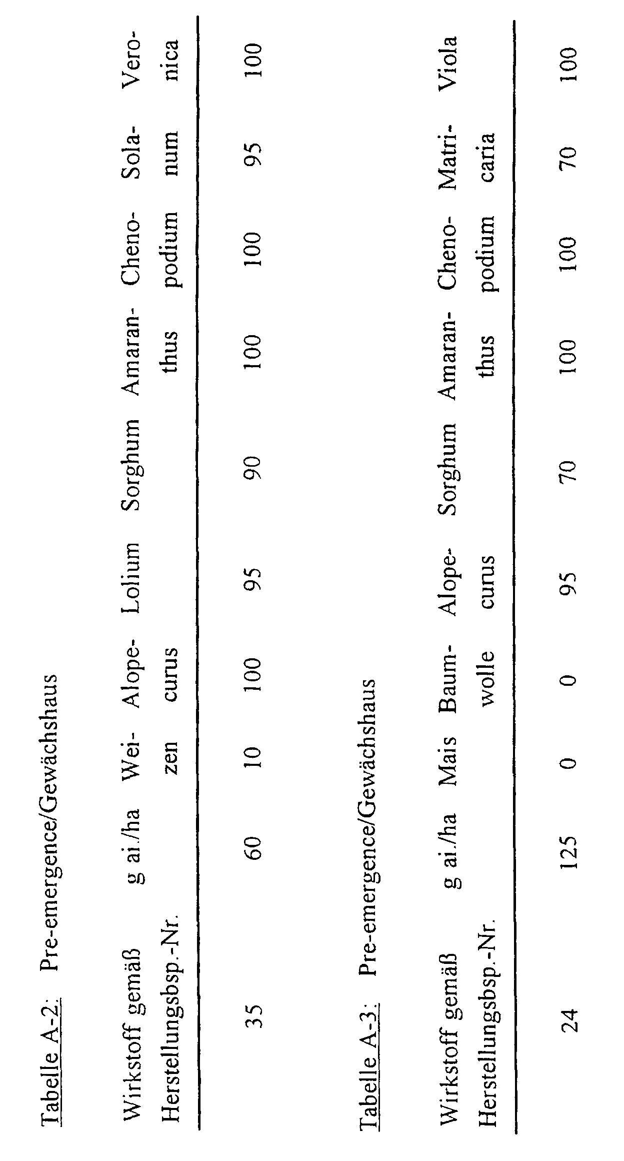

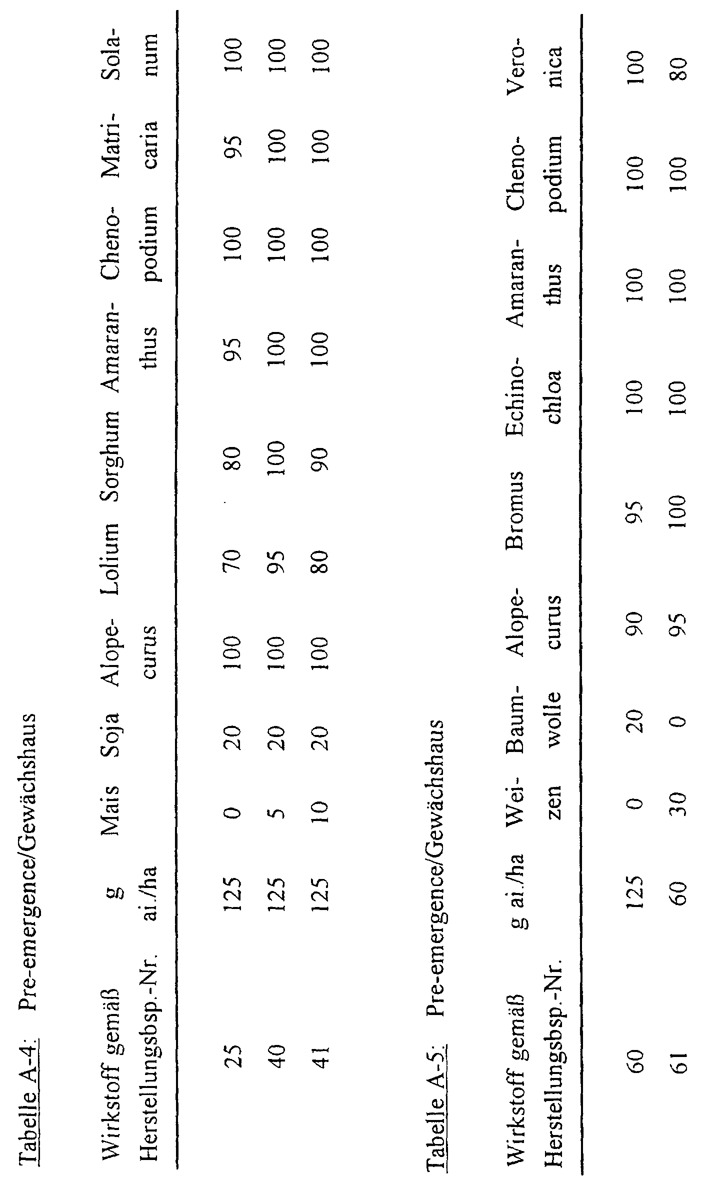

### Beispiel B

### Post-emergence-Test

| | |
|---|---|
| Lösungsmittel | 5 Gewichtsteile Aceton |
| Emulgator | 1 Gewichtsteil Alkylarylpolyglykolether |

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Mit der Wirkstoffzubereitung spritzt man Testpflanzen, welche eine Höhe von 5 - 15 cm haben so, daß die jeweils gewünschten Wirkstoffmengen pro Flächeneinheit ausgebracht werden. Die Konzentration der Spritzbrühe wird so gewählt, daß in 1000 1 Wasser/ha die jeweils gewünschten Wirkstoffmengen ausgebracht werden.

Nach drei Wochen wird der Schädigungsgrad der Pflanzen bonitiert in % Schädigung im Vergleich zur Entwicklung der unbehandelten Kontrolle.

Es bedeuten:
- 0 % =: keine Wirkung (wie unbehandelte Kontrolle)
- 100 % =: totale Vernichtung

In diesem Test zeigen beispielsweise die Verbindungen gemäß Herstellungsbeispiel 6, 7, 11, 13, 20, 21, 22, 24, 30, 34, 39, 40, 43, 44, 46, 48, 49, 50, 51, 52, 53, 55, 56 und 59 bei teilweise guter Verträglichkeit gegenüber Kulturpflanzen, wie z.B. Weizen, sehr starke Wirkung gegen Unkräuter (vgl. Tabellen B-1 bis B-4); "ai." = Wirkstoff.

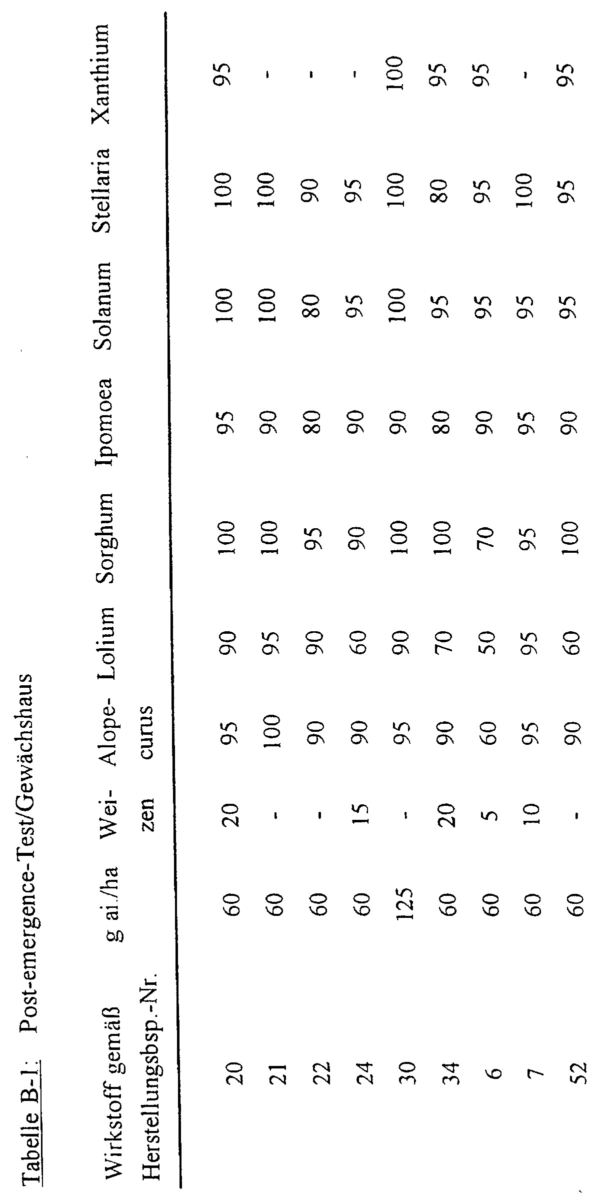

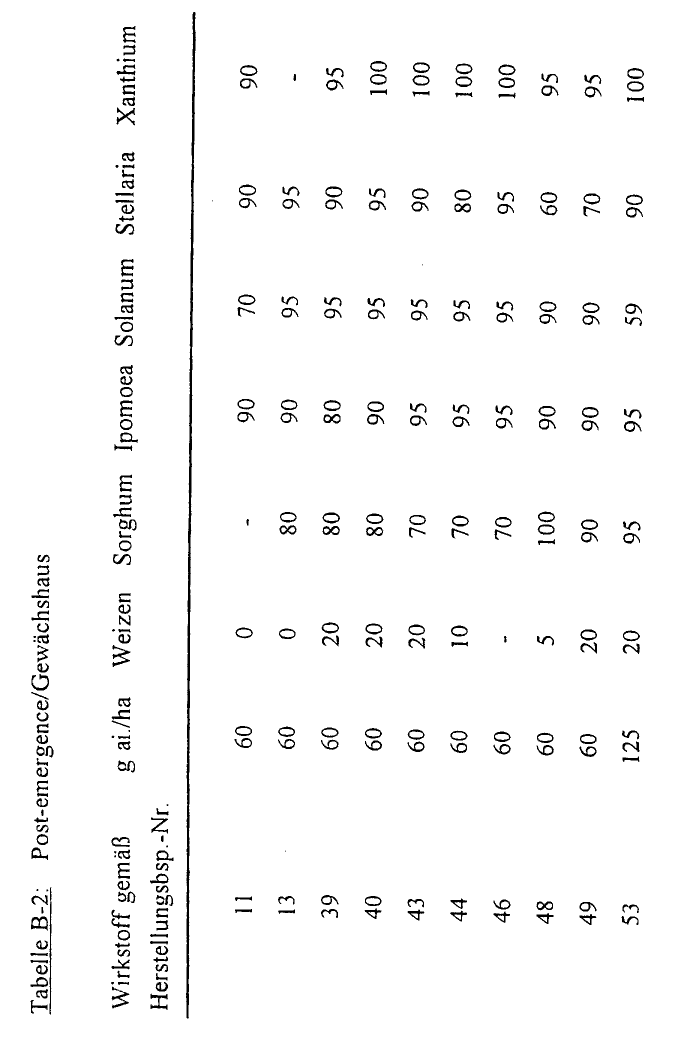

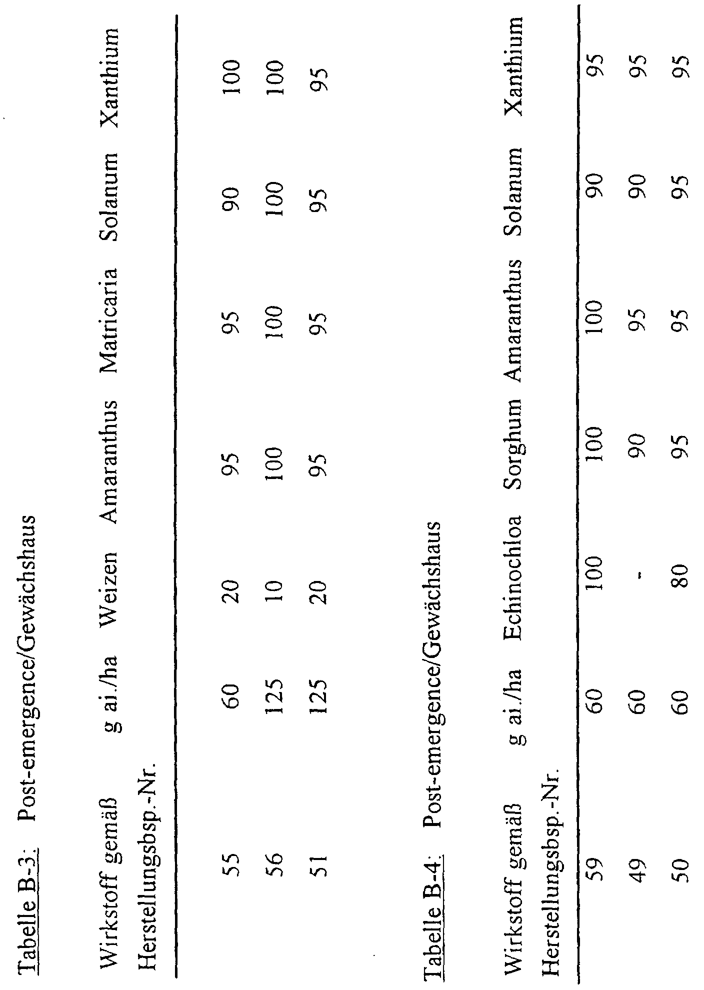

## Patentansprüche

1. Sulfonylamino(thio)carbonylverbindungen der allgemeinen Formel (I), 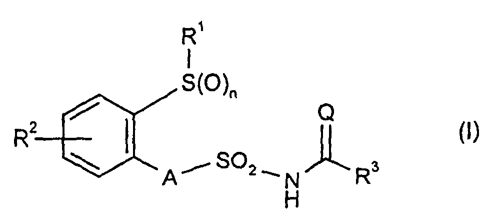 in welcher
n für die Zahlen 0, 1 oder 2 steht,
A für eine Einfachbindung steht,
Q für Sauerstoff oder Schwefel steht,
R¹ für Wasserstoff, Formyl, für jeweils gegebenenfalls durch Cyano, Fluor, Chlor, Brom, Phenyl oder C₁-C₄-Alkoxy substituiertes Alkyl, Alkoxy, Alkylamino, Alkoxyamino, Dialkylamino, N-Alkoxy-N-alkyl-amino, Alkylcarbonyl, Alkoxycarbonyl, Alkylsulfonyl, Alkenyl oder Alkinyl mit jeweils bis zu 6 Kohlenstoffatomen, oder für jeweils gegebenenfalls durch Cyano, Fluor, Chlor, Brom oder C₁-C₄-Alkyl substituiertes C₃-C₆-Cycloalkyl, C₃-C₆-Cycloalkyl-carbonyl oder C₃-C₆-Cycloalkylsulfonyl steht,
R² für Cyano, Fluor, Chlor, Brom oder für jeweils gegebenenfalls durch Cyano, Fluor, Chlor, Brom oder C₁-C₄-Alkoxy substituiertes Alkyl, Alkoxy, Alkylthio, Alkylsulfinyl, Alkylsulfonyl, Dialkylaminosulfonyl, Alkenyl, Alkinyl, Alkenyloxy oder Alkinyloxy mit jeweils bis zu 6 Kohlenstoffatomen steht und
R³ für gegebenenfalls substituiertes Heterocyclyl der nachstehenden Formel steht, 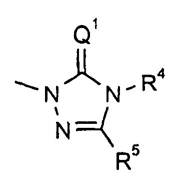 worin
Q¹ für Sauerstoff oder Schwefel steht sowie
R⁴ für Wasserstoff, Hydroxy, Amino, Cyano, für C₂-C₁₀-Alkylidenamino, für gegebenenfalls durch Fluor, Chlor, Brom, Cyano, C₁-C₄-Alkoxy, C₁-C₄-Alkyl-carbonyl oder C₁-C₄-Alkoxy-carbonyl substituiertes C₁-C₆-Alkyl, für jeweils gegebenenfalls durch Fluor, Chlor und/oder Brom substituiertes C₂-C₆-Alkenyl oder C₂-C₆-Alkinyl, für jeweils gegebenenfalls durch Fluor, Chlor, Brom, Cyano, C₁-C₄-Alkoxy oder C₁-C₄-Alkoxy-carbonyl substituiertes C₁-C₆-Alkoxy, C₁-C₆-Alkylamino oder C₁-C₆-Alkyl-carbonylamino, für C₃-C₆-Alkenyloxy, für Di-(C₁-C₄-alkyl)-amino, für jeweils gegebenenfalls durch Fluor, Chlor, Brom, Cyano und/oder C₁-C₄-Alkyl substituiertes C₃-C₆-Cycloalkyl, C₃-C₆-Cycloalkylamino oder C₃-C₆-Cycloalkyl-C₁-C₄-alkyl, oder für jeweils gegebenenfalls durch Fluor, Chlor, Brom, Cyano, Nitro, C₁-C₄-Alkyl, Trifluormethyl und/oder C₁-C₄-Alkoxy substituiertes Phenyl oder Phenyl-C₁-C₄-alkyl steht,
R⁵ für Wasserstoff, Hydroxy, Mercapto, Amino, Cyano, Fluor, Chlor, Brom, Iod, für gegebenenfalls durch Fluor, Chlor, Brom, Cyano, C₁-C₄-Alkoxy, C₁-C₄-Alkyl-carbonyl oder C₁-C₄-Alkoxy-carbonyl substituiertes C₁-C₆-Alkyl, für jeweils gegebenenfalls durch Fluor, Chlor und/oder Brom substituiertes C₂-C₆-Alkenyl oder C₂-C₆-Alkinyl, für jeweils gegebenenfalls durch Fluor, Chlor, Cyano, C₁-C₄-Alkoxy oder C₁-C₄-Alkoxy-carbonyl substituiertes C₁-C₆-Alkoxy, C₁-C₆-Alkylthio, C₁-C₆-Alkylamino oder C₁-C₆-Alkyl-carbonylamino, für C₃-C₆-Alkenyloxy, C₃-C₆-Alkinyloxy, C₃-C₆-Alkenylthio, C₃-C₆-Alkinylthio, C₃-C₆-Alkenylamino oder C₃-C₆-Alkinylamino, für Di-(C₁-C₄-alkyl)-amino, für jeweils gegebenenfalls durch Methyl und/oder Ethyl substituiertes Aziridino, Pyrrolidino, Piperidino oder Morpholino, für jeweils gegebenenfalls durch Fluor, Chlor, Brom, Cyano und/oder C₁-C₄-Alkyl substituiertes C₃-C₆-Cycloalkyl, C₅-C₆-Cycloalkenyl, C₃-C₆-Cycloalkyloxy, C₃-C₆-Cycloalkyithio, C₃-C₆-Cycloalkylamino, C₃-C₆-Cycloalkyl-C₁-C₄-alkyl, C₃-C₆-Cycloalkyl-C₁-C₄-alkoxy, C₃-C₆-Cycloalkyl-C₁-C₄-alkylthio oder C₃-C₆-Cycloalkyl-C₁-C₄-alkylamino, oder für jeweils gegebenenfalls durch Fluor, Chlor, Brom, Cyano, Nitro, C₁-C₄-Alkyl, Trifluormethyl, C₁-C₄-Alkoxy und/oder C₁-C₄-Alkoxy-carbonyl substituiertes Phenyl, Phenyl-C₁-C₄-alkyl, Phenoxy, Phenyl-C₁-C₄-alkoxy, Phenylthio, Phenyl-C₁-C₄-alkylthio, Phenylamino oder Phenyl-C₁-C₄-alkylamino steht, oder
R⁴ und R⁵ zusammen für gegebenenfalls verzweigtes Alkandiyl mit 3 bis 11 Kohlenstoffatomen stehen,
sowie die Salze von Verbindungen der Formel (I).

2. Verbindungen der Formel (I) gemäß Anspruch 1, **dadurch gekennzeichnet, dass** darin
n für die Zahlen 0, 1 oder 2 steht,
A für eine Einfachbindung steht,
Q für Sauerstoff oder Schwefel steht,
R¹ für Wasserstoff, Formyl, für jeweils gegebenenfalls durch Fluor, Chlor, Brom, Methoxy oder Ethoxy substituiertes Methyl, Ethyl, n- oder i-Propyl, n-, i- oder s-Butyl, Methoxy, Ethoxy, n- oder i-Propoxy, n-, i-, s-oder t-Butoxy, Methylamino, Ethylamino, n- oder i-Propylamino, n-, i-, s- oder t-Butylamino, Methoxyamino, Ethoxyamino, n- oder i-Propoxy-amino, n-, i-, s- oder t-Butoxyamino, Dimethylamino, Diethylamino, N-Methoxy-N-methyl-amino, Acetyl, Propionyl, Butyroyl, Methoxycarbonyl, Ethoxycarbonyl, n- oder i-Propoxycarbonyl, Methylsulfonyl, Ethylsulfonyl, n- oder i-Propylsulfonyl, n-, i-, s- oder t-Butylsulfonyl, Propenyl, Butenyl, Propinyl oder Butinyl, oder für jeweils gegebenenfalls durch Fluor, Chlor oder Methyl substituiertes Cyclopropyl, Cyclopropylcarbonyl oder Cyclopropylsulfonyl steht,
R² für Cyano, Fluor, Chlor, Brom, oder für jeweils gegebenenfalls durch Fluor, Chlor, Methoxy oder Ethoxy substituiertes Methyl, Ethyl, n- oder i-Propyl, n-, i- oder s-Butyl, Methoxy, Ethoxy, n- oder i-Propoxy, n-, i- oder s-Butoxy, Methylthio, Ethylthio, n- oder i-Propylthio, n-, i-, s- oder t-Butylthio, Methylsulfinyl, Ethylsulfinyl, Methylsulfonyl, Ethylsulfonyl, Dimethylaminosulfonyl oder Diethylaminosulfonyl, für Propenyl, Butenyl, Propinyl, Butinyl, Propenyloxy, Butenyloxy, Propinyloxy oder Butinyloxy steht und
R³ für gegebenenfalls substituiertes Heterocyclyl der nachstehenden Formel steht 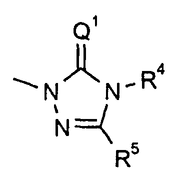 worin
Q¹ für Sauerstoff oder Schwefel steht sowie
R⁴ für Wasserstoff, Hydroxy, Amino, für C₃-C₈-Alkylidenamino, für jeweils gegebenenfalls durch Fluor, Chlor, Cyano, Methoxy oder Ethoxy substituiertes Methyl, Ethyl, n- oder i-Propyl, n-, i-, s- oder t-Butyl, für jeweils gegebenenfalls durch Fluor, Chlor oder Brom substituiertes Propenyl, Butenyl, Propinyl oder Butinyl, für jeweils gegebenenfalls durch Fluor, Chlor, Cyano, Methoxy oder Ethoxy substituiertes Methoxy, Ethoxy, n- oder i-Propoxy, n-, i-, s- oder t-Butoxy, Methylamino, Ethylamino, n- oder i-Propylamino, n-, i-, s- oder t-Butylamino, für Propenyloxy oder Butenyloxy, für Dimethylamino oder Diethylamino, für jeweils gegebenenfalls durch Fluor, Chlor, Methyl und/oder Ethyl substituiertes Cyclopropyl, Cyclobutyl, Cyclopentyl, Cyclohexyl, Cyclopropylamino, Cyclobutylamino, Cyclopentylamino, Cyclohexylamino, Cyclopropylmethyl, Cyclobutylmethyl, Cyclopentylmethyl oder Cyclohexylmethyl, oder für jeweils gegebenenfalls durch Fluor, Chlor, Methyl, Trifluormethyl und/oder Methoxy substituiertes Phenyl oder Benzyl steht,
R⁵ für Wasserstoff, Hydroxy, Mercapto, Amino, Fluor, Chlor, Brom, für jeweils gegebenenfalls durch Fluor, Chlor, Cyano, Methoxy oder Ethoxy substituiertes Methyl, Ethyl, n- oder i-Propyl, n-, i-, s- oder t-Butyl, für jeweils gegebenenfalls durch Fluor, Chlor oder Brom substituiertes Ethenyl, Propenyl, Butenyl, Propinyl oder Butinyl, für jeweils gegebenenfalls durch Fluor, Chlor, Cyano, Methoxy oder Ethoxy substituiertes Methoxy, Ethoxy, n- oder i-Propoxy, n-, i-, s- oder t-Butoxy, Methylthio, Ethylthio, n- oder i-Propylthio, n-, i-, s- oder t-Butylthio, Methylamino, Ethylamino, n- oder i-Propylamino, n-, i-, s- oder t-Butylamino, für Propenyloxy, Butenyloxy, Propinyloxy, Butinyloxy, Propenylthio, Propadienylthio Butenylthio, Propinylthio, Butinylthio, Propenylamino, Butenylamino, Propinylamino oder Butinylamino, für Dimethylamino, Diethylamino oder Dipropylamino, für jeweils gegebenenfalls durch Fluor, Chlor, Methyl und/oder Ethyl substituiertes Cyclopropyl, Cyclobutyl, Cyclopentyl, Cyclohexyl, Cyclopentenyl, Cyclohexenyl, Cyclopropyloxy, Cyclobutyloxy, Cyclopentyloxy, Cyclohexyloxy, Cyclopropylthio, Cyclobutylthio, Cyclopentylthio, Cyclohexylthio, Cyclopropylamino, Cyclobutylamino, Cyclopentylamino, Cyclohexylamino, Cyclopropylmethyl, Cyclobutylmethyl, Cyclopentylmethyl, Cyclohexylmethyl, Cyclopropylmethoxy, Cyclobutylmethoxy, Cyclopentylmethoxy, Cyclohexylmethoxy, Cyclopropylmethylthio, Cyclobutylmethylthio, Cyclopentylmethylthio, Cyclohexylmethylthio, Cyclopropylmethylamino, Cyclobutylmethylamino, Cyclopentylmethylamino oder Cyclohexylmethylamino, oder für jeweils gegebenenfalls durch Fluor, Chlor, Methyl, Trifluormethyl, Methoxy und/oder Methoxycarbonyl substituiertes Phenyl, Benzyl, Phenoxy, Benzyloxy, Phenylthio, Benzylthio, Phenylamino oder Benzylamino steht, oder
R⁴ und R⁵ zusammen für gegebenenfalls verzweigtes Alkandiyl mit 3 bis 11 Kohlenstoffatomen stehen.

3. Verbindungen der Formel (I) gemäß Anspruch 1, **dadurch gekennzeichnet, dass** darin
n für die Zahlen 0, 1 oder 2 steht,
A für eine Einfachbindung steht,
Q für Sauerstoff oder Schwefel steht,
R¹ für jeweils gegebenenfalls durch Fluor und/oder Chlor substituiertes Methyl, Ethyl, n- oder i-Propyl, n-, i-, s- oder t-Butyl steht,
R² für Fluor, Chlor, Brom, oder für jeweils gegebenenfalls durch Fluor und/oder Chlor substituiertes Methyl, Ethyl, Methoxy, Ethoxy, Methylthio oder Ethylthio - jeweils in 6-Position - steht und
R³ für gegebenenfalls substituiertes Triazolinyl der nachstehenden Formel steht 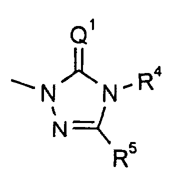 worin
Q¹ für Sauerstoff oder Schwefel steht sowie
R⁴ für jeweils gegebenenfalls durch Fluor, Chlor, Cyano, Methoxy oder Ethoxy substituiertes Methyl, Ethyl, n- oder i-Propyl, für Propenyl oder Propinyl, für Methoxy, Ethoxy, n- oder i- Propoxy, oder für Cyclopropyl steht,
R⁵ für Wasserstoff, Chlor, Brom, für jeweils gegebenenfalls durch Fluor, Chlor, Cyano, Methoxy oder Ethoxy substituiertes Methyl, Ethyl, n- oder i-Propyl, für jeweils gegebenenfalls durch Fluor und/oder Chlor substituiertes Propenyl oder Propinyl, für jeweils gegebenenfalls durch Fluor, Chlor, Cyano, Methoxy oder Ethoxy substituiertes Methoxy, Ethoxy, n- oder i-Propoxy, Methylthio, Ethylthio, n- oder i-Propylthio, für Propenyloxy oder Cyclopropyl, steht.

4. Verfahren zur Herstellung von Verbindungen der Formel (I) gemäß Anspruch und von deren Salzen, **dadurch gekennzeichnet, dass** man
(a) Aminosulfonylverbindungen der allgemeinen Formel (II) 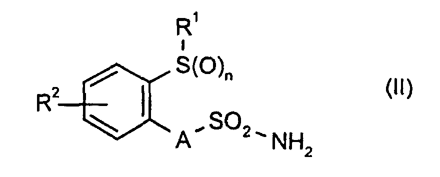 in welcher
n, A, R¹ und R² die in Anspruch 1 angegebene Bedeutung haben,
mit (Thio)Carbonsäurederivaten der allgemeinen Formel (III) 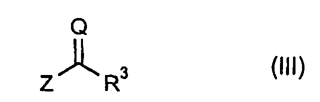 in welcher
Q und R³ die in Anspruch 1 angegebene Bedeutung haben und
Z für Halogen, Alkoxy, Aryloxy oder Arylalkoxy steht,
gegebenenfalls in Gegenwart eines Säureakzeptors und gegebenenfalls in Gegenwart eines Verdünnungsmittels umsetzt,
oder, dass man
(b) Sulfonyliso(thio)cyanate der allgemeinen Formel (IV) 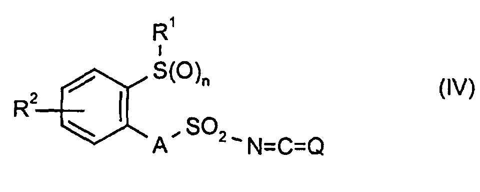 in welcher
n, A, Q, R¹ und R² die oben angegebene Bedeutung haben,
mit Heterocyclen der allgemeinen Formel (V)
H-R³ (V)
in welcher
R³ die oben angegebene Bedeutung hat,
gegebenenfalls in Gegenwart eines Reaktionshilfsmittels und gegebenenfalls in Gegenwart eines Verdünnungsmittels umsetzt,
oder, dass man
(c) Chlorsulfonylverbindungen der allgemeinen Formel (VI) 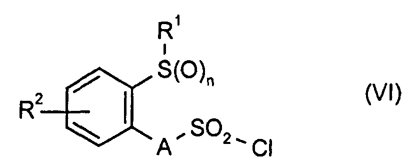 in welcher
n, A, R¹ und R² die oben angegebene Bedeutung haben,
mit Heterocyclen der allgemeinen Formel (V)
H-R³ (V)
in welcher
R³ die oben angegebene Bedeutung hat,
und Metall(thio)cyanaten der allgemeinen Formel (VII)
MQCN (VII)
in welcher
Q die oben angegebene Bedeutung hat,
gegebenenfalls in Gegenwart eines Reaktionshilfsmittels und gegebenenfalls in Gegenwart eines Verdünnungsmittels umsetzt,
oder, dass man
(d) Chlorsulfonylverbindungen der allgemeinen Formel (VI) 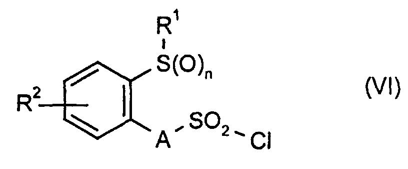 in welcher
n, A, R¹ und R² die oben angegebene Bedeutung haben,
mit (Thio)Carbonsäureamiden der allgemeinen Formel (VIII) 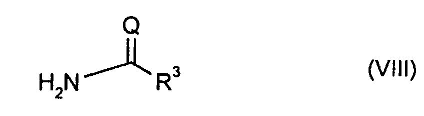 in welcher
Q und R³ die oben angegebene Bedeutung haben,
gegebenenfalls in Gegenwart eines Säureakzeptors und gegebenenfalls in Gegenwart eines Verdünnungsmittels umsetzt,
oder, dass man
(e) Sulfonylamino(thio)carbonylverbindungen der allgemeinen Formel (IX) 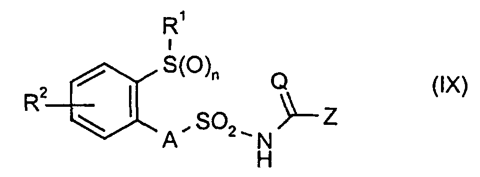 in welcher
n, A, Q, R¹ und R² die oben angegebene Bedeutung haben und
Z für Halogen, Alkoxy, Aryloxy oder Arylalkoxy steht,
mit Heterocyclen der allgemeinen Formel (V)
H-R³ (V)
in welcher
R³ die oben angegebene Bedeutung hat,
gegebenenfalls in Gegenwart eines Säureakzeptors und gegebenenfalls in Gegenwart eines Verdünnungsmittels umsetzt,
oder, dass man
(f) Heterocyclen der allgemeinen Formel (V)
H-R³ (V)
in welcher
R³ die oben angegebene Bedeutung hat,
mit Chlorsulfonyliso(thio)cyanat gegebenenfalls in Gegenwart eines Verdünnungsmittels umsetzt und die hierbei gebildeten Addukte in situ mit Benzolderivaten der allgemeinen Formel (X) 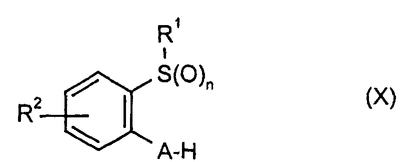 in welcher
n, A, R¹ und R² die oben angegebene Bedeutung haben,
gegebenenfalls in Gegenwart eines Säureakzeptors und gegebenenfalls in Gegenwart eines Verdünnungsmittels umsetzt,
und gegebenenfalls die nach den Verfahren (a), (b), (c), (d), (e) oder (f) erhaltenen Verbindungen der Formel (I) nach üblichen Methoden in Salze überführt.

5. Herbizide Mittel, **gekennzeichnet durch** einen Gehalt an mindestens einer Verbindung der Formel (I) oder einem ihrer Salze gemäß Anspruch 1.

6. Verwendung von Verbindungen der allgemeinen Formel (I) oder deren Salzen gemäß Anspruch 1 zur Bekämpfung von unerwünschtem Pflanzenwachstum.

7. Verfahren zur Bekämpfung von Unkräutern, **dadurch gekennzeichnet, dass** man Verbindungen der allgemeinen Formel (I) oder deren Salze gemäß Anspruch 1 auf die Unkräuter oder ihren Lebensraum einwirken läßt.

8. Verfahren zur Herstellung von herbiziden Mitteln, **dadurch gekennzeichnet, dass** man Verbindungen der allgemeinen Formel (I) oder deren Salze gemäß Anspruch 1 mit Streckmitteln und/oder oberflächenaktiven Mitteln vermischt.

9. Verbindungen der Formel (II) 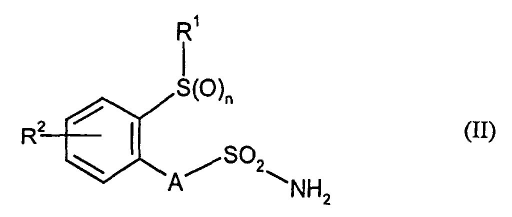 bei denen n, A, R¹ und R² für eine der in der folgenden Tabelle angegebenen Kombinationen stehen.
| n | A | R¹ | (Position-) R² |
|---|---|---|---|
| 0 | - | CH₃ | (6-)C₂H₅ |
| 0 | - | C₂H₅ | (6-)OCH₃ |
| 0 | - | C₂H₅ | (6-)F |
| 0 | - | C₂H₅ | (6-)Cl |
| 0 | - | C₂H₅ | (6-)CF₃ |
| 1 | - | C₂H₅ | (6-)CF₃ |
| 2 | - | C₂H₅ | (6-)CF₃ |
| 0 | - | i-C₃H₇ | (6-)OCH₃ |
| 0 | - | i-C₃H₇ | (6-)Cl |
| 0 | - | CH₃ | (6-)Cl |
| 0 | - | CH₃ | (6-)OCH₃ |
| 0 | - | CH₃ | (6-)OCF₃ |
| 0 | - | i-C₃H₇ | (6-)OCF₃ |
| 0 | - | i-C₃H₇ | (6-)CH₃ |
| 0 | - | i-C₃H₇ | (6-)C₂H₅ |
| 0 | - | i-C₃H₇ | (6-)SCH₃ |
| 0 | - | C₂H₅ | (6-)SC₂H₅ |
| 0 | - | C₂H₄OCOCF₃ | (6-)OCH₃ |
| 0 | - | CH₃ | (6-)SCH₃ |
| 0 | - | C₂H₅ | (6-)CH₃ |
| 0 | - | CH₃ | (6-)CH₃ |
| 0 | - | CH₃ | (6-)CF₃ |
| 0 | - | H | (6-)OCF₃ |
| 0 | - | C₂H₄F | (6-)OCF₃ |
| 0 | - | C₂H₄F | (6-)CF₃ |
| 0 | - | i-C₃H₇ | (6-)CF₃ |
| 0 | - | CH₂C≡CH | (6-)OCF₃ |
| 0 | - | CF₃ | (6-)OCF₃ |
| 0 | - | C₂H₃F₂ | (6-)CF₃ |
| 0 | - | C₃H₆F | (6-)CF₃ |
| 0 | - | CH₂F | (6-)OCF₃ |
| 0 | - | CF₃ | (6-)CF₃ |
| 0 | - | CF₃ | (6-)OCH₃ |

## Claims

1. Sulphonylamino(thio)carbonyl compounds of the general formula (I) 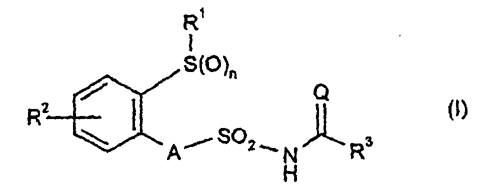 in which
n represents the numbers 0, 1 or 2,
A represents a single bond,
Q represents oxygen or sulphur,
R¹ represents hydrogen, formyl, represents in each case optionally cyano-, fluorine-, chlorine-, bromine-, phenyl- or C₁-C₄-alkoxy-substituted alkyl, alkoxy, alkylamino, alkoxyamino, dialkylamino, N-alkoxy-N-alkylamino, alkylcarbonyl, alkoxycarbonyl, alkylsulphonyl, alkenyl or alkynyl having in each case up to 6 carbon atoms, or represents in each case optionally cyano-, fluorine-, chlorine-, bromine- or C₁-C₄-alkyl-substituted C₃-C₆-cycloalkyl, C₃-C₆-cycloalkyl-carbonyl or C₃-C₆-cycloalkylsulphonyl,
R² represents cyano, fluorine, chlorine, bromine or represents in each case optionally cyano-, fluorine-, chlorine-, bromine- or C₁-C₄-alkoxy-substituted alkyl, alkoxy, alkylthio, alkylsulphinyl, alkylsulphonyl, dialkylaminosulphonyl, alkenyl, alkynyl, alkenyloxy or alkynyloxy having in each case up to 6 carbon atoms and
R³ represents optionally substituted heterocyclyl of the formula below 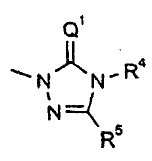 in which
Q¹ represents oxygen or sulphur and
R⁴ represents hydrogen, hydroxyl, amino, cyano, represents C₂-C₁₀-alkylideneamino, represents optionally fluorine-, chlorine-, bromine-, cyano-, C₁-C₄-alkoxy-, C₁-C₄-alkyl-carbonyl- or C₁-C₄-alkoxy-carbonyl-substituted C₁-C₆-alkyl, represents in each case optionally fluorine-, chlorine- and/or bromine-substituted C₂-C₆-alkenyl or C₂-C₆-alkynyl, represents in each case optionally fluorine-, chlorine-, bromine-, cyano-, C₁-C₄-alkoxy- or C₁-C₄-alkoxy-carbonyl-substituted C₁-C₆-alkoxy, C₁-C₆-alkylamino or C₁-C₆-alkyl-carbonylamino, represents C₃-C₆-alkenyloxy, represents di-(C₁-C₄-alkyl)-amino, represents in each case optionally fluorine-, chlorine-, bromine-, cyano- and/or C₁-C₄-alkyl-substituted C₃-C₆-cycloalkyl, C₃-C₆-cycloalkylamino or C₃-C₆-cycloalkyl-C₁-C₄-alkyl, or represents in each case optionally fluorine-, chlorine-, bromine-, cyano-, nitro-, C₁-C₄-alkyl-, trifluoromethyl- and/or C₁-C₄-alkoxy-substituted phenyl or phenyl-C₁-C₄-alkyl,
R⁵ represents hydrogen, hydroxyl, mercapto, amino, cyano, fluorine, chlorine, bromine, iodine, represents optionally fluorine-, chlorine-, bromine-, cyano-, C₁-C₄-alkoxy-, C₁-C₄-alkyl-carbonyl- or C₁-C₄-alkoxy-carbonyl-substituted C₁-C₆-alkyl, represents in each case optionally fluorine-, chlorine- and/or bromine-substituted C₂-C₆-alkenyl or C₂-C₆-alkynyl, represents in each case optionally fluorine-, chlorine-, cyano-, C₁-C₄-alkoxy- or C₁-C₄-alkoxy-carbonyl-substituted C₁-C₆-alkoxy, C₁-C₆-alkylthio, C₁-C₆-alkylamino or C₁-C₆-alkyl-carbonylamino, represents C₃-C₆-alkenyloxy, C₃-C₆-alkynyloxy, C₃-C₆-alkenylthio, C₃-C₆-alkynylthio, C₃-C₆-alkenylamino or C₃-C₆-alkynylamino, represents di-(C₁-C₄-alkyl)-amino, represents in each case optionally methyl- and/or ethyl-substituted aziridino, pyrrolidino, piperidino or morpholino, represents in each case optionally fluorine-, chlorine-, bromine-, cyano- and/or C₁-C₄-alkyl-substituted C₃-C₆-cycloalkyl, C₅-C₆-cycloalkenyl, C₃-C₆-cycloalkyloxy, C₃-C₆-cycloalkylthio, C₃-C₆-cycloalkylamino, C₃-C₆-cycloalkyl-C₁-C₄-alkyl, C₃-C₆-cycloalkyl-C₁-C₄-alkoxy, C₃-C₆-cycloalkyl-C₁-C₄-alkylthio or C₃-C₆-cycloalkyl-C₁-C₄-alkylamino, or represents in each case optionally fluorine-, chlorine-, bromine-, cyano-, nitro-, C₁-C₄-alkyl-, trifluoromethyl-, C₁-C₄-alkoxy- and/or C₁-C₄-alkoxy-carbonyl-substituted phenyl, phenyl-C₁-C₄-alkyl, phenoxy, phenyl-C₁-C₄-alkoxy, phenylthio, phenyl-C₁-C₄-alkylthio, phenylamino or phenyl-C₁-C₄-alkylamino, or
R⁴ and R⁵ together represent optionally branched alkanediyl having 3 to 11 carbon atoms,
and the salts of compounds of the formula (I).

2. Compounds of the formula (I) according to Claim 1, **characterized in that**
n represents the numbers 0, 1 or 2,
A represents a single bond,
Q represents oxygen or sulphur,
R¹ represents hydrogen, formyl, represents in each case optionally fluorine-, chlorine-, bromine-, methoxy- or ethoxy-substituted methyl, ethyl, n- or i- propyl, n-, i- or s-butyl, methoxy, ethoxy, n- or i-propoxy, n-, i-, s- or t-butoxy, methylamino, ethylamino, n- or i-propylamino, n-, i-, s- or t-butylamino, methoxyamino, ethoxyamino, n- or i-propoxyamino, n-, i-, s- or t-butoxyamino, dimethylamino, diethylamino, N-methoxy-N-methylamino, acetyl, propionyl, butyroyl, methoxycarbonyl, ethoxycarbonyl, n- or i-propoxycarbonyl, methylsulphonyl, ethylsulphonyl, n- or i-propylsulphonyl, n-, i-, s- or t-butylsulphonyl, propenyl, butenyl, propynyl or butynyl, or represents in each case optionally fluorine-, chlorine- or methyl-substituted cyclopropyl, cyclopropylcarbonyl or cyclopropylsulphonyl,
R² represents cyano, fluorine, chlorine, bromine, or represents in each case optionally fluorine-, chlorine-, methoxy- or ethoxy-substituted methyl, ethyl, n- or i-propyl, n-, i- or s-butyl, methoxy, ethoxy, n- or i-propoxy, n-, i- or s-butoxy, methylthio, ethylthio, n- or i-propylthio, n-, i-, s- or t-butylthio, methylsulphinyl, ethylsulphinyl, methylsulphonyl, ethylsulphonyl, dimethylaminosulphonyl or diethylaminosulphonyl, represents propenyl, butenyl, propynyl, butynyl, propenyloxy, butenyloxy, propynyloxy or butynyloxy and
R³ represents optionally substituted heterocyclyl of the formula below 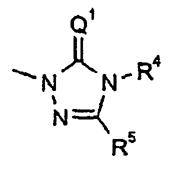 in which
Q¹ represents oxygen or sulphur and
R⁴ represents hydrogen, hydroxyl, amino, represents C₃-C₈-alkylideneamino, represents in each case optionally fluorine-, chlorine-, cyano-, methoxy- or ethoxy-substituted methyl, ethyl, n- or i-propyl, n-, i-, s- or t-butyl, represents in each case optionally fluorine-, chlorine- or bromine-substituted propenyl, butenyl, propynyl or butynyl, represents in each case optionally fluorine-, chlorine-, cyano-, methoxy- or ethoxy-substituted methoxy, ethoxy, n- or i-propoxy, n-, i-, s- or t-butoxy, methylamino, ethylamino, n- or i-propylamino, n-, i-, s- or t-butylamino, represents propenyloxy or butenyloxy, represents dimethylamino or diethylamino, or represents in each case optionally fluorine-, chlorine-, methyl- and/or ethyl-substituted cyclopropyl, cyclobutyl, cyclopentyl, cyclohexyl, cyclopropylamino, cyclobutylamino, cyclopentylamino, cyclohexylamino, cyclopropylmethyl, cyclobutylmethyl, cyclopentylmethyl or cyclohexylmethyl, or represents in each case optionally fluorine-, chlorine-, methyl-, trifluoromethyl- and/or methoxy-substituted phenyl or benzyl,
R⁵ represents hydrogen, hydroxyl, mercapto, amino, fluorine, chlorine, bromine, represents in each case optionally fluorine-, chlorine-, cyano-, methoxy- or ethoxy-substituted methyl, ethyl, n- or i-propyl, n-, i-, s- or t-butyl, represents in each case optionally fluorine-, chlorine- or bromine-substituted ethenyl, propenyl, butenyl, propynyl or butynyl, represents in each case optionally fluorine-, chlorine-, cyano-, methoxy- or ethoxy-substituted methoxy, ethoxy, n- or i-propoxy, n-, i-, s- or t-butoxy, methylthio, ethylthio, n- or i-propylthio, n-, i-, s- or t-butylthio, methylamino, ethylamino, n- or i-propylamino, n-, i-, s- or t-butylamino, represents propenyloxy, butenyloxy, propynyloxy, butynyloxy, propenylthio, propadienylthio, butenylthio, propynylthio, butynylthio, propenylamino, butenylamino, propynylamino or butylamino, represents dimethylamino, diethylamino or dipropylamino, represents in each case optionally fluorine-, chlorine-, methyl- and/or ethyl-substituted cyclopropyl, cyclobutyl, cyclopentyl, cyclohexyl, cyclopentenyl, cyclohexenyl, cyclopropyloxy, cyclobutyloxy, cyclopentyloxy, cyclohexyloxy, cyclopropylthio, cyclobutylthio, cyclopentylthio, cyclohexylthio, cyclopropylamino, cyclobutylamino, cyclopentylamino, cyclohexylamino, cyclopropylmethyl, cyclobutylmethyl, cyclopentylmethyl, cyclohexylmethyl, cyclopropylmethoxy, cyclobutylmethoxy, cyclopentylmethoxy, cyclohexylmethoxy, cyclopropylmethylthio, cyclobutylmethylthio, cyclopentylmethylthio, cyclohexylmethylthio, cyclopropylmethylamino, cyclobutylmethylamino, cyclopentylmethylamino or cyclohexylmethylamino, or represents in each case optionally fluorine-, chlorine-, methyl-, trifluoromethyl-, methoxy- and/or methoxycarbonyl-substituted phenyl, benzyl, phenoxy, benzyloxy, phenylthio, benzylthio, phenylamino or benzylamino, or
R⁴ and R⁵ together represent optionally branched alkanediyl having 3 to 11 carbon atoms.

3. Compounds of the formula (I) according to claim I, **characterized in that**
n represents the numbers 0, 1 or 2,
A represents a single bond,
Q represents oxygen or sulphur,
R¹ represents in each case optionally fluorine- and/or chlorine-substituted methyl, ethyl, n- or i-propyl, n-, i-, s- or t-butyl,
R² represents fluorine, chlorine, bromine, or represents in each case optionally fluorine- and/or chlorine-substituted methyl, ethyl, methoxy, ethoxy, methylthio or ethylthio - in each case in the 6-position - and
R³ represents optionally substituted triazolinyl of the formula below 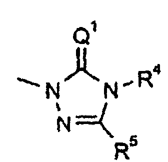
in which
Q¹ represents oxygen or sulphur and
R⁴ represents in each case optionally fluorine-, chlorine-, cyano-, methoxy- or ethoxy-substituted methyl, ethyl, n- or i-propyl, represents propenyl or propynyl, represents methoxy, ethoxy, n- or i-propoxy, or represents cyclopropyl,
R⁵ represents hydrogen, chlorine, bromine, represents in each case optionally fluorine-, chlorine-, cyano-, methoxy- or ethoxy-substituted methyl, ethyl, n- or i-propyl, represents in each case optionally fluorine- and/or chlorine-substituted propenyl or propynyl, represents in each case optionally fluorine-, chlorine-, cyano-, methoxy- or ethoxy-substituted methoxy, ethoxy, n- or i-propoxy, methylthio, ethylthio, n- or i-propylthio, represents propenyloxy or cyclopropyl.

4. Process for preparing compounds of the formula (I) according to Claim 1 and salts thereof, **characterized in that**
(a) aminosulphonyl compounds of the general formula (II) 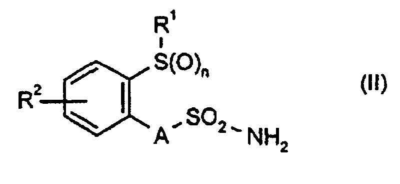 in which
n, A, R¹ and R² are as defined in Claim 1
are reacted with (thio)carboxylic acid derivatives of the general formula (III) 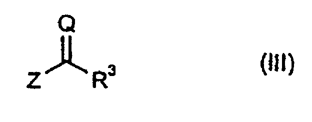 in which
Q and R³ are as defined in Claim 1 and
Z represents halogen, alkoxy, aryloxy or arylalkoxy,
if appropriate in the presence of an acid acceptor and if appropriate in the presence of a diluent,
or **in that**
(b) sulphonyl iso(thio)cyanates of the general formula (IV) 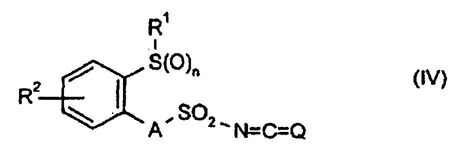 in which
n, A, Q, R¹ and R² are as defined above
are reacted with heterocycles of the general formula (V)
H-R³ (V)
in which
R³ is as defined above,
if appropriate in the presence of a reaction auxiliary and if appropriate in the presence of a diluent,
or **in that**
(c) chlorosulphonyl compounds of the general formula (VI) 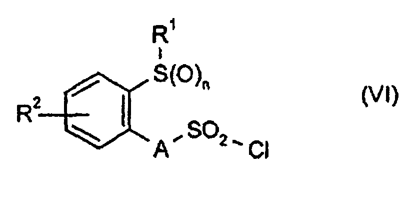 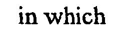
n, A, R¹ and R² are as defined above
are reacted with heterocycles of the general formula (V)
H-R³ (V)
in which
R³ is as defined above
and metal (thio)cyanates of the general formula (VII)
MQCN (VII)
in which
Q is as defined above,
if appropriate in the presence of a reaction auxiliary and if appropriate in the presence of a diluent,
or **in that**
(d) chlorosulphonyl compounds of the general formula (VI) 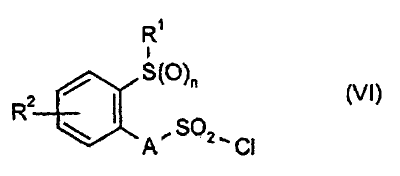 in which
n, A, R¹ and R² are as defined above
are reacted with (thio)carboxamides of the general formula (VIII) 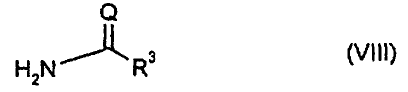 in which
Q and R³ are as defined above,
if appropriate in the presence of an acid acceptor and if appropriate in the presence of a diluent,
or **in that**
(e) sulphonylamino(thio)carbonyl compounds of the general formula (IX) 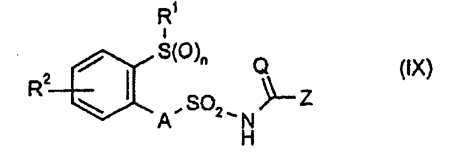 in which
n, A, Q, R¹ and R² are as defined above and
Z represents halogen, alkoxy, aryloxy or arylalkoxy
are reacted with heterocycles of the general formula (V)
H-R³ (V)
in which
R³ is as defined above,
if appropriate in the presence of an acid acceptor and if appropriate in the presence of a diluent,
or **in that**
(f) heterocycles of the general formula (V)
H-R³ (V)
in which
R³ is as defined above
are reacted with chlorosulphonyl iso(thio)cyanate, if appropriate in the presence of a diluent, and the adducts formed in this process are reacted in situ with benzene derivatives of the general formula (X) 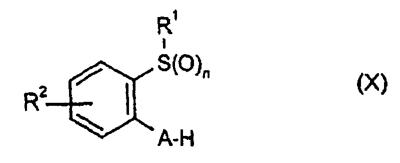 in which
n, A, R¹ and R² are as defined above,
if appropriate in the presence of an acid acceptor and if appropriate in the presence of a diluent,
and the compounds of the formula (I) obtained by process (a), (b), (c), (d), (e) or (f) are, if appropriate, converted into salts, by customary methods.

5. Herbicidal compositions, **characterized in that** they comprise at least one compound of the formula (I) or a salt thereof according to Claim 1.

6. Use of compounds of the general formula (I) or salts thereof according to Claim 1 for controlling unwanted vegetation.

7. Method for controlling weeds, **characterized in that** compounds of the general formula (I) or salts thereof according to Claim 1 are allowed to act on the weeds or their habitat.

8. Process for preparing herbicidal compositions, **characterized in that** compounds of the general formula (I) or salts thereof according to Claim 1 are mixed with extenders and/or surfactants.

9. Compounds of the formula (II) 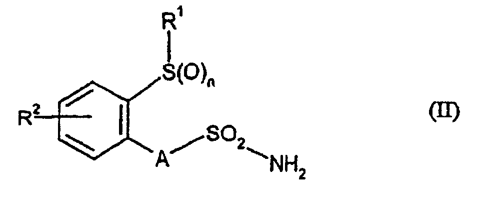 in which n, A, R¹ and R² represent one of the combinations given in the table below.
| N | A | R¹ | (position-)R² |
|---|---|---|---|
| 0 | - | CH₃ | (6-)C₂H₅ |
| 0 | - | C₂H₅ | (6-)OCH₃ |
| 0 | - | C₂H₅ | (6-)F |
| 0 | - | C₂H₅ | (6-)Cl |
| 0 | - | C₂H₅ | (6-)CF₃ |
| 1 | - | C₂H₅ | (6-)CF₃ |
| 2 | - | C₂H₅ | (6-)CF₃ |
| 0 | - | i-C₃H₇ | (6-)OCH₃ |
| 0 | - | i-C₃H₇ | (6-)Cl |
| 0 | - | CH₃ | (6-)Cl |
| 0 | - | CH₃ | (6-)OCH₃ |
| 0 | - | CH₃ | (6-)OCF₃ |
| 0 | - | i-C₃H₇ | (6-)OCF₃ |
| 0 | - | i-C₃H₇ | (6-)CH₃ |
| 0 | - | i-C₃H₇ | (6-)C₂H₅ |
| 0 | - | i-C₃H₇ | (6-)SCH₃ |
| 0 | - | C₂H₅ | (6-)SC₂H₅ |
| 0 | - | C₂H₄OCOCF₃ | (6-)OCH₃ |
| 0 | - | CH₃ | (6-)SCH₃ |
| 0 | - | C₂H₅ | (6-)CH₃ |
| 0 | - | CH₃ | (6-)CH₃ |
| 0 | - | CH₃ | (6-)CF₃ |
| 0 | - | H | (6-)OCF₃ |
| 0 | - | C₂H₄F | (6-)OCF₃ |
| 0 | - | C₂H₄F | (6-)CF₃ |
| 0 | - | i-C₃H₇ | (6-)CF₃ |
| 0 | - | CH₂C≡CH | (6-)OCF₃ |
| 0 | - | CF₃ | (6-)OCF₃ |
| 0 | - | C₂H₃F₂ | (6-)CF₃ |
| 0 | - | C₃H₆F | (6-)CF₃ |
| 0 | - | CH₂F | (6-)OCF₃ |
| 0 | - | CF₃ | (6-)CF₃ |
| 0 | - | CF₃ | (6-)OCH₃ |

## Revendications

1. Composés sulfonylamino(thio)carbonyliques de formule générale (I), 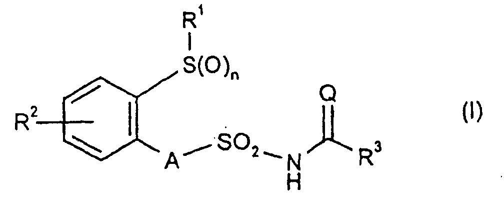 dans laquelle
n représente les nombres 0, 1 ou 2,
A représente une liaison simple,
Q représente l'oxygène ou le soufre,
R¹ représente l'hydrogène, un reste formyle, un reste alkyle, alkoxy, alkylamino, alkoxyamino, dialkylamino, N-alkoxy-N-alkylamino, alkylcarbonyle, alkoxycarbonyle, alkylsulfonyle, alcényle ou alcynyle ayant chacun 1 à 6 atomes de carbone et portant chacun, le cas échéant, un substituant cyano, fluoro, chloro, bromo, phényle ou alkoxy en C₁ à C₄, ou bien un reste cycloalkyle en C₃ à C₆ (cyloalkyle en C₃ à C₆)-carbonyle ou (cycloalkyle en C₃ à C₆) sulfonle dont chacun est éventuellement substitué par un radical cyano, fluoro, chloro, bromo ou alkyle en C₁ à C₄,
R² est un groupe le fluor, le chloré, le brome ou un reste alkyle, alkoxy, alkylthio, alkyl-sulfinyle, alkylsulfonyle, dialkylaminosulfonyle, alcényle, alcynyle, alcényloxy ou alcynyloxy ayant chacun jusqu'à 6 atomes de carbone et portant chacun, le cas échéant, un substituant cyano, fluoro, chloro, bromo ou alkoxy en C₁ à C₄, et
R³ est un reste hétérocyclyle éventuellement substitué de formule suivante : 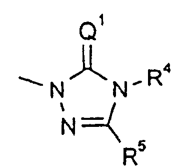
dans laquelle
Q¹ représente l'oxygène ou le soufre
R⁴ représente l'hydrogène, un groupe hydroxy, amino, cyano, un reste alkylidène-amino en C₂ à C₁₀, un reste alkyle en C₁ à C₆ portant éventuellement un substituant fluoro, chloro, bromo, cyano, alkoxy en C₁ à C₄ (alkyle en C₁ à C₄) carbonyle ou (alkoxy en C₁ à C₄)-carbonyle, un reste alcényle en C₂ à C₆ ou alcynyle en C₂ à C₆ portant chacun, le cas échéant, un substituant fluoro, chloro et/ou bromo, un reste alkoxy en C₁ à C₆, alkylamino en C₁ à C₆ ou (alcyle en C₁ à C₆) carbonylamino portant chacun, le cas échéant, un substituant fluoro, chloro, bromo, cyano, alkoxy en C₁ à C₄ ou (alkoxy en C₁ à C₄)-carbonyle, un reste alcényloxy en C₃ à C₆, un reste di(alkyle en C₁ à C₄)-amino, un reste cycloalkyle en C₃ à C₆, cycloalkylamino en C₃ à C₆ ou (cycloalkyle en C₃ à C₆)-alkyle en C₁ à C₄ portant chacun, le cas échéant, un substituant fluoro, chloro, bromo, cyano et/ou alkyle en C₁ à C₄, ou un reste phényle ou phényl-(alkyle en C₁ à C₄) portant chacun, le cas échéant, un substituant fluoro, chloro, bromo, cyano, nitro, alkyle en C₁ à C₄, trifluorométhyle et/ou alkoxy en C₁ à C₄,
R⁵ représente l'hydrogène, un groupe hydroxy, mercapto, amino, cyano, le fluor, le chlore, le brome, l'iode, un reste alkyle en C₁ à C₆ portant éventuellement un substituant fluoro, chloro, bromo, cyano, alkoxy en C₁ à C₄, (alkyle en C₁ à C₉) carbonyle ou (alkoxy en C₁ à C₄), carbonyle, un reste alcényle en C₂ à C₆ ou alcynyle en C₂ à C₆ dont chacun porte éventuellement un substituant fluoro, chloro et/ou bromo, un reste alkoxy en C₁ à C₆, alkylthio en C₁ à C₆, alkylamino en C₁ à C₆ ou (alkyle en C₁ à C₆)carbonylamino dont chacun porte, le cas échéant, un substituant fluoro, chloro, cyano, alkoxy en C₁ à C₄ ou (alkoxy en C₁ à C₄)-carbonyle, un reste alcényloxy en C₃ à C₆, alcynyloxy en C₃ à C₆, alcénylthio en C₃ à C₆, alcynylthio en C₃ à C₆, alcénylamino en C₃ à C₆ ou alcynylamino en C₃ à C₆, un reste di(alkyle en C₁ à C₄) amino, un reste aziridino, pyrrolidino, pipéridino ou morpholino portant chacun, le cas échéant, un substituant méthyle et/ou éthyle, un reste cycloalkyle en C₃ à C₆, cycloalcényle en C₅ ou C₆, cycloalkyloxy en C₃ à C₆, cycloalkylthio en C₃ à C₆, cycloalkylamino en C₃ à C₆ (cycloalkyle en C₃ à C₆) -alkyle en C₁ à C₄ (cycloalkyle en C₃ à C₆) - (alkox en C₁ à C₄ ) (cycloalkyle en C₃ à C₆)-(alkylthio en C₁ à C₄ ou (cycloalkyle en C₃ à C₆)-alkylamino en C₁ à C₄ dont chacun porte, le cas échéant, un substituant fluoro, chloro, bromo, cyano et/ou alkyle en C₁ à C₄, ou un reste phényle, phényl-(alkyle en C₁ à C₄), phénoxy, phényl-(alkoxy en C₁ à C₄, phénylthio, phényl- (alkylthio en C₁ à C₄), phénylamino ou phényl-(alkylamino en C₁ à C₄) portant chacun, le cas échéant, un substituant fluoro, chloro, bromo, cyano, nitro, alkyle en C₁ à C₄, trifluorométhyle, alkoxy en C₁ à C₄ et/ou (alkoxy en C₁ à C₄)-carbonyle, ou bien
R⁴ et R⁵ forment ensemble un reste alkandiyle de 3 à 11 atomes de carbone éventuellement ramifié, ainsi que les sels de composés de formule (I).

2. Composés de formule (I) suivant la revendication 1, **caractérisés en ce que**
n représente les nombres 0, 1 ou 2,
A est une liaison simple,
Q représente l'oxygène ou le soufre,
R¹ représente l'hydrogène, un reste formyle, un reste méthyle, éthyle, n-propyle, isopropyle, n-butyle, isobutyle, sec.-butyle, méthoxy, éthoxy, n-propoxy, isopropoxy, n-butoxy, isobutoxy, sec.-butoxy, tertio-butoxy, méthylamino, éthylamino, n-propylamino, isopropylamino, n-butylamino, isobutylamino, sec.-butylamino, tertio-butylamino, méthoxyamino, éthoxyamino, n-propoxyamino, isopropoxyamino, n-butoxyamino, isobutoxyamino, sec.-butoxyamino, tertio-butoxyamino, diméthylamino, diéthylamino, n-méthoxy, N-méthylamino, acétyle, propionyle, butyroyle, méthoxy-carbonyle, éthoxycarbonyle, n-propoxycarbonyle, isopropoxycarbonyle, méthylsulfonyle, éthylsulfonyle, n-propylsulfonyle, isopropylsulfonyle, n-butylsulfonyle, isobutylsulfonyle, sec.-butylsulfonyle, tertio-butylsulfonyle, propényle, butényle, propynyle ou butynyle portant chacun, le cas échéant, un substituant fluoro, chloro, bromo, métoxy ou éthoxy, ou un reste cyclopropyle, cyclopropylcarbonyle ou cyclopropylsulfonyle dont chacun est éventuellement substitué par du fluor, du chlore ou un radical méthyle,
R² est un groupe cyano, le fluor, le chlore, le brome ou un reste méthyle, éthyle, n-propyle, isopropyle, n-butyle, isobutyle, sec.-butyle, méthoxy, éthoxy, n-propoxy, isopropoxy, n-butoxy, isobutoxy, sec.-butoxy, méthylthio, éthylthio, n-propylthio, isopropylthio, n-butylthio, isobutylthio, sec.-butylthio, tertiobutylthio, méthylsulfinyle, éthylsulfinyle, méthylsulfonyle, éthylsulfonyle, diméthylaminosulfonyle ou diéthylaminosulfonyle dont chacun est éventuellement substitué par du fluor, du chlore, un radical méthoxy ou éthoxy, un reste propényle, butényle, propynyle, butynyle, propényloxy, butényloxy, propinyloxy ou butynyloxy et
R³ est un reste hétérocyclyle éventuellement substitué de formule suivante : 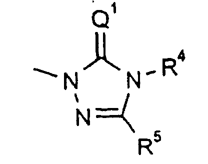
dans laquelle
Q¹ représente l'oxygène ou le soufre,
R⁴ est l'hydrogène, un groupe hydroxy, amino, un reste alkylidène-amino en C₃ à C₈, un reste méthyle, éthyle, n-propyle, isopropyle, n-butyle, isobutyle, sec.-butyle ou tertio-butyle portant chacun, le cas échéant, un substituant fluoro, chloro, cyano, méthoxy ou éthoxy, un reste propényle, butényle, propynyle ou butynyle portant chacun, le cas échéant, un substituant fluoro, chloro ou bromo, un reste méthoxy, éthoxy, n-propoxy, isopropoxy, n-butoxy, isobutoxy, sec.-butoxy, tertiobutoxy, méthylamino, éthylamino, n-propylamino, isopropylamino, n-butylamino, isobutylamino, sec.-butylamino, tertio-butylamino portant chacun, le cas. échéant, un substituant fluoro, chloro, cyano, méthoxy ou éthoxy, un reste propényloxy ou butényloxy, un reste dimétylamino ou diéthylamino, un reste cyclopropyle, cyclobutyle, cyclopentyle, cyclohexyle, cyclopropylamino, cyclobutylamino, cyclopentylamino, cyclohexylamino, cyclopropylméthyle, cyclobutylméthyle, cyclopentylméthyle ou cyclohexylméthyle, dont chacun porte éventuellement un substituant fluoro, chloro, méthyle et/ou éthyle, un reste phényle ou benzyle portant chacun, le cas échéant, un substituant fluoro, chloro, méthyle, trifluorométhyle et/ou méthoxy,
R⁵ représente l'hydrogène, un groupe hydroxy, mercapto, amino, le fluor, le chlore, le brome, un reste méthyle, éthyle, n-propyle, isopropyle, n-butyle, isobutyle, sec.-butyle ou tertio-butyle portant chacun; le cas échéant, un substituant fluoro, chloro, cyano, méthoxy ou éthoxy, un reste éthényle, propényle, butényle, propynyle ou butynyle portant chacun, le cas échéant, un substituant fluoro, chloro ou bromo, un reste méthoxy, éthoxy, n-propoxy, isopropoxy, n-butoxy, isobutoxy, sec.-butoxy, tertiobutoxy, méthylthio, éthylthio, n-propylthio, isopropylthio, nbutylthio, isobutylthio, sec.-butylthio, tertio-butylthio, méthylamino, éthylamino, n-propylamino, isopropylamino, n-butylamino, isobutylamino, sec.-butylamino ou tertio-butylamino portant chacun, le cas échéant, un substituant fluoro, chloro, cyano, méthoxy ou éthoxy, un reste propényloxy, butényloxy, propynyloxy, butynyloxy, propénylthio, propadiénylthio, buténylthio, propynylthio, butynylthio, propénylamino, buténylamino, propynylamino ou butynylamino, un reste diméthylamino, diéthylamnino ou dipropylamino, un reste cyclopropyle, cyclobutyle, cyclopentyle, cyclohexyle, cyclopentényle, cyclohexényle, cyclopropyloxy, cyclobutyloxy, cyclopentyloxy, cyclohexyloxy, cyclopropylthio, cyclobutylthio, cyclopentylthio, cyclohexylthio, cyclopropylamino, cyclobutylamino, cyclopentylamino, cyclohexylamino, cyclopropylméthyle, cyclobutylméthyle, cyclopentylméthyle, cyclohexylméthyle, cyclopropylméthoxy, cyclobutylméthoxy, cyclopentylméthoxy, cyclohexylméthoxy, cyclopropylméthylthio, cyclobutylméthylthio, cyclopentylméthylthio, cyclohexylméthylthio, cyclopropylméthylamino, cyclobutylméthylamino, cyclopentylméthylamino ou cyclohexylméthylamino portant chacun, le cas échéant, un substituant fluoro, chloro, méthyle et/ou éthyle, ou un reste phényle, benzyle, phénoxy, benzyloxy, phénylthio, benzylthio, phénylamino ou benzylamino portant chacun, le cas échéant, un substituant fluoro, chloro, méthyle, trifluorméthyle, méthoxy et/ou méthoxycarbonyle, ou bien
R⁴ et R⁵ forment ensemble un reste alcanediyle éventuellement ramifié ayant 3 à 11 atomes de carbone.

3. Composés suivant la revendication 1, de formule (I) dans laquelle
n représente les nombres 0, 1 ou 2,
A est une liaison simple,
Q représente l'oxygène ou le soufre,
R¹ est un reste méthyle, éthyle, n-propyle, isopropyle, n-butyle, isobutyle, sec.-butyle ou tertio-butyle dont chacun est éventuellement substitué par du fluor et/ou du chlore,
R² représente le fluor, le chlore, le brome ou un reste méthyle, éthyle, méthoxy, éthoxy, méthylthio ou éthylthio - chacun en position 6 - portant chacun, le cas échéant, un substituant fluoro et/ou chloro, et
R³ est un reste triazolinyle éventuellement substitué de formule suivante : 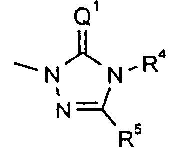
dans laquelle
Q¹ représente l'oxygène ou le soufre, .
R⁴ est un reste méthyle, éthyle, n-propyle ou isopropyle portant éventuellement un substituant fluoro, chloro, cyano, méthoxy ou éthoxy, un reste propényle ou propynyle, un reste méthoxy, éthoxy, n-propoxy, isopropoxy ou un reste cyclopropyle,
R⁵ représente l'hydrogène, le chlore, le brome, un reste méthyle, éthyle, n-propyle ou isopropyle portant chacun, le cas échéant, un substituant fluoro, chloro, cyano, méthoxy ou éthoxy, un reste propényle ou propynyle portant chacun, le cas échéant, un substituant fluoro et/ou chloro, un reste méthoxy, éthoxy, n-propoxy, isopropoxy, méthylthio, éthylthio, n-propylthio ou isopropylthio portant chacun, le cas échéant, un substituant fluoro, chloro, cyano, méthoxy ou éthoxy, un reste propényloxy ou cyclopropyle.

4. Procédé de production de composés de formule (I) suivant la revendication 1 et de leurs sels, **caractérisé en ce que** :
(a) on fait réagir des composés aminosulfonyliques de formule générale (II) 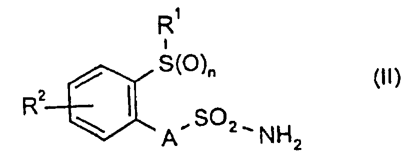 dans laquelle
n, A, R¹ et R² ont la définition indiquée dans la revendication 1,
avec des dérivés d'acides (thio)carboxyliques de formule générale (III) 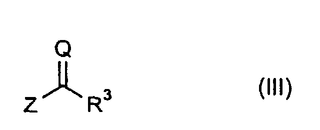 dans laquelle
Q et R³ ont la définition indiquée dans la revendication 1 et Z représente un halogène, un reste alkoxy, aryloxy ou arylalkoxy,
éventuellement en présence d'un accepteur d'acide et, le cas échéant, en présence d'un diluant, ou **en ce que** :
(b) on fait réagir des sulfonyliso(thio)cyanates de formule générale (IV) 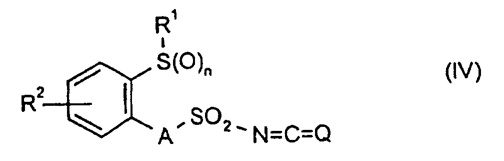 dans laquelle
n, A, Q, R¹ et R² ont la définition indiquée ci-dessus, avec des hétérocycles de formule générale (V)
H-R³ (V)
dans laquelle
R¹ a la définition indiquée ci-dessus,
le cas échéant en présence d'un auxiliaire de réaction et en présence éventuelle d'un diluant, ou, **en ce que** :
(c) on fait réagir des composés chlorosulfonyliques de formule générale (VI) 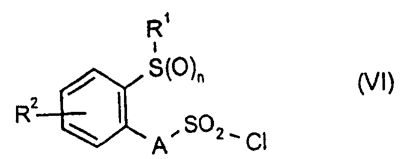 dans laquelle
n, A, R¹ et R² ont la définition indiquée ci-dessus, avec des hétérocyles de formule générale (V)
H-R³ (V)
dans laquelle
R³ a la définition indiquée ci-dessus, et avec des (thio)cyanates métalliques de formule générale (VII)
MQCN (VII)
dans laquelle
Q a la définition indiquée ci-dessus,
le cas échéant, en présence d'un auxiliaire de réaction et en présence éventuelle d'un diluant, ou **en ce que** :
(d) on fait réagir des composés chlorosulfonyliques de formule générale (VI) 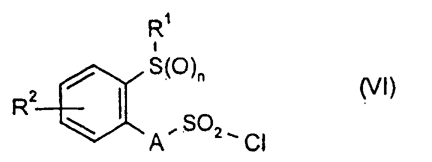 dans laquelle
n, A, R¹ et R² ont la définition indiquée ci-dessus, avec des amides d'acides(thio)carboxyliques de formule générale (VIII) 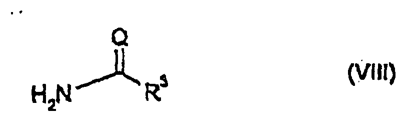 dans laquelle
Q et R³ ont la définition indiquée ci-dessus, le cas échéant, en présence d'un accepteur d'acide et éventuellement en présence d'un diluant, ou **en ce que** :
(e) on fait réagir des composés sulfonylamino(thio)carbonyliques de formule générale IX) 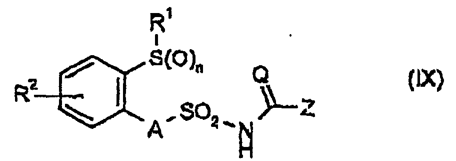 dans laquelle
n, A, Q, R¹ et R² ont la définition indiquée ci-dessus et
Z représente un halogène, un reste alkoxy, aryloxy ou arylalkoxy, avec des hétérocycles de formule générale (v)
H-R³ (V)
dans laquelle
R³ a la définition indiquée ci-dessus, le cas échéant, en présence d'un accepteur d'acides et en présence éventuelle d'un diluant,
ou **en ce que** :
(f) on fait réagir des hétérocycles de formule générale (V)
H-R³ (V)
dans laquelle
R³ a la définition indiquée ci-dessus,
avec un chlorosulfonyliso(thio)cyanate, le cas échéant, en présence d'un diluant et on fait réagir in situ les produits d'addition ainsi formés avec des dérivés benzéniques de formule générale (X) 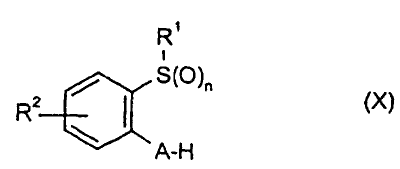 dans laquelle
n, A, R¹ et R² ont la définition indiquée ci-dessus, le cas échéant en présence d'un accepteur d'acide et en présence éventuelle d'un diluant, et on transforme éventuellement en sels par des modes opératoires classiques les composés obtenus par les procédés (a), (b) (c) , (d) , (e) ou (f).

5. Compositions herbicides; **caractérisées par** une teneur en au moins un composé de formule (I) ou un sel de ce composé suivant la revendication 1.

6. Utilisation de composés de formule générale (I) ou de leurs sels suivant la revendication 1 pour combattre une végétation indésirable.

7. Procédé pour combattre des mauvaises herbes, **caractérisé en ce qu'**on fait agir des composés de formule générale (I) ou leurs sels suivant la revendication 1 sur les mauvaises herbes ou sur leur milieu.

8. Procédé de préparation de compositions herbicides, **caractérisé en ce qu'**on mélange des composés de formule générale (I) ou leurs sels suivant la revendication 1 avec des diluants et/ou des agents tensio-actifs.

9. Composés de formule (II) 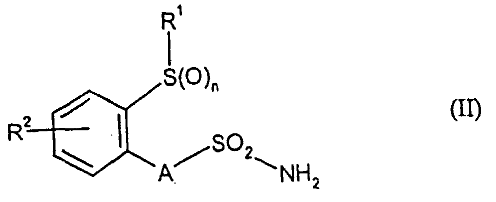 dans lesquels
| n | A | R¹ | (Position-)R² |
|---|---|---|---|
| 0 | - | CH₃ | (6-)C₂H₅ |
| 0 | - | C₂H₅ | (6-)OCH₃ |
| 0 | - | C₂H₅ | (6-)F |
| 0 | - | C₂H₅ | (6-)Cl |
| 0 | - | C₂H₅ | (6-)CF₃ |
| 1 | - | C₂H₅ | (6-)CF₃ |
| 2 | - | C₂H₅ | (6-)CF₃ |
| 0 | - | i-C₃H₇ | (6-)OCH₃ |
| 0 | - | i-C₃H₇ | (6-)Cl |
| 0 | - | CH₃ | (6-)Cl |
| 0 | - | CH₃ | (6-)OCH₃ |
| 0 | - | CH₃ | (6-)OCF₃ |
| 0 | - | i-C₃H₇ | (6-)OCF₃ |
| 0 | - | i-C₃H₇ | (6-)CH₃ |
| 0 | - | i-C₃H₇ | (6-)C₂H₅ |
| 0 | - | i-C₃H₇ | (6-)SCH₃ |
| 0 | - | C₂H₅ | (6-)SC₂H₅ |
| 0 | - | C₂H₄OCOCF₃ | (6-)OCH₃ |
| 0 | - | CH₃ | (6-)SCH₃ |
| 0 | - | C₂H₅ | (6-)CH₃ |
| 0 | - | CH₃ | (6-)CH₃ |
| 0 | - | CH₃ | (6-)CF₃ |
| 0 | - | H | (6-)OCF₃ |
| 0 | - | C₂H₄F | (6-)OCF₃ |
| 0 | - | CH₂C≡CH | (6-)OCF₃ |
| 0 | - | CF3 | (6-)OCF₃ |
| 0 | - | C₂H₃F₂ | (6-)CF₃ |
| 0 | - | C₃H₆F | (6-)CF₃ |
| 0 | - | CH₂F | (6-)OCF₃ |
| 0 | - | CF₃ | (6-)CF₃ |
| 0 | - | CF₃ | (6-)OCH₃ |
